(19) Europäisches Patentamt
European Patent Office
Office européen des brevets

(11) Veröffentlichungsnummer : **0 035 474**
**B1**

(12) # EUROPÄISCHE PATENTSCHRIFT

(45) Veröffentlichungstag der Patentschrift : **05.06.85**

(21) Anmeldenummer : **81810064.6**

(22) Anmeldetag : **26.02.81**

(51) Int. Cl.[4] : **C 07 D487/04**, A 61 K 31/415, A 61 K 31/505, A 61 K 31/55, A 61 K 31/44// C07D209/18, C07D401/12, C07D409/12 ,(C07D487/04, 239:00, 209:00),(C07D487/04, 235:00, 209:00),(C07D487/04, 221:00, 209:00)

(54) **Neue Amidine, Verfahren zu ihrer Herstellung, diese enthaltende pharmazeutische Präparate und ihre Verwendung.**

(30) Priorität : **04.03.80 CH 1703/80**

(43) Veröffentlichungstag der Anmeldung : **09.09.81 Patentblatt 81/36**

(45) Bekanntmachung des Hinweises auf die Patenterteilung : **05.06.85 Patentblatt 85/23**

(84) Benannte Vertragsstaaten : **AT BE CH DE FR IT LI LU NL SE**

(56) Entgegenhaltungen :
**EP-A- 0 008 950**
**CHEMICAL ABSTRACTS, Band 86, Nr. 7, 14. Februar 1977, Zusammenfassung 37493q, Seite 13, COLUMBUS OHIO (US)**
**E. Schröder et al., Arzneimittelchemie, Stuttgart 1976, S. 12-15, 410,411,400-403,68-75,110,111,122,123,266-269**
**Burger's Medicinal Chemistry, 4. Ed. Part III, New York 1979, S. 1236**

(73) Patentinhaber : **CIBA-GEIGY AG**
**Postfach**
**CH-4002 Basel (CH)**

(72) Erfinder : **Renner, Ulrich, Dr.**
**Auf der Bischoffhöhe 26**
**CH-4125 Riehen (CH)**
Erfinder : **Jaeggi, Knut A., Dr.**
**General Guisan-Strasse 44**
**CH-4054 Basel (CH)**

**EP 0 035 474 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N,N'-überbrückte Carbonsäureamidine der allgemeinen Formel

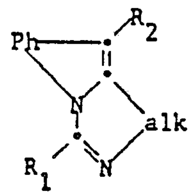

(I)

worin $R_1$ gegebenenfalls durch Niederalkyl, Niederalkoxy, gegebenenfalls Halogen enthaltendes Niederalkylthio, Niederalkansulfinyl bzw. Niederalkansulfonyl, gegebenenfalls durch Niederalkyl gegebenenfalls mono- oder disubstituiertes Sulfamoyl und/oder Halogen substituiertes Phenyl oder gegebenenfalls Niederalkyl, Niederalkoxy und/oder Halogen enthaltendes 5- oder 6-gliedriges und als Heteroatom Stickstoff, Sauerstoff oder Schwefel bzw. Stickstoff und zusätzlich Schwefel oder Sauerstoff aufweisendes, monocyclisches Heteroaryl darstellt, $R_2$ 1-Carboxyniederalkyl der Formel $—CH(R_3)—R_2'$ ist, in der $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1-3 C-Atome trennendes Niederalkylen bzw. durch 2 C-Atome trennendes Niederalkenylen ist, und ihre Salze, Verfahren zu ihrer Herstellung, diese enthaltende pharmazeutische Präparate und ihre Verwendung als Arzneimittelwirkstoff. Unter den mit « nieder » bezeichneten Resten sind solche zu verstehen, die bis und mit 7 C-Atome enthalten.

Ein aromatischer Rest $R_1$ ist beispielsweise gegebenenfalls durch Niederalkyl, Niederalkoxy, gegebenenfals Halogen enthaltendes Niederalkylthio, Niederalkansulfinyl oder Niederalkansulfonyl, gegebenenfalls substituiertes Sulfamoyl und/oder Halogen substituiertes Phenyl oder gegebenenfalls z. B. durch Niederalkyl, Niederalkoxy und/oder Halogen substituiertes 5- oder 6-gliedriges und als Heteroatom Stickstoff, Sauerstoff oder Schwefel bzw. Stickstoff und zusätzlich Schwefel oder Sauerstoff aufweisendes, monocyclisches Heteroaryl. Substituiertes Sulfamoyl bedeutet beispielsweise N-Mono- bzw. N,N-Diniederalkyl-sulfamoyl.

Solche 5-gliedrige Reste sind z. B. Pyrrolyl, wie 2-Pyrrolyl, Furyl, wie 2-Furyl, Thienyl, wie 2- oder 3-Thienyl, oder Thiazolyl, wie 2-Thiazolyl. 6-gliedriges Heteroaryl enthält mindestens ein N-Atom und ist z. B. Pyridyl, wie 2-, 3- oder 4-Pyridyl, oder Pyrimidyl, wie 2-Pyrimidyl.

Verestertes 1-Carboxyniederalkyl weist als veresterte Carboxygruppe beispielsweise Niederalkoxycarbonyl auf, welches auch durch gegebenenfalls substituiertes Phenyl oder Pyridyl einfach oder durch Hydroxy, Halogen bzw. Niederalkoxy ein- oder mehrfach substituiert sein kann, wie gegebenenfalls durch Hydroxy, Niederalkoxy und/oder Halogen substituiertes Niederalkoxycarbonyl, z. B. Mono- bzw. Dihydroxyniederalkoxy-, Halogen- oder Niederalkoxyniederalkoxycarbonyl, oder durch Niederalkyl, Niederalkoxy und/oder Halogen substituiertes Phenylniederalkoxycarbonyl.

Amidiertes 1-Carboxyniederalkyl weist als amidierte Carboxygruppe beispielsweise Carbamoyl auf, welches gegebenenfalls durch Hydroxy bzw. Amino einfach, durch Niederalkyl bzw. Hydroxyniederalkyl ein- bzw. zweifach oder durch 4- bis 7-gliedriges Niederalkylen bzw. 3-Oxa-, 3-Thia- oder 3-Azaalkylen zweifach substituiert sein kann. Als Beispiele seien genannt : N-Hydroxy-, N-Amino-, N-Mono- bzw. N,N-Diniederalkyl- oder N-Mono- bzw. N,N-Dihydroxyalkylcarbamoyl. Durch 4- bis 7-gliedriges Niederalkylen zweifach N-substituiertes Carbamoyl ist z. B. Pyrrolidino- oder Piperidinocarbonyl bzw. Morpholino-, Thiomorpholino-, Piperazino- oder N-Niederalkyl-, wie N-Methylpiperazino-carbonyl.

1,2-Phenylen ist gegebenenfalls zusätzlich ein- oder mehrfach, z. B. durch Niederalkyl, Niederalkoxy, Halogen oder Trifluormethyl, substituiert.

Der Rest alk ist beispielsweise ein die Methingruppe von der Iminogruppe durch 1 bis 3 C-Atome trennender Niederalkylenrest, wie Ethylen, 1,2- oder vor allem 1,3-Propylen, oder ein die Methingruppe von der Iminogruppe durch 2 C-Atome trennender Vinylenrest.

In der vorliegenden Beschreibung sind unter « niederen » organischen Resten und Verbindungen vorzugsweise solche zu verstehen, die bis und mit 4 Kohlenstoffatome enthalten.

Die vor- und nachstehend verwendeten Allgemeindefinitionen haben im Rahmen der vorliegenden Beschreibung die folgenden Bedeutungen.

Niederalkyl ist z. B. Methyl, Ethyl, n-Propyl, Isopropyl, n-Butyl, Isobutyl, sek.-Butyl, tert.-Butyl, ferner ein Pentyl-, Hexyl- oder Heptylrest.

Niederalkoxy ist z. B. Methoxy, Ethoxy, n-Propyloxy, Isopropyloxy, n-Butyloxy, Isobutyloxy, sek.-Butyloxy oder tert.-Butyloxy.

Niederalkylthio ist z. B. Methyl-, Ethyl-, n-Propyl-, Isopropyl-, n-Butyl-, Isobutyl-, sek.-Butyl-, tert.-Butyl, n-Pentyl-, n-Hexyl- oder n-Heptylthio, Niederalkansulfinyl bzw. -sulfonyl ist z. B. Methan-, Ethan- oder n-Propansulfinyl bzw. -sulfonyl.

N-Niederalkylsulfamoyl bzw. N,N-Diniederalkylsulfamoyl ist z. B. N-Methyl-, N-Ethyl-, N-Propyl-, N,N-Dimethyl-, N,N-Diethyl-, N,N-Methylethyl- oder N,N-Dipropyl-sulfamoyl.

Halogen ist z. B. Halogen bis und mit Atomnummer 35, wie Fluor, Chlor oder Brom.

Halogenniederalkylthio ist z. B. Chlormethyl-, Chlorethyl- oder Chlorpropylthio oder eine der entsprechenden Fluor- bzw. Bromniederalkylthiogruppen.

Halogenniederalkansulfinyl bzw. -sulfonyl ist z. B. Chlormethan-, Chlorethan- oder Chlorpropansulfinyl bzw. -sulfonyl sowie die entsprechenden Fluor- bzw. Bromniederalkansulfinyl- bzw. -niederalkansulfonylgruppen.

Niederalkoxycarbonyl ist z. B. Methoxy-, Ethoxy-, n-Propyloxycarbonyl, ferner ein Butyloxy-, Pentyloxy-, Hexyloxy- oder Heptyloxycarbonylrest.

Phenyl- bzw. Pyridylniederalkoxycarbonyl ist z. B. Phenylmethoxycarbonyl, Phenylethoxycarbonyl, 2-, 3- oder 4-Pyridylmethoxycarbonyl.

Hydroxyniederalkoxycarbonyl bzw. Dihydroxyniederalkoxycarbonyl ist z. B. 1- oder 2-Hydroxyethyl-, 1- oder 3-Hydroxypropyl- bzw. 1- oder 4-Hydroxybutylcarbonyl bzw. 2,3-Dihydroxypropyloxycarbonyl, 2,3-, 2,4- oder 3,4-Dihydroxybutyloxycarbonyl.

Niederalkoxyniederalkoxycarbonyl ist z. B. 2-Methoxyethyloxy-, 1- oder 2-Ethoxyethyloxy- 2- oder 3-Methoxypropyloxy- bzw. 2-, 3- oder 4-Methoxybutyloxycarbonyl.

Hydroxyalkyl ist z. B. Hydroxymethyl oder Hydroxyethyl, ferner Hydroxypropyl oder Hydroxybutyl.

N-Niederalkylcarbamoyl bzw. N,N-Diniederalkylcarbamoyl ist z. B. N-Methyl-, N-Ethyl-, N-Propyl-, N-Isopropyl-, N-Butyl-carbamoyl bzw. N,N-Dimethyl- oder N,N-Diethyl- oder N,N-Methylethylcarbamoyl.

N-Hydroxyniederalkylcarbamoyl bzw. N,N-Dihydroxyniederalkylcarbamoyl ist z. B. N-Hydroxymethyl- oder N-2-Hydroxyethylcarbamoyl bzw. N,N-2,3-Dihydroxypropylcarbamoyl.

Salze von erfindungsgemässen Verbindungen der Formel I sind vorzugsweise pharmazeutisch verwendbare Salze, wie entsprechende Säureadditionssalze und/oder, wenn $R_2$ 1-Carboxyniederalkyl ist, innere Salze oder Salze mit Basen. Geeignete Säureadditionssalze sind beispielsweise Salze mit anorganischen Säuren, wie Mineralsäuren, oder organischen Säuren, wie Sulfaminsäuren, z. B. Cyclohexylsulfaminsäure, gegebenenfalls ungesättigten Dicarbonsäuren oder gegebenenfalls durch Hydroxy zusätzlich substituierten bzw. zusätzlich Oxo und/oder Carboxy aufweisende Carbonsäuren, oder Sulfonsäuren, Mineralsäuren sind beispielsweise Schwefelsäure oder Halogenwasserstoffsäuren, wie Brom- oder Chlorwasserstoffsäure. Als gegebenenfalls ungesättigte Dicarbonsäuren kommen z. B. Oxalsäure, Malon-, Fumar- oder Maleinsäure in Betracht, als gegebenenfalls durch Hydroxy zusätzlich substituierte bzw. zusätzlich Oxo und/oder Carboxy aufweisende Carbonsäuren werden z. B. Wein-, Aepfel-, Brenztrauben- oder Citronensäure verwendet. Sulfonsäuren sind z. B. Benzol-, p-Toluol- oder Methansulfonsäure.

Geeignete Salze mit Basen sind beispielsweise Metall-, wie Alkali- oder Erdalkalimetallsalze, z. B. Natrium-, Kalium-, oder Magnesiumsalze, Uebergangsmetallsalze, wie Zink- oder Kupfersalze, oder Salze mit Ammoniak oder von substituierten organischen Aminen, wie Morpholin, Thiomorpholin, Piperidin, Pyrrolidin, wie Mono-, Di-, bzw. Triniederalkylaminen oder Mono-, Di-, bzw. Trihydroxyniederalkylaminen, z. B. Mono-, Di- oder Triethanolamin. Mononiederalkylamine sind beispielsweise Ethyl- oder tert.-Butylamin. Diniederalkylamine sind z. B. Diethyl- oder Dipropylamin, und als Triniederalkylamine kommen z. B. Triethyl-, Tributylamin oder Dimethylpropylamin in Betracht.

In Chemical Abstracts, Vol. 86 : 37,493q werden antidepressiv wirkende Verbindungen beschrieben, die ähnlich wie die erfindungsgemässen Verbindungen ein Pyrimido-indol-Grundgerüst aufweisen, sich aber im Substitutionsmuster wie auch hinsichtlich ihrer pharmakologischen Eigenschaften von den Verbindungen der Formel I unterscheiden.

Die Verbindungen der Formel I weisen wertvolle pharmakologische Eigenschaften auf. Insbesondere besitzen sie eine ausgeprägte antinociceptive (analgetische) Wirkung, die sich beispielsweise anhand des Essigsäure-Writhing-Syndroms an der Ratte im Dosisbereich von etwa 1 bis etwa 30 mg/kg p. o. und im Phenyl-p-Benzochinon-Writhing-Test an der Maus im Dosisbereich von etwa 1 bis etwa 30 mg/kg p. o. zeigen lässt.

Ferner weisen sie eine deutliche anti-inflammatorische und antiarthritische Wirkung auf, die sich durch Hemmung des Kaolinpfotenoedems bei der Normalratte im Dosisbereich von etwa 10 bis 100 mg/kg p. o. nachweisen lässt und die sich zudem in der Hemmung des Carragenin-Pfotenoedems an der Ratte analog der von Pasquale et al., Agents and Actions, 5, 256 (1976), beschriebenen Methode in Dosen von etwa 3 bis etwa 300 mg/kg p. o. zeigen lässt.

Ausserdem. hemmen Verbindungen der Formel I in kurativer Applikation bei viermaliger Gabe von etwa 10 bis 100 mg/kg p. o. das Kaolinpfotenoedem der Adjuvans-Arthritis-Ratte.

Die Verbindungen der Formel I sind deshalb vorzüglich geeignet als Arzneimittel zur Behandlung entzündlicher Erkrankungen, vor allem solcher des rheumatischen bzw. arthritischen Bereichs, als Antiphlogistika und/oder als periphere Analgetika.

Die Erfindung betrifft beispielsweise Verbindungen der Formel I, worin $R_1$ gegebenenfalls durch Niederalkyl, Niederalkoxy, Niederalkylthio, Halogenniederalkylthio, Niederalkansulfinyl, Halogenniederalkansulfinyl, Niederalkansulfonyl, Halogenniederalkansulfonyl, Sulfamoyl, N-Mono- bzw. N,N-Diniederalkylsulfamoyl und/oder Halogen substituiertes Phenyl oder gegebenenfalls durch Niederalkyl, Niederalkoxy und/oder Halogen substituiertes Pyrrolyl, Furyl, Thienyl, Thiazolyl, Pyridyl bzw. Pyrimidyl bedeutet, $R_2$ eine Gruppe der Formel —CH($R_3$)—$R_2'$ ist, in der $R_2'$ Carboxy, gegebenenfalls durch Niederalkyl, Niederalkoxy oder Halogen substituiertes Phenyl- bzw. Pyridylniederalkoxycarbonyl, Hydroxy- bzw. Niederalkoxyniederalkoxycarbonyl, Niederalkoxycarbonyl, Carbamoyl, N-Hydroxy- bzw. N-Aminocarbamoyl, N-Mono- bzw. N,N-Diniederalkylcarbamoyl bzw. -hydroxyniederalkylcarbamoyl oder durch 4- bis 7-gliedriges Niederalkylen substituiertes Carbamoyl bzw. 3-Oxa-, 3-Thia- oder 3-Azaalkylenaminocarbonyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1 bis 3 C-Atome trennendes Niederalkylen bzw. durch 2 C-Atome trennendes Niederalkenylen ist, sowie ihre Salze, insbesondere pharmazeutisch verwendbaren Salze.

Die Erfindung betrifft in erster Linie Verbindungen der Formel I, worin $R_1$ gegebenenfalls durch Niederalkyl mit bis und mit 4 C-Atomen, wie Methyl, Niederalkoxy mit bis und mit 4 C-Atomen, wie Methoxy, Halogenniederalkylthio mit bis und mit 4 C-Atomen, wie Chlormethylthio, Niederalkylthio mit bis und mit 4 C-Atomen wie Methylthio, Halogenniederalkansulfinyl mit bis und mit 4 C-Atomen, wie Chlormethansulfinyl, Niederalkansulfinyl mit bis und mit 4 C-Atomen, wie Methansulfinyl, Halogenniederalkansulfonyl, wie Chlormethansulfonyl, Niederalkansulfonyl mit bis und mit 4 C-Atomen, wie Methansulfonyl, Sulfamoyl, N-Mono- bzw. N,N-Diniederalkansulfamoyl mit jeweils bis und mit 4 C-Atomen im Alkyl, wie N-Methan- bzw. N,N-Diethylsulfamoyl, und/oder durch Halogen mit Atomnummer bis und mit 35, wie Chlor, substituiertes Phenyl oder gegebenenfalls durch Niederalkyl mit bis und mit 4 C-Atomen, wie Methyl, durch Niederalkoxy mit bis und mit 4 C-Atomen, wie Methoxy, und/oder Halogen mit Atomnummer bis und mit 35, wie Chlor, substituiertes Pyridyl, wie 2-, 3- oder 4-Pyridyl, bzw. Thienyl, wie 2-Thienyl, bedeutet, $R_2$ eine Gruppe der Formel —CH($R_3$)—$R_2'$, in der $R_2'$ Carboxy, gegebenenfalls durch Niederalkyl mit bis und mit 4 C-Atomen, wie Methyl, Niederalkoxy mit bis und mit 4 C-Atomen, wie Methoxy, und/oder Halogen, wie Chlor, substituiertes Phenyl- bzw. Pyridylniederalkoxycarbonyl, wie 2-, 3- oder 4-Pyridylniederalkoxycarbonyl, Niederalkoxycarbonyl, wie Methoxycarbonyl, Mono- bzw. Dihydroxyniederalkoxycarbonyl, wie 2-Hydroxyethoxy- bzw. 2,3-Dihydroxypropyloxycarbonyl, Niederalkoxyniederalkoxycarbonyl, wie 2-Methoxyethoxycarbonyl, N-Hydroxy- bzw. N-Amino-carbamoyl, N-Mono- bzw. N,N-Diniederalkoxycarbamoyl, wie N-Methyl- bzw. N,N-Diethylcarbamoyl, oder Carbamoyl ist, $R_3$ Wasserstoff oder Niederalkyl mit bis und mit 4 C-Atomen, wie Methyl, bedeutet, Ph gegebenenfalls durch Niederalkyl mit bis und mit 4 C-Atomen, wie Methyl, Niederalkoxy mit bis und mit 4 C-Atomen, wie Methoxy, und/oder Halogen bis und mit Atomnummer 35, wie Fluor, substituiertes 1,2-Phenylen bedeutet und alk 1,2-Ethylen ist, sowie ihre Salze, insbesondere pharmazeutisch verwendbaren Salze.

Die Erfindung betrifft in allererster Linie Verbindungen der Formel I, worin $R_1$ gegebenenfalls durch Halogen, Niederalkylthio, Niederalkansulfinyl, Niederalkansulfonyl, Halogenniederalkylthio und/oder Sulfamoyl substituiertes Phenyl, Pyridyl oder Thienyl bedeutet, $R_2$ eine Gruppe der Formel —$CH_2$—$R_2'$ ist, in der $R_2'$ Carboxy, Niederalkoxycarbonyl oder Carbamoyl darstellt, Ph gegebenenfalls durch Niederalkoxy, Niederalkyl und/oder Halogen substituiertes 1,2-Phenylen bedeutet und alk 1,2-Ethylen oder Vinylen ist, sowie ihre Salze, insbesondere pharmazeutisch verwendbaren Salze.

Die Erfindung betrifft bevorzugt Verbindungen der Formel I, worin $R_1$ gegebenenfalls durch Halogen mit Atomnummer bis und mit 35, wie Chlor, Niederalkylthio mit bis und mit 4 C-Atomen, wie Methylthio, Niederalkansulfinyl mit bis und mit 4 C-Atomen, wie Methansulfinyl, Halogenniederalkylthio mit bis und mit 4 C-Atomen, wie Chlormethylthio, und/oder Sulfamoyl substituiertes Phenyl, Pyridyl, insbesondere 2-Pyridyl, oder Thienyl, insbesondere 2-Thienyl, bedeutet, $R_2$ eine Gruppe der Formel —$CH_2$—$R_2'$ ist, in der $R_2'$ Carboxy, Niederalkoxycarbonyl mit bis und mit 5 C-Atomen, wie Ethoxycarbonyl, oder Carbamoyl darstellt, Ph gegebenenfalls durch Niederalkoxy mit bis und mit 4 C-Atomen, wie Methoxy, Niederalkyl mit bis und mit 4 C-Atomen, wie Methyl, und/oder Halogen mit Atomnummer bis und mit 35, wie Fluor, substituiertes 1,2-Phenylen bedeutet und alk 1,2-Ethylen oder Vinylen ist, sowie ihre Salze, insbesondere pharmazeutisch verwendbaren Salze.

Die Erfindung betrifft in besonders bevorzugter Weise Verbindungen der Formel I, worin $R_1$ gegebenenfalls durch Halogenniederalkylthio mit bis und mit 4 C-Atomen, wie Chlormethylthio, Niederalkylthio mit bis und mit 4 C-Atomen, wie Methylthio, Niederalkansulfinyl mit bis und mit 4 C-Atomen, wie Methansulfinyl, oder Halogen mit Atomnummer bis und mit 35, wie Chlor, in p-Stellung oder durch Sulfamoyl in 3-Stellung und zusätzlich durch Halogen mit Atomnummer bis und mit 35, wie Chlor, in 4-Stellung substituiertes Phenyl, Pyridyl, insbesondere 2-Pyridyl, oder Thienyl, insbesondere 2-Thienyl, bedeutet, $R_2$ eine Gruppe der Formel —$CH_2$—$R_2'$, in der $R_2'$ Carboxy, Niederalkoxycarbonyl mit bis und mit 5 C-Atomen, wie Ethoxycarbonyl, oder Carbamoyl darstellt, Ph gegebenenfalls in bezug auf das gebundene Stickstoffatom in 4-Stellung durch Niederalkoxy mit bis und mit 4 C-Atomen, wie Methoxy, in 3- und 5-Stellung durch Niederalkyl mit bis und mit 4 C-Atomen, wie Methyl, oder in 4-Stellung durch Halogen mit Atomnummer bis und mit 35, wie Fluor, substituiertes 1,2-Phenylen darstellt und alk 1,2-Ethylen oder Vinylen ist, sowie ihre Salze, insbesondere pharmazeutisch verwendbaren Salze.

Die Erfindung betrifft besonders bevorzugt Verbindungen der Formel I, worin $R_1$ gegebenenfalls in p-Stellung durch Niederalkylthio mit bis und mit 4 C-Atomen, wie Methylthio, durch Niederalkansulfinyl mit bis und mit 4 C-Atomen, wie Methansulfinyl, oder Halogen bis und mit Atomnummer 35, wie Chlor,

substituiertes Phenyl bedeutet, $R_2$ Niederalkoxycarbonylmethyl ist, Ph gegebenenfalls in p-Stellung zum Stickstoffatom durch Niederalkoxy mit bis und mit 4 C-Atomen, wie Methoxy, oder Halogen bis und mit Atomnummer 35, wie Fluor, substituiertes 1,2-Phenylen darstellt und alk 1,2-Ethylen bedeutet, sowie ihre Salze, insbesondere ihre pharmazeutisch verwendbaren Salze.

Hervorragender Erfindungsgegenstand sind Verbindungen der Formel I, worin $R_1$ gegebenenfalls in p-Stellung durch Niederalkylthio mit bis und mit 4 C-Atomen, wie Methylthio, durch Niederalkansulfinyl mit bis und mit 4 C-Atomen, wie Methansulfinyl, oder Halogen bis und mit Atomnummer 35, wie Chlor, substituiertes Phenyl bedeutet, $R_2$ Carboxymethyl ist, Ph gegebenenfalls in p-Stellung zum Stickstoffatom durch Niederalkoxy mit bis und mit 4 C-Atomen, wie Methoxy, oder Halogen bis und mit Atomnummer 35, wie Fluor, substituiertes 1,2-Phenylen darstellt und alk 1,2-Ethylen bedeutet, sowie ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Die Verbindungen der Formel I und ihre Salze können nach an sich bekannten Methoden hergestellt werden, beispielsweise indem man aus Verbindungen der allgemeinen Formeln

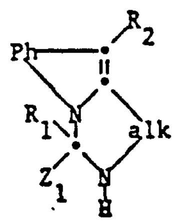

(II) oder

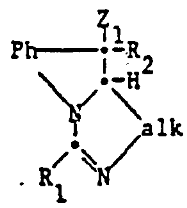

(III)

worin $Z_1$ gegebenenfalls funktionell abgewandeltes Hydroxy oder Mercapto bedeutet, oder Salzen davon unter Einführung einer zusätzlichen Bindung $H—Z_1$ abspaltet und gewünschtenfalls eine erfindungsgemäss erhältliche Verbindung in eine andere Verbindung der Formel I bzw. eine erfindungsgemäss erhältliche freie Verbindung in ein Salz oder ein verfahrensgemäss erhältliches Salz in die freie Verbindung bzw. in ein anderes Salz überführt.

Funktionell abgewandeltes Hydroxy bzw. Mercapto ist beispielsweise durch ein Niederalkanol, wie Methanol oder Ethanol, oder einen gegebenenfalls substituierten aromatischen Alkohol, wie Phenol, verethertes Hydroxy bzw. Mercapto oder mit einer anorganischen Säure, wie einer Mineralsäure, z. B. Halogenwasserstoffsäure, wie Chlorwasserstoffsäure, verestertes Hydroxy und bedeutet beispielsweise Niederalkoxy, wie Methoxy, oder gegebenenfalls substituiertes Aryloxy, wie Phenoxy, Niederalkylthio, wie Methylthio, oder Halogen, wie Chlor oder Brom.

Die Abspaltung von $H—Z_1$ erfolgt in üblicher Weise, beispielsweise spontan, thermisch, d. h. unter Erwärmen, und/oder in Gegenwart eines katalytischen Mittels. Die thermische Abspaltung verläuft üblicherweise in einem Temperaturbereich von etwa 50° bis etwa 200 °C. Als katalytische Mittel verwendet man z. B. basische bzw. saure Katalysatoren, wobei als Basen beispielsweise Alkalimetallhydroxide, -amide bzw. -hydride, wie Kaliumhydroxid, Natriumamid bzw. Natriumhydrid, Metalloxide, wie Aluminiumoxid, vor allem organische Stickstoffbasen, wie tertiäre Amine, z. B. Pyridin, Chinolin oder N,N-Dimethylanilin, und als saure Katalysatoren beispielsweise Mineralsäuren oder saure Salze bzw. Anhydride davon, wie Schwefelsäure oder Phosphorsäuren, Hydrogensulfate, wie Alkalimetallhydrogensulfate, z. B. Kaliumhydrogensulfat, Phosphorpentoxid, oder Mineralsäurehalogenide, wie Schwefelsäurehalogenide, z. B. Sulfurylchlorid, eingesetzt werden. Dabei arbeitet man erforderlichenfalls in Gegenwart eines inerten Lösungs- bzw. Verdünnungsmittels, in einem geschlossenen Gefäss und/oder unter Inertgas, z. B. Stickstoff.

Inerte Lösungs- bzw. Verdünnungsmittel sind gegebenenfalls substituierte Kohlenwasserstoffe, wie gegebenenfalls halogenierte aliphatische oder aromatische Kohlenwasserstoffe, z. B. Chloroform oder Chlorbenzol, Ether, wie aliphatische, cycloaliphatische oder aromatische Ether, z. B. Diethylether, Dioxan, Tetrahydrofuran, Diphenylether oder Anisol, Ketone, wie aliphatische Ketone, z. B. Aceton oder Methylethylketon, Amide, wie Dialkylamide, z. B. Dimethylformamid, oder Sulfoxide, wie Diniederalkylsulfoxide, z. B. Dimethylsulfoxid.

Ausgangsstoffe der Formeln II und III können nach an sich bekannten Verfahren hergestellt werden, beispielsweise indem man Verbindungen der Formel

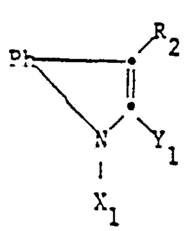

(IVa)

oder Salze davon, worin $X_1$ Wasserstoff und $Y_1$ eine Gruppe der Formel —alk—NH—C(=$Z_1$')($R_1$) bzw. $X_1$ —C(=$Z_1$')($R_1$) und $Y_1$ eine Gruppe der Formel —alk—$NH_2$ und $Z_1$' gegebenenfalls funktionell abgewandeltes Oxo ist, cyclisiert, und gewünschtenfalls eine so erhältliche freie Verbindung der Formel II in eine andere freie Verbindung bzw. in ein Salz oder ein verfahrensgemäss erhältliches Salz in die freie Verbindung bzw. in ein anderes Salz überführt.

Funktionell abgewandeltes Oxo ist beispielsweise Thioxo, ketalisiertes oder thioketalisiertes Oxo, verestertes Dioxy oder Imino. Ketalisierte Oxoverbindungen sind beispielsweise Ketale mit Niederalkanolen, wie Methanol oder Ethanol, oder Niederalkandiolen, wie Ethylenglykol oder Propylenglykole, z. B. mit 1,3-Dihydroxypropan, und Thioketale sind beispielsweise Thioketale mit Niederalkanthiolen, z. B. Methanthiol oder Ethanthiol, oder Niederalkandithiolen, wie 1,2-Ethandithiol oder Propandithiolen, z. B. mit Propan-1,3-dithiol. Imino ist beispielsweise gegebenenfalls durch Niederalkyl oder Phenyl substituiertes Imino, wie N-Niederalkyl-, z. B. N-Propylimino.

Die Cyclisierung erfolgt in bekannter Weise, beispielsweise in Gegenwart von Katalysatoren, wie sauren Katalysatoren. Solche sind beispielsweise Mineralsäuren, wie Schwefelsäure oder Polyphosphorsäure, Mineralsäurehalogenide, wie Sulfurylchlorid oder Phosphorhalogenide, z. B. Phosphorpentachlorid, oder organische Sulfonsäuren, wie Benzol-, p-Toluol- oder Methansulfonsäure. Dabei arbeitet man erforderlichenfalls in einem der oben erwähnten inerten Lösungs- bzw. Verdünnungsmittel, vorzugsweise unter Erwärmen, z. B. im Temperaturbereich von etwa 20° bis etwa 200 °C, in einem geschlossenen Gefäss und/oder unter Inertgas, z. B. Stickstoff.

In einer vorteilhaften Ausführungsform des vorstehend beschriebenen Verfahrens geht man von Verbindungen der Formel IVa aus und führt die Cyclisierung zu Verbindungen der Formel II und die Abspaltung von H—$Z_1$ aus den Verbindungen der Formel II ohne Isolierung der Zwischenprodukte *in situ* durch.

Eine besonders vorteilhafte Ausführungsform des vorstehenden, über Verbindungen der Formel II verlaufenden Verfahrens besteht beispielsweise darin, dass man Verbindungen der Formel

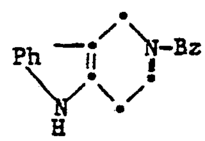

(IVd)

worin $B_z$ ein gegebenenfalls substituierter α-Phenylniederalkylrest, vorzugsweise Benzyl bedeutet, insbesondere mit Benzylbromid quaternisiert, mit Hilfe von Cyaniden, wie Alkalimetallcyaniden, z. B. Natriumcyanid, die Bindung am quartären Stickstoff spaltet und in einer erhaltenen Verbindung der Formel IVe

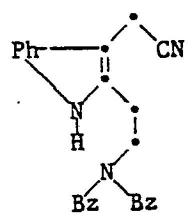

(IVe)

die Cyanogruppe wie gewünscht solvolysiert, die Benzylgruppen hydrogenolytisch in Gegenwart eines Hydrierungskatalysators, z. B. von Palladium, abspaltet und die nunmehr freie Aminoverbindung mit einer Verbindung der Formel

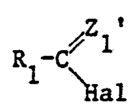 ,

worin $Z_1$' gegebenenfalls funktionell abgewandeltes Oxo und Hal Halogen bedeutet, umsetzt und schliesslich mittels eines Cyclisierungsmittels, vorzugsweise eines Mineralsäurehalogenids, wie Phosphoroxychlorid oder Phosphorchlorid, zu einer Verbindung der Formel II umsetzt.

Die Verbindungen der Formel I bzw. Salze davon lassen sich ferner herstellen, indem man Verbindungen der Formel

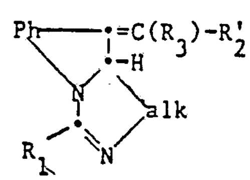

(V)

worin $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl bedeutet, oder Salze davon isomerisiert und gewünschtenfalls eine so erhältliche freie Verbindung der Formel I in eine andere freie Verbindung bzw. in ein Salz oder ein verfahrensgemäss erhältliches Salz in die freie Verbindung bzw. in ein anderes Salz überführt.

Die Isomerisierung von Verbindungen der Formel V zu Verbindungen der Formel I erfolgt in üblicher Weise, erforderlichenfalls mit Hilfe von Säuren, wie Mineralsäuren, z. B. Schwefelsäure, von Basen, wie von Alkalimetallhydroxiden bzw. -carbonaten, z. B. Natriumhydroxid bzw. Kaliumcarbonat, oder wie von organischen Aminen, z. B. tertiären Aminen, wie Pyridin, oder durch Zufuhr von Energie, wie bei Temperaturen ab 100 °C, gegebenenfalls in Gegenwart eines katalytischen Mittels, wie eines Borates oder Phosphates, z. B. eines Alkalimetallborates oder -phosphates, und, wenn erforderlich, in einem Lösungs- bzw. Verdünnungsmittel, in einem geschlossenen Gefäss und/oder unter Inertgas, z. B. Stickstoff.

Inerte Lösungs- bzw. Verdünnungsmittel sind gegebenenfalls substituierte Kohlenwasserstoffe, wie gegebenenfalls halogenierte aliphatische oder aromatische Kohlenwasserstoffe, z. B. Chloroform oder Chlorbenzol, Ether, wie aliphatische, cycloaliphatische oder aromatische Ether, z. B. Diethylether, Dioxan, Diphenylether oder Anisol, Ketone, wie aliphatische Ketone, z. B. Aceton oder Methylethylketon, Amide, wie Dialkylamide, z. B. Dimethylformamid, oder Sulfoxide, wie Diniederalkylsulfoxide, z. B. Dimethylsulfoxid.

Ausgangsstoffe der Formel V bzw. Salze davon können nach an sich bekannten Methoden hergestellt werden, beispielsweise durch Umsetzung von Verbindungen der Formel

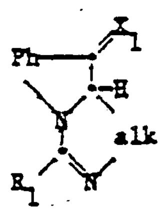

(VIa)

worin $X_1$ Oxo bzw. Thioxo bedeutet, mit Verbindungen der Formel $P(Z_2)_3{=}C(R_3){-}R_2'$ bzw. $X_1{=}P(Z_3)_2{-}CH(R_3){-}R_2'$, welche sowohl als Phosphonium-Ylide als auch als Phosphorane vorliegen können, und worin $X_1$ Oxo oder Thioxo, $Z_2$ Alkyl und/oder Phenyl, $Z_3$ Alkyl und/oder Phenyl bzw. Alkoxy und/oder Phenoxy und $R_3$ Wasserstoff oder Niederalkyl bedeutet. Ausgangsstoffe der Formel V bzw. deren Salze lassen sich z. B. ebenfalls herstellen durch Umsetzung von Verbindungen der Formel

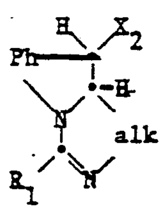

(VIb)

worin $X_2$ eine Gruppe der Formel $-C(R_3){=}X_2'$ und $X_2'$ gegebenenfalls funktionell abgewandeltes Oxo bedeutet, mit Cyanwasserstoff oder einem Salz, z. B. einem Alkalimetallsalz davon. Nach sich gegebenenfalls anschliessender Solvolyse spaltet man aus so erhältlichen Zwischenprodukten der Formel

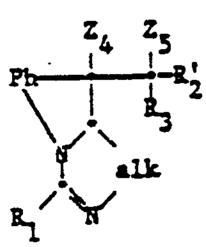

(VII)

worin $Z_4$ gegebenenfalls in Salzform vorliegendes Hydroxy bzw. Thio und $Z_5$ ein Rest der Formel $-P^{\oplus}(Z_2)_3$ bzw. $-P^{\oplus}(Z_3)_2{-}O^{\ominus}$, $Z_4$ Wasserstoff und $Z_5$ Hydroxy bzw. Mercapto bedeuten, eine Verbindung der Formel $Z_4$-$Z_5$ ab.

Alkoxy ist beispielsweise Niederalkoxy, wie Methoxy, Ethoxy, Propyloxy oder ein Butyloxy. Gegebenenfalls funktionell abgewandeltes Oxo bedeutet Oxo, Thioxo oder gegebenenfalls durch Niederalkyl oder Phenyl substituiertes Imino.

In einer vorteilhaften Ausführungsform des oben stehend beschriebenen Verfahrens zur Herstellung von Verbindungen der Formel I, beispielsweise ausgehend von Verbindungen der Formel VII, kann die Herstellung von Verbindungen der Formel V, und die Isomerisierung *in situ* erfolgen.

Die Abspaltung von $Z_4$-$Z_5$ erfolgt in üblicher Weise, beispielsweise durch Energiezufuhr, z. B. einer Reaktionstemperatur von etwa 50° bis etwa 200 °C, oder in Gegenwart eines katalytischen Mittels. Solche sind beispielsweise basische bzw. saure Katalysatoren, wobei als Basen beispielsweise Alkalimetallhydroxide, -amide, -carbonate bzw. -hydride, wie Kaliumhydroxid, Natriumamid, Kaliumcarbonat bzw. Natriumhydrid, Metalloxide, wie Aluminiumoxid, oder organische Stickstoffbasen, wie tertiäre Amine, z. B. Pyridin, Chinolin oder N,N-Dimethylanilin, und als saure Katalysatoren beispielsweise Mineralsäuren, wie Schwefelsäure, Hydrogensulfate, wie Alkalimetallhydrogensulfate, z. B. Kaliumhydrogensulfat, Polyphosphorsäure, Mineralsäureanhydride, wie Phosphorpentoxid, oder Mineralsäurehalogenide, wie Schwefelsäurehalogenide, z. B. Sulfurylchlorid, eingesetzt werden.

Bei der Verfahrensweise zur Bildung von Ausgangsstoffen der Formel V arbeitet man erforderlichenfalls in Gegenwart eines inerten Lösungs- bzw. Verdünnungsmittels, in einem geschlossen Gefäss und/oder unter Inertgas, z. B. Stickstoff.

Verbindungen der Formeln VIa oder VIb ihrerseits lassen sich nach an sich bekannten, analogen Verfahren herstellen, beispielsweise indem man Verbindungen der Formeln

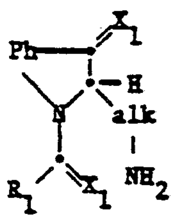 (VId) bzw. 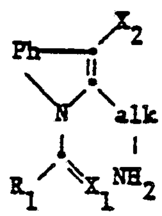 (VIe)

in Gegenwart eines Kondensationsmittels kondensiert. Als Kondensationsmittel eignen sich beispielsweise Säuren, wie Mineralsäuren, z. B. Schwefelsäure, Polyphosphorsäure, Halogenwasserstoff, z. B. Chlorwasserstoffsäure, oder Phosphorsäurehalogenide, wie Phosphoroxychlorid oder Phosphortrichlorid.

Phosphorane der Formel $P(Z_2)_3{=}C(R_3){-}R_2'$ bzw. deren Phosphonium-Ylide können nach an sich bekannten Methoden hergestellt werden, beispielsweise durch Umsetzung von Phosphinen der Formel $P(Z_2)_3$ mit quartären Ammoniumbasen der Formel $R_2'{-}CH(R_3){-}N^{\oplus}(alk')_3B^{\ominus}$, worin alk' ein Alkylrest, wie ein Niederalkylrest, und $B^{\ominus}$ ein Anion, wie Halogenid- oder ein Hydroxylanion, bedeutet, und durch anschliessende Reaktion mit starken Basen, wie Alkalimetallorganylen, z. B. Butyllithium oder Phenyllithium. Zu den entsprechenden quartären Ammoniumbasen gelangt man ebenfalls durch Reaktion von Phosphinen $P(Z_2)_3$ mit bekannten Verbindungen der Formel $R_2{-}Hal$ in Gegenwart von Basen, wie Alkalimetallhydroxiden, -niederalkanolaten, -hydriden oder -amiden, z. B. Natriumhydroxid, Natriummethylat, Kaliumhydrid oder Kaliumamid.

Verbindungen der Formel $X_1{=}P(Z_3)_2{-}CH(R_3){-}R_2'$ lassen sich beispielsweise herstellen, indem man Verbindungen der Formel $P(Z_3)_3$, worin $Z_3$ Alkoxy oder Phenoxy bedeutet, mit Verbindungen der Formel $Hal{-}CH(R_3){-}R_2'$, worin Hal Halogen bedeutet, umsetzt.

Verbindungen der Formel VId sind erhältlich durch Acylierung von Verbindungen der Formel

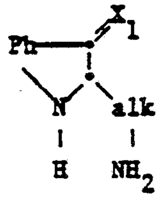 (VIg)

mit Verbindungen der Formel

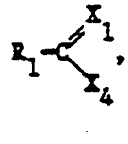

worin $X_4$ Halogen oder Acyl bedeutet.

Die Verbindungen der Formel I bzw. Salze davon können ferner hergestellt werden, indem man beispielsweise eine Verbindung der allgemeinen Formel

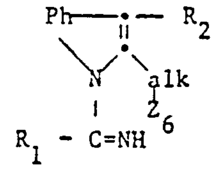

(VIII)

worin $Z_6$ gegebenenfalls funktionell abgewandeltes Hydroxy bzw. Mercapto oder Amino bedeutet, bzw. ein Salz davon cyclisiert und gewünschtenfalls eine so erhältliche freie Verbindung der Formel I in eine andere freie Verbindung bzw. in ein Salz oder ein verfahrensgemäss erhältliches Salz in die freie Verbindung bzw. in ein anderes Salz überführt.

Funktionell abgewandeltes Hydroxy bzw. Mercapto ist beispielsweise durch Niederalkanol, wie Methanol oder Ethanol, oder einen gegebenenfalls substituierten aromatischen Alkohol, wie Phenol, verethertes Hydroxy bzw. Mercapto oder mit einem geeigneten Anhydrid, wie Acetanhydrid, mit einer organischen Säure, wie Sulfonsäure, z. B. Niederalkyl- oder gegebenenfalls substituierte Arylsulfonsäure, wie Methansulfonsäure oder p-Toluolsulfonsäure, oder mit einer anorganischen Säure, wie einer Mineralsäure, z. B. Halogenwasserstoffsäure, wie Chlorwasserstoffsäure, verestertes Hydroxy bzw. Mercapto, und bedeutet beispielsweise Niederalkoxy, wie Methoxy, oder gegebenenfalls substituiertes Aryloxy, wie Phenoxy, Niederalkylthio, wie Methylthio, bzw. Niederalkanoyloxy, wie Acetyloxy, Niederalkansulfonyloxy oder gegebenenfalls substituiertes Arylsulfonyloxy, wie Methansulfonyloxy, oder p-Toluolsulfonyloxy, oder Halogen, wie Chlor oder Brom.

Die Cyclisierung erfolgt in an sich bekannter Weise, beispielsweise in Gegenwart eines Kondensationsmittels, wie eines sauren Kondensationsmittels. Hierunter fallen beispielsweise Säuren, wie Mineralsäuren, z. B. Schwefelsäure oder Polyphosphorsäure, Mineralsäurehalogenide, wie Phosphorsäurehalogenide, z. B. Phosphoroxichlorid, Phosphortribromid oder Phosphorpentachlorid. Die Reaktion wird erforderlichenfalls in einem Lösungs- bzw. Verdünnungsmittel bei einem Temperaturbereich von etwa 20° bis 200 °C, in einem geschlossenen Gefäss und/oder unter Inertgas, z. B. Stickstoff, durchgeführt.

Inerte Lösungs- bzw. Verdünnungsmittel sind gegebenenfalls substituierte Kohlenwasserstoffe, wie aliphatische oder aromatische Halogenkohlenwasserstoffe, z. B. Chloroform oder Chlorbenzol, gegebenenfalls gemischte Ether, wie aliphatische, cycloaliphatische oder aromatische Ether, z. B. Diethylether, Dioxan, Diphenylether oder Anisol, Ketone, wie aliphatische Ketone, wie Aceton oder Methylethylketon, Amide wie Dialkylamide, z. B. Dimethylformamid, oder Sulfoxide, wie Niederalkylsulfoxide, z. B. Dimethylsulfoxid.

Die Ausgangsstoffe der Formel VIII können mittels an sich bekannter Verfahrensweise hergestellt werden, beispielsweise indem man in Verbindungen der Formel

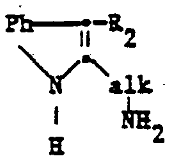

(IX)

die primäre Aminogruppe mittels Alkalimetallnitriten in Gegenwart von Säuren durch Hydroxy substituiert, dieses gegebenenfalls reaktionsfähig verestert, den Indolstickstoff mit einer Verbindung der Formel $R_1$—COOH bzw. einem funktionell abgewandelten Derivat davon acyliert und anschliessend mit Ammoniak das entsprechende Amidin bildet.

Die Acylierung des Indolstickstoffs erfolgt nach an sich bekannter Weise, beispielsweise durch Umsetzung mit gegebenenfalls funktionell abgewandelten Carboxy-Derivaten, wie Säuren, Säureanhydriden oder aktivierten Estern. Anhydridisiertes Carboxy ist dabei z. B. mit anorganischen Säuren, wie Halogenwasserstoffsäure, mit Stickstoffwasserstoffsäure, mit Cyanwasserstoffsäure oder mit organischen Säuren, wie gegebenenfalls durch Halogen substituierte Niederalkansäuren, z. B. Essigsäure, anhydridisiert. Hierunter fallen z. B. Säurehalogenide, z. B. Säurechloride, entsprechende Säureazide, Säurenitrile oder Acyloxycarbonyl.

Die Acylierung mit einer Verbindung der Formel $R_1$—COOH bzw. einem gegebenenfalls funktionell abgewandelten Derivat davon erfolgt in üblicher Weise. Ausgehend von einem Anhydrid, insbesonders einem Säurehalogenid, wird sie bevorzugt in Anwesenheit einer starken Base, beispielsweise eines Alkalimetallhydrids, z. B. Natriumhydrid, eines Alkalimetallamids, z. B. Natriumamid, oder eines Alkalimetallalkoholats, z. B. Kaliummethylat, durchgeführt.

Die Acylierung wie die anschliessende Umsetzung mit Ammoniak wird z. B. in einem inerten Lösungsmittel vorgenommen, wie in einem alkylierten Amid, z. B. N,N-Dimethylformamid, einem gegebenenfalls halogenierten Kohlenwasserstoff, z. B. Chloroform oder Chlorbenzol, oder einem Nitril, z.

B. Acetonitril, oder einer Mischung davon, erforderlichenfalls bei erniedrigter oder erhöhter Temperatur und/oder in einer Inertgasatmosphäre.

Die Verbindungen der Formel IX lassen sich ihrerseits nach an sich bekannten Verfahren herstellen, beispielsweise analog der Fischer'schen Indolsynthese durch Behandlung von Phenylhydrazonen bzw. entsprechend 1,3-substituierter 4-Piperidone mit Säuren, wie mit äthanolischer Salzsäure, oder durch Acylierung und Kondensation von entsprechend substituierten α-Hydroxyketonen mit gegebenenfalls substituierten Anilinen.

Die Verbindungen der Formel IX lassen sich ausserdem in einer vorzugsweise Ausführungsform herstellen, indem man beispielsweise von Verbindungen der Formel

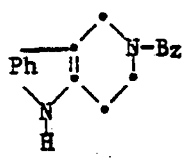

(IVd)

ausgeht, worin $B_z$ ein gegebenenfalls substituierter α-Phenylniederalkylrest, vorzugsweise Benzyl, bedeutet, insbesondere mit Benzylbromid, quaternisiert und mittels eines Nucleophils, vorzugsweise mit Cyaniden, die Bindung am so erhaltenen quartären Stickstoff spaltet und in einer als Zwischenstufe erhältlichen Verbindung der Formel

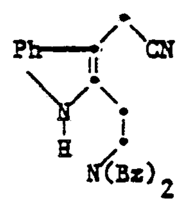

(IVe)

die Cyanogruppe wie gewünscht solvolysiert und die Benzylgruppen beispielsweise hydrogenolytisch in Gegenwart eines Hydrierungskatalysators, z. B. von Palladium, abspaltet.

Die Verbindungen der allgemeinen Formel I bzw. Salze davon sind ferner herstellbar, indem man beispielsweise in einer Verbindung der Formel

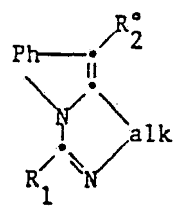

(X)

worin $R_2°$ eine Gruppe der Formel —$CH(R_3)$—$R_2''$ bedeutet und wobei $R_3$ Wasserstoff oder Niederalkyl und $R_2''$ von $R_2'$ verschiedenes funktionell abgewandeltes Carboxy, eine Gruppe der Formel —C(=O)—$N_2^{\oplus}B^{\ominus}$, wobei $B^{\ominus}$ das Anion einer Mineralsäure, z. B. Chlorid, Bromid oder Tetrafluoroborat, ist, oder gegebenenfalls bis zur Stufe des Formyl oxidiertes Methyl bedeutet, oder Salzen davon $R_2°$ solvolytisch oder oxidativ in die Gruppe $R_2$ überführt, und gewünschtenfalls eine so erhältliche freie Verbindung der Formel I in eine andere freie Verbindung bzw. in ein Salz oder ein verfahrensgemäss erhältliches Salz in die freie Verbindung bzw. in ein anderes Salz überführt. Funktionell abgewandelte und von $R_2'$ verschiedene, funktionell abgewandelte Carboxy-Verbindungen sind beispielsweise gegebenenfalls funktionell abgewandelte Orthoestergruppen, wie Trihalogen-, Halogendiniederalkoxy- bzw. Triniederalkoxymethylgruppen, anhydridisiertes Carboxy, wie Cyano, eine Gruppe der Formel =C=O, Cyano-, Azido- oder Halogencarbonyl, Acyloxycarbonyl, wie Acetyloxycarbonyl oder Derivate von Carboxy der Formel $R_2'$ oder $R_2''$, in denen Oxo gegebenenfalls durch Thio bzw. gegebenenfalls substituiertes Imino ersetzt ist, wie gegebenenfalls verestertes Thiocarboxy, wie Niederalkylthiocarboxy, z. B. Ethylthiocarboxy, amidiertes Thiocarboxyl, Iminoester, wie Imid- bzw. Amidhalogenidgruppierungen, z. B. Iminochlor- oder Aminodichlormethyl, Iminoethergruppierungen, wie Niederalkyl- oder Niederalkyleniminoethergruppierungen, z. B. Methoxy- oder Ethoxyiminomethylen, oder Amidinogruppen, wie Amidino oder Niederalkyl-, z. B. Methylamidino.

Bis zur Stufe des Formyl oxidiertes Methyl bzw. funktionell abgewandelte Gruppen davon sind beispielsweise gegebenenfalls reaktionfähig verestertes oder verethertes Hydroxymethyl bzw. gegebenenfalls funktionell abgewandeltes Formyl, wie Hydroxymethyl, Mono- oder Dihalogenmethyl, Niederalkoxymethyl, Formyl oder Formimino.

Funktionell abgewandelte Carboxy-Verbindungen, wie gegebenenfalls funktionell abgewandelte Orthoester, anhydridisiertes Carboxy oder Acyloxycarbonyl, lassen sich direkt oder in mehreren Solvolyseschritten zu freiem, verestertem oder amidiertem Carboxy solvolysieren.

Die Solvolyse von $R_2''$ erfolgt in bekannter Weise, beispielsweise durch Hydrolyse mit Wasser, durch Ammonolyse mit Ammoniak, durch Aminolyse mit einem gewünschten primären oder sekundären Amin oder durch Alkoholyse mit einem entsprechenden Alkohol. Dabei wird erforderlichenfalls in Gegenwart eines Katalysators, in einem Lösungs- bzw. Verdünnungsmittel, in einem geschlossenen Gefäss, in einem Temperaturbereich von etwa 0° bis etwa 150 °C und/oder unter Inertgas, z. B. Stickstoff, gearbeitet.

Katalysatoren sind beispielsweise basische Kondensationsmittel, wie Alkalimetall- oder Erdalkalimetallhydroxide, z. B. Natrium-, Kalium- oder Calciumhydroxid, oder tertiäre organische Amine, wie Pyridin oder Trialkylamine, z. B. Triethylamin, oder saure Hydrolysemittel, wie Mineralsäuren, z. B. Halogenwasserstoffsäuren, wie Chlorwasserstoffsäure, oder organische Carbon- oder Sulfonsäuren, wie Niederalkancarbonsäuren oder gegebenenfalls substituierte Benzolsulfonsäuren, z. B. Essigsäure oder p-Toluolsulfonsäure.

Gegebenenfalls bis zur Stufe des Formyl oxidiertes Methyl, wie Methyl, Hydroxymethyl oder Formyl, bzw. funktionell abgewandelte Derivate davon, wie Halogenmethyl, z. B. Chlormethyl, Mercaptomethyl, Thioformyl oder gegebenenfalls substituiertes Formimino, lassen sich beispielsweise direkt oder über mehrere Oxidationsschritte, gegebenenfalls über Hydroxymethyl oder Formyl, zu Carboxy oxidieren. Veretheres Hydroxymethyl, vorzugsweise Niederalkoxymethyl, z. B. Ethoxymethyl, wird in Gegenwart eines Oxidationsmittels zu verestertem Carboxy, insbesondere Niederalkoxycarbonyl, oxidiert. Die Umsetzung von Formyl zu Carbamoyl wird beispielsweise mit Hilfe einer Aminoverbindung in Gegenwart eines Oxidationsmittels, wie eines Uebergangsmetalloxides, z. B. Mangandioxid, und erforderlichenfalls in Gegenwart eines Nucleophils, insbesondere eines Cyanids, durchgeführt.

Oxidation von $R_2''$ wird auf üblichem Wege durchgeführt, beispielsweise unter Verwendung der üblichen Oxidationsmittel. Solche sind z. B. gegebenenfalls katalytisch angeregter Sauerstoff, Alkalimetallsalze von Chromaten bzw. Manganaten, wie Natriumchromat oder Kaliumpermanganat, oder Uebergangsmetalloxide, wie Mangandioxid oder Chromtrioxid. Dabei arbeitet man erforderlichenfalls in einem inerten Lösungsmittel, in einem geschlossenen Gefäss und/oder unter Kühlen oder Erwärmen, z. B. bei etwa 0° bis etwa 150 °C.

Die Ausgangsstoffe der Formel X können nach analogen Verfahren hergestellt werden, beispielsweise indem man Verbindungen der Formel

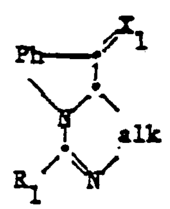

(VIa)

worin $X_1$ Oxo bzw. Thioxo bedeutet, mit Verbindungen der Formel $P(Z_2)_3—R_2°$, welche sowohl als Phosphonium-Ylide als auch als Phosphorane vorliegen können, bzw. $X_1{=}P(Z_3)_2—R_2°$, und worin $Z_2$ Alkyl und/oder Phenyl und $Z_3$ Alkyl und/oder Phenyl bzw. Alkoxy, wie Niederalkoxy, und/oder Phenoxy und $R_2''$ von $R_2'$ verschiedenes funktionell abgewandeltes Carboxy, eine Gruppe der Formel $—C(=O)N_2^{\oplus}B^{\ominus}$ oder gegebenenfalls bis zur Stufe des Formyl oxidiertes Methyl bedeutet, umsetzt, aus gegebenenfalls so erhältlichen Zwischenprodukten der Formel

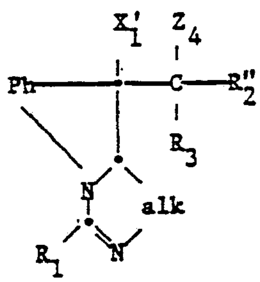

(VIh)

worin $X_1'—O^{\ominus}$ bzw. $—S^{\ominus}$ und $Z_4$ einen Rest der Formel $—P^{\oplus}(Z_2)_3$ bzw. $—P^{\oplus}(X_1')(Z_3)_2$ bedeutet, eine Verbindung der Formel $X_1{=}P(Z_2)_3$ bzw. $X_1{=}P(X_1')(Z_3)_2$ abspaltet und eine so erhaltene Verbindung zu einer Verbindung der Formel X isomerisiert.

Die Umsetzung erfolgt üblicherweise in einem inerten Lösungsmittel, beispielsweise einem gegebenenfalls halogenierten Kohlenwasserstoff, wie einem Aromaten, z. B. Benzol oder Toluol, einem Ether, wie Tetrahydrofuran oder Dioxan, oder einem Amid, z. B. Dimethylformamid, in einem Temperaturbereich

von etwa 20° bis etwa 150 °C und/oder gegebenenfalls in Gegenwart eines Katalysators, wie einer Base, z. B. eines Alkalimetallalkoholats, wie Kalium-tert.-butylat.

In einer bevorzugten Ausführungsform gelangt man zu den Ausgangsstoffen der Formel X, worin $R_2°$ ein solvolytisch oder oxidativ in $R_2$ überführbarer Rest ist, und geht beispielsweise von Verbindungen der Formel

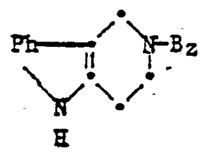

(IVd)

aus, worin $B_z$ ein gegebenenfalls substituierter α-Phenylniederalkylrest, vorzugsweise Benzyl, bedeutet, quartärniert das tertiäre Stickstoffatom, insbesondere mit Benzylchlorid, spaltet die Bindung am quartären Stickstoffatom mit Hilfe einer starken Base, wie mit Cyaniden, z. B. Natriumcyanid, und überführt in einer so erhaltenen Verbindung der Formel

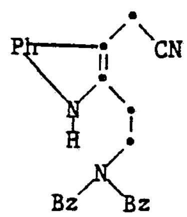

(VIe)

die Cyanogruppe in $R_2°$, beispielsweise durch Solvolyse zu Carboxy oder Niederalkoxycarbonyl und anschliessender Reduktion zu Hydroxymethyl bzw. Niederalkoxymethyl, spaltet die Bz-Gruppen hydrogenolytisch in Gegenwart eines Hydrierungskatalysators ab. Anschliessend setzt man die so erhaltene Verbindung mit einer Verbindung der Formel

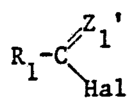
,

worin $Z_1'$ gegebenenfalls funktionell abgewandeltes Oxo und Hal Halogen bedeuten, um und führt die Cyclisierung zu entsprechenden Verbindungen der Formel X in Gegenwart eines üblichen Cyclisierungsmittels, wie Mineralsäurehalogenids, z. B. Phosphoroxychlorid, durch.

Die Verbindungen der Formel I, worin $R_2$ amidiertes 1-Carboxymethyl der Formel $—CH(R_3)—R_2'$ ist, in der $R_2'$ gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, kann man ferner herstellen, durch Addition von Wasser und Abspaltung eines Protons an Verbindungen der Formel

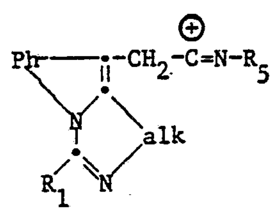

(XIa)

welche *in situ* durch Umlagerung von Verbindungen der Formel

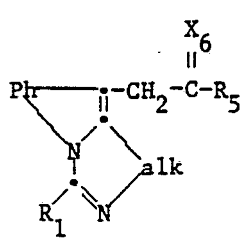

(XIb)

entstanden sind, worin $R_5$ gegebenenfalls substituiertes Niederalkyl bzw. Phenyl und $X_6$ ein Rest der Formel =N—OH bzw. =N—N$^{\oplus}$≡N1 darstellt.

Die Umsetzung erfolgt in üblicher Weise, beispielsweise in Gegenwart eines sauren Katalysators, wie einer Mineralsäure, z. B. Schwefelsäure oder Phosphorsäure, und gegebenenfalls unter Erwärmen.

Die Ausgangsstoffe der Formel XIb lassen sich nach analogen Verfahren herstellen, beispielsweise durch Umsetzung von Verbindungen der Formel

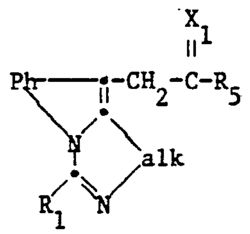

(XIc)

worin $X_1$ Oxo oder Thioxo bedeutet, mit Hydroxylamin oder einem Säureadditionssalz davon oder mit einem Azid, wie einem Alkalimetallazid, z. B. Natriumazid.

Verbindungen der Formel I, worin $R_2$ verestertes oder amidiertes 1-Carboxyniederalkyl der Formel —CH($R_3$)—$R_2'$ ist, in der $R_2'$ gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet und alk Vinylen bedeutet, werden beispielsweise hergestellt, indem man entsprechende Verbindungen der Formel

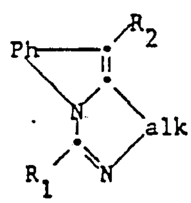

(I)

worin alk Ethylen bedeutet, unter Abspaltung von Wasserstoff bei gleichzeitiger Bildung einer zusätzlichen Bindung dehydriert und gewünschtenfalls eine erfindungsgemäss erhältiche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

Die Dehydrierung erfolgt in an sich bekannter Weise, insbesondere bei erhöhter Temperatur, beispielsweise in einem Temperaturintervall von etwa 100° bis etwa 300 °C, und unter Verwendung eines Dehydrierungsmittels. Als solche Mittel kommen beispielsweise Dehydrierungskatalysatoren, z. B. Nebengruppenelemente, vorzugsweise der Nebengruppe VIII, wie Palladium oder Platin, oder deren Salze, wie Ruthenium-triphenyl-phosphid-chlorid, in Betracht, wobei die Katalysatoren gegebenenfalls auf geeignetem Trägermaterial, wie Kohle, Aluminiumoxid oder Siliziumdioxid, aufgezogen sind. Weitere Dehydrierungsmittel sind beispielsweise Chinone, wie p-Benzochinone, z. B. Tetrachlor-p-benzochinon oder 2,3-Dichlor-5,6-dicyano-p-benzochinon, oder wie Anthrachinone, z. B. Phenanthren-9,10-chinon. Die Umsetzung wird in einem inerten, gegebenenfalls hochsiedenden Lösungsmittel, wie in einem Ether, z. B. Diphenylether, erforderlichenfalls unter Druck, in einem geschlossenen Gefäss und/oder unter Inertgas, z. B. Stickstoff durchgeführt.

Die als Ausgangsstoffe zu verwendenden Verbindungen der Formel I können nach den vorstehend beschriebenen Verfahren hergestellt werden.

Eine erfindungsgemäss erhältliche Verbindung der Formel I kann in an sich bekannter Weise in eine andere Verbindung der Formel I umgewandelt werden.

Enthält die Gruppe $R_2$ ein freies Carboxy, so lässt sich dieses nach an sich bekannten Veresterungsmethoden in entsprechend verestertes Carboxy überführen, beispielsweise indem man gegebenenfalls reaktionsfähiges abgewandeltes Carboxy oder ein Salz davon durch Alkoholyse mit einem gewünschten Alkohol, beispielsweise einem reaktionsfähigen Derivat davon oder einem davon abgeleiteten Olefin, umsetzt oder durch Alkylierung mit Diazoniederalkan.

Geeignete reaktionsfähige funktionelle Carboxyderivate sind beispielsweise Anhydride, wobei als Anhydride insbesondere gemischte Anhydride, z. B. solche mit anorganischen Säuren, wie Halogenwasserstoffsäuren, z. B. Chlorwasserstoffsäure, wie Stickstoffwasserstoff- oder Cyanwasserstoffsäuren oder mit organischen Carbonsäuren, wie Niederalkansäuren, z. B. Essigsäure, verwendet werden.

Reaktionsfähige Derivate eines Alkohols sind beispielsweise Carbon-, Phosphorig-, Schweflig- oder

Kohlensäureester, z. B. Niederalkancarbonsäureester, Triniederalkylphosphit, Diniederalkylsulfit oder Pyrocarbonat, oder Mineral- bzw. Sulfonsäureester, z. B. Chlor-, Brom- oder Schwefelsäureester, Benzol-, Toluol- oder Methansulfonsäureester, des betreffenden Alkohols.

Die Veresterung von freiem Carboxy wird in Gegenwart eines Kondensationsmittels durchgeführt. Als katalytisch wasserabspaltende Mittel für die Veresterung mit Alkoholen kommen z. B. Säuren, beispielsweise Protonsäuren, wie Chlorwasserstoff-, Bromwasserstoff-, Schwefel-, Phosphor-, Bor-, Benzolsulfon- und/oder Toluolsulfonsäure, bzw. Lewissäuren, wie Bortrifluorid-Etherat, in Frage. Uebliche wasserbindende Kondensationsmittel sind z. B. durch Kohlenwasserstoffreste substituierte Carbodiimide, beispielsweise N,N'-Diethyl-, N,N-Dicyclohexyl- oder N-Ethyl-N'-(3-dimethylaminopropyl)-carbodiimide. Kondensationsmittel für die Veresterung mit reaktionsfähigen Estern sind z. B. basische Kondensationsmittel, wie anorganische Basen, z. B. Alkalimetall- oder Erdalkalimetallhydroxide bzw. -carbonate, wie Natrium-, Kalium- oder Calciumhydroxid bzw. -carbonat, wie organische Stickstoffbasen, z. B. tertiäre organische Amine, wie Triethylamin oder Pyridin. Die Veresterung wird vorteilhaft mit einem Ueberschuss an eingesetztem Alkohol durchgeführt. Dabei arbeitet man vorzugsweise in einem wasserfreien Medium, erforderlichenfalls in Gegenwart eines inerten Lösungsmittels, wie in halogenierten Kohlenwasserstoffen, z. B. Chloroform oder Chlorbenzol, in Ethern, z. B. Tetrahydrofuran oder Dioxan.

Die Umsetzung mit einem Olefin kann beispielsweise in Gegenwart eines sauren Katalysators, z. B. einer Lewissäure, z. B. von Bortrifluorid, einer Sulfonsäure, z. B. von p-Toluolsulfonsäure, oder vor allem eines basischen Katalysators, z. B. von Natrium oder Kaliumhydroxid, vorteilhaft in einem inerten Lösungsmittel, wie einem Ether, z. B. in Diethylether oder Tetrahydrofuran, erfolgen.

Weiterhin kann man freies Carboxy bzw. reaktionsfähig funktionelle Carboxyderivate durch Solvolyse mit Ammoniak oder einem primären bzw. sekundären Amin, wobei auch Hydroxylamine bzw. Hydrazine eingesetzt werden können, in üblicher Weise unter Dehydratisierung, gegebenenfalls in Anwesenheit eines Kondensationsmittels, in eine gewünschte amidierte Form überführen. Als Kondensationsmittel werden vorzugsweise Basen verwendet, beispielsweise anorganische Basen, wie Alkalimetallhydroxide, z. B. Natrium- oder Kaliumhydroxid, organische Stickstoffbasen, wie tert.-Amine, z. B. Pyridin, Tributylamin oder N-Dimethylanilin, oder Tetrahalogensilane, wie Tetrachlorsilan.

Ferner kann man erfindungsgemäss enhältliche Verbindungen der Formel I, worin $R_2$ als Substituenten verestertes Carboxy enthält, in üblicher Weise umestern, beispielsweise durch Reaktion mit einem entsprechenden Alkohol bzw. einem Metallsalz davon, wie einem Alkalimetallsalz, z. B. dem Natrium- oder Kaliumsalz, erforderlichenfalls in Gegenwart eines Katalysators, beispielsweise einer starken Base, wie eines Alkalimetallhydroxides, -amides oder -alkoholates, z. B. Kaliumhydroxid, Natriumamid oder -methylat, oder einer starken Säure, wie einer Mineralsäure, z. B. Schwefelsäure, Phosphorsäure oder Salzsäure, oder wie einer organischen Sulfonsäure, z. B. einer aromatischen Sulfonsäure, wie p-Toluolsulfonsäure.

Verestertes Carboxy kann weiterhin nach bekannten Verfahren, z. B. durch Hydrolyse in Gegenwart eines Katalysators, in die freie Carboxygruppe überführt werden. Als Katalysatoren kommen vorzugsweise Basen, z. B. Alkalimetallhydroxide, wie Natrium- oder Kaliumhydroxid in Frage. Verestertes Carboxy lässt sich ferner in üblicher Weise, beispielsweise durch Solvolyse, gegebenenfalls in Gegenwart eines Katalysators, beispielsweise eines sauren bzw. basischen Mittels, in Carboxy, durch Ammonolyse oder Aminolyse mit Ammoniak oder mit einem primären bzw. sekundären Amin, in amidiertes Carboxy umwandeln. Als Basen finden beispielsweise Alkalimetallhydroxide, wie Natrium- bzw. Kaliumhydroxide, und als Säuren beispielsweise Mineralsäuren, wie Schwefelsäure, Phosphorsäure oder Salzsäure, Verwendung. Ebenso kann man erfindungsgemäss erhältliche Verbindungen der Formel I, worin die Gruppe $R_2$ den Substituenten amidiertes Carboxy enthält, nach an sich bekannten Methoden die Amidbindung spalten und so das Carbamoyl in freies Carboxy überführen. Hierzu arbeitet man in Gegenwart eines Katalysators, beispielsweise einer Base, wie eines Alkalimetall- oder Erdalkalimetallhydroxides oder -carbonates, z. B. Natrium-, Kalium-, oder Calciumhydroxid oder -carbonat, oder einer Säure, wie einer Mineralsäure, z. B. Salzsäure, Schwefelsäure oder Phosphorsäure.

Enthält die Gruppe $R_2$ der Formel I eine veresterte Carboxygruppe, so kann man diese beispielsweise durch übliche Solvolyse, vorteilhaft durch einen Ueberschuss an Ammoniak oder mindestens ein Wasserstoffatom enthaltendes Amin, gegebenenfalls in Gegenwart eines Katalysators, in eine amidierte Carboxygruppe überführen. Dabei verwendet man als Katalysatoren beispielsweise Säuren, wie Mineralsäuren, z. B. Salz-, Schwefel- oder Phosphorsäure, oder Basen, wie Alkalimetallhydroxide, z. B. Natrium- oder Kaliumhydroxid.

Enthält die Gruppe $R_2$ der Formel I als Substituenten amidiertes Carboxy, so kann man dieses beispielsweise durch übliche Solvolyse mit einem Alkohol in Gegenwart eines Katalysators in verestertes Carboxy überführen. Dabei verwendet man beispielsweise saure Katalysatoren, wie Mineralsäuren, z. B. Phosphorsäure, Salzsäure oder Schwefelsäure.

Falls der Substituent $R_1$ der Formel I durch Niederalkylthio substituiert ist, kann man diesen auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z. B. Perjodsäure oder Perschwefelsäure, organische Persäuren, wie entsprechende Percarbon- oder Persulfonsäuren, z. B. Perameisen-, Peressig-, Trifluorperessig- bzw. Perbenzoesäure oder p-Toluolpersulfonsäure, oder Gemische aus Wasserstoffperoxid und Säuren, z. B.

Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z. B. Essigsäure oder Trifluoressigsäure, oder Uebergangsmetalloxide, wie Oxide von Elementen der VII. Nebengruppe, z. B. Vanadium-, Molybdän- oder Wolframoxid, zu nennen sind. Die Oxidation wird unter milden Bedingungen, z. B. bei Temperaturen von – 50° bis + 100 °C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Ueberschuss ein.

Verbindungen der Formel I, worin $R_1$ einen durch Niederalkylsulfinyl bzw. -sulfonyl substituierten aromatischen Rest darstellt, lassen sich nach an sich bekannten Methoden zu den entsprechenden Niederalkylthio-Verbindungen, von Niederalkansulfonyl-Derivaten ausgehend, auch zu Niederalkan-sulfinyl reduzieren. Als Reduktionsmittel eignen sich beispielsweise katalytisch aktivierter Wasserstoff, wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Metallkationen, wie Zinn II-, Blei II-, Kupfer I-, Mangan II-, Titan II-, Vanadium II-, Molybdän III- oder Wolfram III- Verbindungen, Halogenwasserstoffe, wie Chlor-, Brom- oder Jodwasserstoff, Hydride, wie komplexe Metallhydride, z. B. Lithiumaluminium-, Natriumbor-, Tributylzinnhydrid, Phosphorverbindungen, wie Phosphorhalogenide, z. B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Sauerstoff-Komplexe, wie Jod-Pyridin-Schwefeldioxidkomplex, in Frage.

Erhaltene Salze können in an sich bekannter Weise in die freien Verbindungen umgewandelt werden, z. B. durch Behandeln mit einem sauren Reagens, wie einer Mineralsäure, bzw. einer Base, z. B. Alkalilauge.

Die neuen Verbindungen können, je nach der Wahl der Ausgangsstoffe und Arbeitsweisen, in Form eines der möglichen Isomeren oder als Gemische derselben, z. B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalischchemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes mit einer mit der racemischen Säure Salze bildenden optisch aktiven Base und Trennung der auf diese Weise erhaltenen Salze, z. B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die Verbindungen einschliesslich ihrer Salze können auch in Form ihrer Hydrate erhalten werden oder andere zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Die Ausgangsstoffe, welche in den Verfahren zur Herstellung der Endprodukte eingesetzt werden, sind neue Verbindungen.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von den Warmblüter-Spezies, dem Alter und dem individuellen Zustand, sowie von der Applikationsweise ab. Im Normalfall is für einen 75 kg schweren Warmblüter bei oraler Applikation eine ungefähre Tagesdosis von 30-300 mg, vorteilhaft in mehreren gleichen Teildosen verteilt, zu veranschlagen.

Die neuen pharmazeutischen Präparate enthalten z. B. von 10 % bis 80 %, vorzugsweise von 20 % bis 60 % des Wirkstoff. Erfindungsgemäss pharmazeutische Präparate zur enteralen bzw. parenteralen

Verabreichung sind. z. B. solche in Dosiseinheitsformen, wie Dragées, Tabletten, Kapseln oder Suppositorien, ferner Ampullen. Diese werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Tragakanth, Methylcellulose und/oder Polyvinylpyrrolidon, und/oder wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulier- und Schmiermittel, z. B. Kieselsäure, Talk, Stearinsäure oder Salze davon, wie Magnesium- oder Calciumstearat, und/oder Polyethylenglykol. Dragée-Kerne werden mit geeigneten, gegebenfalls Magensaft-resistenden Ueberzügen versehen, wobei man u. a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactone, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten ; als Grundmassenstoffe kommen z. B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z. B. eines wasserlöslichen Salzes, ferner Suspensionen des Wirkstoffes, wie entprechende ölige Injektionssuspentionen, wobei man geeignete lipophile Lösungsmittel oder Vehikel, wie fette Oele, z. B. Sesamöl, oder synthetische Fettsäureester, z. B. Ethyloleat oder Triglyceride, verwendet oder wässrige Injektionsuspentionen, welche viskositätserhöhende Stoffe, z. B. Natriumcarboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von 0,5 bis 20 % des Wirkstoffs enthalten.

Creme sind Oel-in-Wasser Emulsionen, die mehr als 50 % Wasser aufweisen. Als ölige Grundlage verwendet man in erster Linie Fettalkohole, z. B. Lauryl-, Cetyl- oder Stearylalkohol, Fettsäuren, z. B. Palmitin- oder Stearinsäure, flüssige bis feste Wachse, z. B. Isopropylmyristat, Wollwachs oder Bienenwachs, und/oder Kohlenwasserstoffe, z. B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z. B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z. B. Natriumlaurylsulfat, Natriumcetylsulfat oder Natriumstearylsulfat, die man üblicherweise in Gegenwart von Fettalkoholen, z. B. Cetylalkohol oder Stearylalkohol, verwendet. Zusätze zur Wasserphase sind u. a. Mittel, welche die Austrocknung der Creme vermindern, z. B. Polyalkohole, wie Glycerin, Sorbit, Propylenglykol und/oder Polyethylenglykole, ferner Konservierungsmittel oder Riechstoffe.

Salben sind Wasser-in-Oel-Emulsionen, die bis zu 70 %, vorzugweise jedoch von 20 % bis 50 % Wasser oder wässrige Phasen enthalten. Als Fettphase kommen in erster Linie Kohlenwasserstoffe, z. B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z. B. Cetylalkohol oder Wollwachsalkohole, bzw. Wollwachs enthalten. Emulgatoren sind entsprechende lipophile Substanzen, wie Sorbitanfettsäureester (Spans), z. B. Sorbitanoleat und/oder Sorbitanisostearat. Zusätze zur Wasserphase sind u. a. Feuchthaltungsmittel, wie Polyalkohole, z. B. Glycerin, Propylenglykol, Sorbit und/oder Polyethylenglykol, sowie Konservierungsmittel oder Riechstoffe.

Fettsalben sind wasserfrei und enthalten als Grundlage insbesondere Kohlenwasserstoff, z. B.

Paraffin, Vaseline und/oder flüssige Paraffine, ferner natürliches oder partialsynthetisches Fett, z. B. Kokosfettsäuretriglycerid, oder vorzugsweise gehärtete Oele, z. B. hydriertes Erdnuss- oder Rizinusöl, ferner Fettsäurepartialester des Glycerins, z. B. Glycerinmono- und -distearat, sowie z. B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z. B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z. B. Dichlordifluormethan und Dichlortetrafluorethan, als Treibmittel verwendet werden. Als Oelphase verwendet man u. a. Kohlenwasserstoffe, z. B. Paraffinöl, Fettalkohole, z. B. Cetylalkohol, Fettsäureester, z. B. Isopropylmyristat, und/oder andere Wachse. Als Emulgatoren verwendet man u. a. Gemische von solchen mit vorwiegend hydrophilen Eigenschaften, wie Polyoxyethylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel.

Tinkturen und Lösungen weisen meistens eine wässerigäthanolische Grundlage auf, der u. a. Polyalkohole, z. B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d. h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z. B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst ; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und der Salze von solchen Verbindungen mit salzbildenden Eigenschaften, vorzugsweise zur Behandlung von Entzündungen, in erster Linie von entzündlichen Erkrankungen des rheumatischen Formenkreises, besonders der chronischen Arthritis.

Die nachfolgenden Beispiele illustrieren die oben beschriebene Erfindung ; Temperaturen sind in Celsiusgraden angegeben.

Die den Verbindungen der Formel I zugrundeliegende Bezeichnung der Verknüpfungsstellen im Pyrimido-Indol-Ringsystem

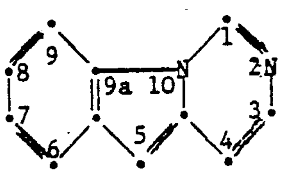

ist in der Literatur uneinheitlich.

So werden die älteren Literaturstellen die Verknüpfungsstellen im Ringsystem mit [3,4-a] bezeichnet, während neuerdings die Bezeichnung [1,6-a] verwendet wird.

Aus prinzipiellen Gründen wird nachfolgend für das vorstehend angegebene Ringgerüst folgende Nomenklatur vorgezogen :

Pyrimido [1,6-a] indol

Beispiel 1

7-Methoxy-1 (p-chlorphenyl)-3,4-dihydro-pyrimido [1,6-a] indol-5-essigsäureethylester-hydrochlorid

165 g (0,4 Mol) 2-[(p-Chlorbenzoyl-amino)-ethyl]-5-methoxy-indol-3-essigsäureethylester werden in 825 ml Phosphortrichlorid 3 Stunden unter Rückfluss zum Sieden erhitzt und anschliessend das überschüssige Phosphortrichlorid bei einer Badtemperatur von 60° unter leichtem Vacuum abdestilliert. Der Rückstand wird in 1 500 ml Methylenchlorid gelöst, mit 2 000 ml Eiswasser ausgerührt, durch Zugabe von 500 ml konz. Ammoniak alkalisch gestellt und nach kurzem Rühren die Methylenchloridphase abgetrennt. Man wäscht mit Wasser neutral, trocknet über $Na_2SO_4$ und destilliert das Methylenchlorid ab. Der Rückstand (136,7 g) wird in 120 ml Aceton und 500 ml Ether gelöst und die Lösung mit 100 ml einer etwa 4-normalen Chlorwasserstofflösung in Ether versetzt. Dabei kristallisiert das Hydrochlorid des 7-Methoxy-1-(p-chlorphenyl)-3,4-dihydro-pyrimido [1,6-a] indol-essigsäureethylesters aus.

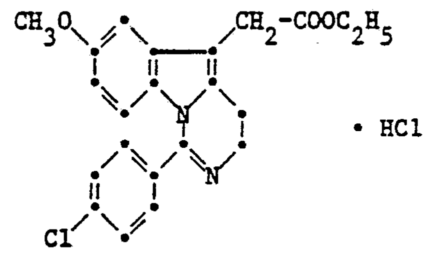

• HCl

Es wird abgesaugt und mit Aceton/Ether 1 : 10 gewaschen : gelbliche Kristalle vom Smp. 204-208°, nach Umkristallisieren aus Methanol/Aceton Smp. 205-208°.

Das Ausgangsprodukt kann wie folgt hergestellt werden :

a) 131 g (0,45 Mol) 3-Benzyl-6-methoxy-tetrahydro-γ-carbolin werden in 1 300 ml Acetonitril bei 40° unter Rühren gelöst und innerhalb von 10 Minuten 94 g (0,55 Mol) Benzylbromid zugesetzt. Nach kurzer Zeit beginnt das Benzylammoniumderivat auszukristallisieren. Es wird noch ca. 15 Stunden bei Raumtemperatur weitergerührt, danach im Eisbad gekühlt und vom Kristallisat abgesaugt. Smp. 150-151°.

b) 464 g (1 Mol) des so hergestellten N,N-Dibenzyl-6-methoxy-tetrahydro-γ-carboliminiumbromids werden in 4 250 ml Methanol unter Erwärmen auf 65° gelöst und innerhalb von 5 Minuten eine Lösung von 196 g (4 Mol) Natriumcyanid in 500 ml Wasser unter Rühren zugesetzt. Die Lösung wird 3 Stunden unter Rückfluss zum Sieden erhitzt. Beim Abkühlen kristallisiert nach Animpfen 2-(Dibenzylamino-ethyl)-3-cyano-methyl-5-methoxy-indol in farblosen Kristallen vom Smp. 102-104°.

c) 220 g (0,537 Mol) des nach b) hergestellten Nitrils werden in 300 ml abs. Ethanol gelöst und die Lösung bei – 5° mit trockenem Chlorwasserstoff gesättigt. Anschliessend wird 5 1/2 Tage bei 20° gerührt. Man lässt die ausgeschiedenen Kristalle absitzen, dekantiert von der überstehenden Lösung ab, löst den Bodensatz in 2 000 ml Eiswasser und rührt etwa 3 Stunden bei 20°. Dann wird unter Eiskühlung mit konz. Ammoniaklösung alkalisch gestellt und mit 1 500 ml Toluol Eiswasser ausgerührt. Die abgetrennte Toluolphase wird mit Wasser gewaschen, über Natriumsulfat getrocknet und über 1 000 g Aluminiumoxid (Akt. Stufe 3) filtriert und mit Toluol nachgewaschen. Nach Abdestillieren des Toluols verbleibt ein hellbraunes Oel, das ohne weitere Reinigung der Hydrierung nach d) unterworfen wird.

d) 181,3 g des nach c) erhaltenen 2-Dibenzylamino-5-methoxy-indol-3-essigsäureethylesters werden in 1 500 ml abs. Alkohol gelöst, und unter Zusatz von 18 g Pd/C (5 %) bei Normaldruck und 20-35° hydriert. Nach Aufnahme von 12 500 ml $H_2$ werden nochmals 18 g Katalysator zugesetzt und bis zum Stillstand der $H_2$-Aufnahme von insgesamt 17 300 ml weiterhydriert. Nach Abfiltrieren vom Katalysator und Nachwaschen mit Methylenchlorid wird die Lösung zur Trockne eingedampft und der Rückstand in 250 ml Ether gelöst. Nach Animpfen kristallisiert der 2-Aminoethyl-5-methoxy-indol-3-essigsäureethylester in farblosen Kristallen vom Smp. 79-80°.

e) 61,7 g (0,223 Mol) des nach d) hergestellten 2-Aminoethyl-5-methoxy-indol-3-essigsäureethylesters werden in 600 ml Methylenchlorid gelöst und die Lösung mit 150 ml 2N-Natronlauge überschichtet. Unter starkem Rühren wird bei 0-5° innerhalb 2 1/2 Stunden eine Lösung von 43 g (0,245 Mol) p-Chlorbenzoylchlorid zugesetzt und danach noch 1 Stunde ausgerührt. Die Methylenchloridphase wird dann abgetrennt, mit Wasser gewaschen, über $MgSO_4$ getrocknet und zur Trockne eingedampft. Der Rückstand kristallisiert beim Aufnehmen in Ether in farblosen Kristallen vom Smp. 136-138° 2-[(p-Chlorbenzoyl-amino)-ethyl]-5-methoxy-indol-3-essigsäureethylester.

Beispiel 2

1-Phenyl-3,4-dihydro-pyrimido [1,6-a] indol-5-essigsäure-ethylester

54 g (0,154 Mol) 2-(Benzoylamino-ethyl)-indol-3-essigsäure-ethylester werden in 250 ml Phosphoroxychlorid 3 Stunden unter Rückfluss zum Sieden erhitzt. Das überschüssige Phosphoroxychlorid wird dann bei 60° im Vacuum abdestilliert und der Rückstand mit 1 000 ml Eiswasser verrührt. Die wässrige Lösung wird durch Filtrieren über Hyflo geklärt, mit konz. Ammoniak alkalisch gestellt und mit 500 ml Ether extrahiert. Die Etherphase wird über Natriumsulfat getrocknet und zur Trockne eingeengt. Den Rückstand löst man in 200 ml Aceton und versetzt mit 25 ml einer etwa 4-normalen Chlorwasserstoff-Lösung in Ether. Nach Animpfen kristallisiert das Hydrochlorid des 1-Phenyl-3,4-dihydro-pyrimido [1,6-a] indol-5-essigsäureethylesters in gelblichen Kristallen vom Smp. 172-175°, die aus 1N Salzsäure umkristallisiert werden können (Smp. 187-189°). Die aus dem Salz freigesetzte Base schmilzt nach Umkristallisieren aus Ether bei 83-84°.

Der als Ausgangsmaterial verwendete 2-(Benzoylamino-ethyl)-indol-3-essigsäureethylester, Smp. 162-163°, kann analog Beispiel 1 a)-e) hergestellt werden.

Beispiel 3

In analoger Weise, wie in Beispiel 2 beschrieben, erhält man aus 2-[(p-Methylthio-benzoyl-amino)-ethyl]-5-fluor-indol-essigsäureethylester vom Smp. 157-158° den 7-Fluor-1-(p-methylthio-phenyl-3,4-dihydro-pyrimido [1,6-a] indol-5-essigsäureethylester vom Smp. 119-120°.

Beispiel 4

In analoger Weise erhält man aus 2-Benzoyl-aminoethyl-4,5-dimethyl-indol-3-essigsäureethylester vom Smp. 183-184° den 6,8-Dimethyl-1-phenyl-3,4-dihydro-pyrimido [1,6-a] indol-5-essigsäureethylester vom Smp. 142-143°.

Beispiel 5

In analoger Weise erhält man aus 2-[(3-Sulfamoyl-4-chlorbenzoyl-amino)-ethyl]-indol-3-essigsäureethylester vom Smp. 212-213° den 1-(3-Sulfamoyl-4-chlor-phenyl)-3,4-dihydro-pyrimido [1,6-a] indol-5-essigsäureethylester vom Smp. 227-228°.

Beispiel 6

In analoger Weise erhält man aus 2-(2,6-Dichlorbenzoyl-aminoethyl)-indol-3-essigsäureethylester vom Smp. 125-126° den 1-(2,6-Dichlorphenyl)-3,4-dihydro-pyrimido [1,6-a] indol-5-essigsäureethylester vom Smp. 92-93° (Hydrochlorid Smp. 138-142°).

Beispiel 7

In analoger Weise erhält man aus 2-(2-Picolinoyl-amino-ethyl)-indol-3-essigsäureethylester vom Smp. 117-118° den 1-(2-Picolinyl)-3,4-dihydro-pyrimido [1,6-a] indol-5-essigsäureethylester vom Smp. 113-114°, (Hydrochlorid 194-204°).

Beispiel 8

In analoger Weise erhält man aus 2-(2-Thienylamino-ethyl)-indol-3-essigsäureethylester vom Smp. 140-141° das Hydrochlorid des 1-(2-Thienyl)-3,4-dihydro-pyrimido [1,6-a] indol-5-essigsäureethylesters vom Smp. 153-157°.

Beispiel 9

7-Methoxy-1-(p-chlorphenyl)-3,4-dihydro-pyrimido [1,6-a]-indol-5-essigsäure

In eine Lösung von 550 g Kaliumhydroxid in 200 ml Wasser und 1 500 ml Methanol werden unter Rühren 117,2 g (0,27 Mol) 7-Methoxy-1-(p-chlorphenyl)-3,4-dihydro-pyrimido [1,6-a] indol-5-essigsäureethyl-ester-hydrochlorid eingetragen. Man rührt 10 Stunden bei Raumtemperatur, destilliert das Methanol im Vacuum ab, löst den Rückstand in 1 500 ml Eiswasser und versetzt die Lösung unter Eiskühlung mit 700 ml konz. Salzsäure. Man saugt vom abgeschiedenen Hydrochlorid ab und kristallisiert aus 2 300 ml 50 % Ethanol um. Dabei fällt 7-Methoxy-1-(p-chlorphenyl)-3,4-dihydro-pyrimido [1,6-a] indol-5-essigsäurehydrochlorid in Form gelblicher Kristalle vom Smp. 225-228° (unter Zersetzung) an.

Beispiel 10

In analoger Weise erhält man aus dem entsprechenden Ethylester das Hydrochlorid der 1-Phenyl-3,4-dihydro-pyrimido [1,6-a] indol-5-essigsäure vom Smp. 235-240° (Zers.).

Beispiel 11

In analoger Weise erhält man aus dem entsprechenden Ethylester das Hydrochlorid der 7-Fluor-1-(p-methylthio-phenyl)-3,4-dihydro-pyrimido [1,6-a] indol-5-essigsäure vom Smp. 220-222° (Zers.).

Beispiel 12

In analoger Weise erhält man aus dem entsprechenden Ethylester das Hydrochlorid der 7-Fluor-1-(p-methylsulfinyl-phenyl)-3,4-dihydro-pyrimido [1,6-a] indol-5-essigsäure vom Smp. 208-210° (Zers.).

Beispiel 13

In analoger Weise erhält man aus dem entsprechenden Ethylester das Hydrochlorid der 6,8-Dimethyl-1-phenyl-3,4-dihydro-pyrimido [1,6-a] indol-5-essigsäure vom Smp. 212-220° (Zers.). Das entsprechende Hemihydrat schmilzt bei über 210°.

Beispiel 14

In analoger Weise erhält man aus dem entsprechenden Ethylester das Hydrochlorid der 1-(3-Sulfamoyl-4-chlor-phenyl)-3,4-dihydro-pyrimido [1,6-a] indol-5-essigsäure vom Smp. 216-220° (Zers.).

Beispiel 15

In analoger Weise erhält man aus dem entsprechenden Ethylester das Hydrochlorid der 1-(2,6-Dichlorphenyl)-3,4-dihydro-pyrimido [1,6-a] indol-5-essigsäure vom Smp. 270-277° (Zers.).

Beispiel 16

In analoger Weise erhält man aus dem entsprechenden Ethylester das Hydrochlorid der 1-(2-Thienyl)-3,4-dihydro-pyrimido [1,6-a] indol-5-essigsäure vom Smp. 230-235° (Zers.). Aus der wässrigen Lösung des Hydrochlorids kristallisiert bei pH 6-7 die 1-(2-Thienyl)-3,4-dihydro-pyrimido [1,6-a] indol-5-essigsäure als inneres Salz vom Smp. 187-189°.

Beispiel 17

1-Phenyl-3,4-dihydro-pyrimido [1,6-a] indol-5-essigsäureamid

1 g (0,003 Mol) 1-Phenyl-3,4-dihydro-pyrimido [1,6-a] indol-5-acetonitril wird in 10 g Polyphosphorsäure 30 Minuten auf 100° erhitzt. Das Reaktionsgemisch wird in 100 ml Wasser gelöst, die Lösung unter Eiskühlung durch Zusatz von konz. Ammoniak alkalisch gestellt und das Reaktionsprodukt mit Methylenchlorid extrahiert. Die Methylenchloridphase wird mit Wasser neutral gewaschen, über $Na_2SO_4$ getrocknet und die Lösung zur Trockne eingeengt. Das als Rückstand verbleibende feste 1-Phenyl-3,4-dihydro-pyrimido [1,6-a] indol-5-essigsäureamid wird aus Methanol umkristallisiert : farblose Kristalle vom Smp. 239-240°. Das entsprechende Hydrochlorid schmilzt bei 253-260° (Zers.).

Das Ausgangsmaterial kann wie folgt hergestellt werden :

a) 38 g (0,1 Mol) 2-(Dibenzylamino-ethyl)-indol-3-acetonitril werden in 300 g Polyphosphorsäure eingetragen und das Gemisch unter Rühren ca. 60 Minuten auf 100° erwärmt. Man giesst auf 1 kg Eis aus, stellt durch Zugabe von konz. Ammoniak alkalisch und extrahiert mit Ether. Nach Waschen, Trocknen und Eindampfen der Etherphase erhält man 2-(Dibenzylamino-ethyl)-indol-3-acetamid als Sirup, der aus wenig Ether kristallisiert werden kann. Smp. 135-136°.

b) 120 g (0,3 Mol) 2-(Dibenzylamino-ethyl)-indol-3-acetamid werden unter Zusatz von 12 g 5 % Pd/C in 1,2 l Methanol l bei Normaldruck und 30-35° hydriert. Nach Aufnahme von 14,4 Wasserstoff wird die Hydrierung abgebrochen, vom Katalysator abfiltriert und die Lösung im Vacuum zur Trockne eingeengt. Der Rückstand wird aus 100 ml Ethanol unter Zusatz von 250 ml Ether umkristallisiert. Das so erhaltene 2-Aminoethyl-indol-3-acetamid schmilzt bei 156-157°.

c) 21,7 g (0,1 Mol) 2-Aminoethyl-indol-3-acetamid werden in einem Gemisch von 200 ml Ether und 100 ml Wasser unter gutem Rühren und Kühlung auf etwa 5° mit 17,5 ml (0,15 Mol) Benzoylchlorid versetzt und nach und nach 100 ml 2N-NaOH zugetropft. Die Reaktion ist nach ca. 1 Stunde beendet. Man rührt noch 1 Stunde bei 0-5° aus und saugt vom abgeschiedenen 2-(Benzoylamino-ethyl)-indol-3-acetamid vom Smp. 223-225° ab.

d) 1,6 g (0,005 Mol) 2-(Benzoylamino-ethyl)-indol-3-acetamid werden in 16 ml Phosphoroxichlorid 2 Stunden unter Rückfluss erhitzt. Man destilliert den Ueberschuss an Phosphoroxichlorid ab, nimmt den öligen Rückstand in Wasser auf und entfernt Nebenprodukte der Reaktion durch Extraktion mit Ether. Die wässrige Phase wird unter Kühlung mit konz. Ammoniak versetzt und mit Ether extrahiert. Nach Waschen, Trocknen und Eindampfen der Etherphase erhält man 1-Phenyl-3,4-dihydro-pyrimido [1,6-a] indol-5-acetonitril als gelbliches Oel, das durch Lösen in Methanol und Versetzen mit etherischer Salzsäure in ein kristallines Hydrochlorid vom Smp. 195-205° übergeführt werden kann.

Beispiel 18

In analoger Weise, wie in Beispiel 17 beschrieben, erhält man aus 1-(3-Sulfamoyl-4-chlor-phenyl)-3,4-dihydro-pyrimido [1,6-a] indol-5-acetonitril vom Smp. 280-285° das Hydrochlorid des 1-(3-Sulfamoyl-4-chlor-phenyl)-3,4-dihydro-pyrimido [1,6-a] indol-5-acetamids vom Smp. 249-257° (Zers.).

Beispiel 19

12 g (0,03 Mol) 1-(p-Methylthio-phenyl)-3,4-dihydro-pyrimido [1,6-a] indol-5-essigsäureethylester werden in 120 ml Eisessig gelöst und mit 4 ml 30 % Wasserstoffperoxid versetzt. Man lässt ca. 20 Stunden bei Raumtemperatur stehen, giesst auf 1 Liter Eiswasser, stellt mit konz. Ammoniak alkalisch und extrahiert mit Essigester. Die Essigesterphase wird gewaschen, getrocknet und eingedampft. Der erhaltene 1-(p-Methylsulfoxy-phenyl)-3,4-dihydro-pyrimido [1,6-a] indol-5-essigsäureethylester kristallisiert aus Ether in gelblichen Kristallen vom Smp. 150-151°.

Beispiel 20

In analoger Weise wie in Beispiel 1 beschrieben erhält man ausgehend von 2-[(p-Methylthio-phenylamino)-ethyl]-5-fluorindol-5-essigsäureethylester das Hydrochlorid von 7-Fluor-1-(p-methylthio-phenyl)-3,4-dihydro-pyrimido [1,6-a] indol-3-essigsäureethylester vom Smp. 210° (Zers.) und ausgehend von 2-[(p-Chlormethylthio-phenyl-amino)-ethyl]-5-fluor-indol-3-essigsäureethylester das Hydrochlorid von 7-Fluor-1-(p-chlormethylthio-phenyl)-3,4-dihydro-pyrimido [1,6-a] indol-5-essigsäureethylester vom Fp. 198-202°.

Beispiel 21

In analoger Weise wie in Beispiel 19 beschrieben erhält man ausgehend von 7-Fluor-1-(p-methylthio-phenyl)-3,4-dihydropyrimido [1,6-a] indol-5-essigsäureethylester-hydrochlorid des Hydrochlorid von 7-Fluor-1-(p-methylsulfoxypheny)-3,4-dihydro-pyrimido [1,6-a] indol-5-essigsäureethylester vom Smp. 261-264°.

Beispiel 22

In analoger Weise wie im Beispiel 9 erhält man durch Verseifung von 7-Fluor-(p-methylthio-phenyl)-pyrimido [1,6-a] indol-5-essigsäureethylester die 7-Fluor-1-(p-methylthio-phenyl)-pyrimido [1,6-a] indol-5-essigsäure vom Smp. 213-220° (Zers.).

Beispiel 23

7-Fluor-1-(p-methylthio-phenyl)-pyrimido [1,6-a] indol-5-essigsäureethylester

2 g 7-Fluor-1-(p-methylthio-phenyl)-3,4-dihydro-pyrimido [1,6-a] indol-5-essigsäureethylester werden in 20 ml Diphenylether mit 0,5 g Palladium/Kohle (10 %) unter Rühren zum Rückfluss erhitzt. Nach 2 Stunden werden weitere 0,5 g Palladium/Kohle zugesetzt und nochmals 3 Stunden erhitzt. Nach Abfiltrieren vom Katalysator wird das Reaktionsgemisch im Vakuum eingeengt, der Rückstand in wenig Essigester aufgenommen und über Kieselgel chromatographiert. Mit Hexan Essigester (9 : 1) werden Fraktionen erhalten, die nach Eindampfen und Umkristallisieren aus Ether 7-Fluor-1-(p-methylthio-phenyl)-pyrimido [1,6-a] indol-5-essigsäureethylester vom Smp. 120-122° liefern.

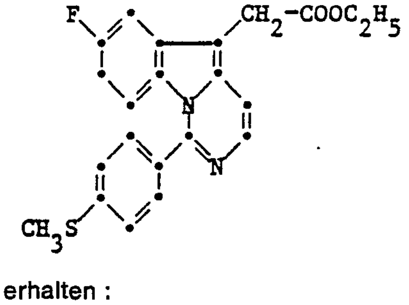

In analoger Weise werden erhalten :
1-Phenyl-pyrimido [1,6-a] indol-5-essigsäureethylester vom Smp. 59°-62°, 7-Methoxy-1-(p-Chlorphenyl)-pyrimido [1,6-a] indol-5-essigsäureethyl-ester, Smp. 130-131°.

Beispiel 24

1,0 g (0,003 Mol) 1-Phenyl-3,4-dihydro-pyrimido [1,6-a] indol-5-acetonitril wird in 50 ml abs. Ethanol und 50 ml mit Chlorwasserstoff gesättigtem Ethanol 48 Stunden bei 0° gerührt. Dann wird das Lösungsmittel unter vermindertem Druck abdestilliert. Das zurückbleibende, rohe Hydrochlorid des 1-Phenyl-3,4-dihydro-pyrimido [1,6-a] indol-5-acetiminoethylethers wird in 10 ml Wasser suspendiert und unter Rühren 15 Minuten auf 40° erhitzt. Nach dem Erkalten kristallisiert das 1-Phenyl-3,4-dihydro-pyrimido [1,6-a] indol-5-essigsäureethylester-hydrochlorid, welches nach Umkristallisieren aus 1N Salzsäure Kristalle vom Smp. 187-189° liefert.

Beispiel 25

3,84 g (0,01 Mol) 7-Fluor-1-(p-methylsulfoxy-phenyl)-3,4-dihydro-pyrimido [1,6-a] indol-5-essigsäureethylester werden in 500 ml Ethylacetat mit 40 g desaktiviertem Raney-Nickel unter Rühren 2 Stunden unter Rückfluss erhitzt. Dann wird vom Katalysator abfiltriert und das Filtrat unter vermindertem Druck eingedampft. Der Rückstand liefert nach Kristallisation aus Ether den 7-Fluor-1-(p-methylthio-phenyl)-3,4-dihydro-pyrimido [1,6-a] indol-5-essigsäureethylester vom Smp. 119-120°.

Beispiel 26

Tabletten, enthaltend 25 mg Wirkstoff, z. B. 7-Fluor-(1-(p-methylthio-phenyl)-3,4-dihydro-pyrimido [1,6-a] indol-5-essigsäure oder ein Salz, z. B. das Hydrochlorid, davon, können folgendermassen hergestellt werden :

Bestandteile (für 1 000 Tabletten)

| | |
|---|---|
| Wirkstoff | 25,0 g |
| Lactose | 100,7 g |
| Weizstärke | 7,5 g |
| Polyethylenglykol 6 000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer der in den Beispielen 1 bis 19 genannten Verbindungen der Formel I hergestellt werden, wobei die Verbindungen auch in Form von Saüreadditionssalzen, wie Hydrochloriden, und Verbindungen, worin $R_2$ 1-Carboxymethyl ist, auch in Form von Salzen und Basen, wie Natrium-, Kalium- oder Zinksalzen vorliegen können.

Beispiel 27

Kautabletten, enthaltend 30 mg Wirkstoff, z. B. 7-Fluor-1-(p-methylthio-phenyl)-3,4-dihydro-pyrimido [1,6-a] indol-5-essigsäure oder ein Salz, z. B. das Hydrochlorid davon, können z. B. folgendermassen hergestellt werden :

Zusammensetzung (für 1 000 Tabletten)

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5 %-ige Gelatinelösung | q.s. |

Herstellung

Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der

Mannit, das Lactosegranulat, die Stearinsäure und das Talkum hinzugegeben, das Ganze gründlich vermischt und zu beidseitig konkaven Tabletten von etwa 100 mm Durchmesser mit Bruchrille auf der Oberseite verpresst.

In analoger Weise können auch Kautabletten enthaltend jeweils 30 mg einer der in den Beispielen 1 bis 19 genannten Verbindungen der Formel I hergestellt werden, wobei die Verbindungen auch in Form von Säureadditionssalzen, wie Hydrochloriden, und Verbindungen, worin $R_2$ 1-Carboxymethyl ist, auch in Form von Salzen mit Basen, wie Natrium-, Kalium- oder Zinksalzen vorliegen können.

Beispiel 28

Tabletten enthaltend 100 mg Wirkstoff, z. B. 7-Fluor-1-(p-methylthio-phenyl)-3,4-dihydro-pyrimido [1,6-a] indol-5-essigsäure oder ein Salz, z. B. das Hydrochlorid, davon, können folgendermassen hergestellt werden :

Zusammensetzung (für 1 000 Tabletten)

| | |
|---|---|
| Wirkstoff | 100,0 g |
| Lactose | 248,5 g |
| Maisstärke | 17,5 g |
| Polethylenglykol 6 000 | 5,0 g |
| Talkum | 15,0 g |
| Magnesiumstearat | 4,0 g |
| entmineralisiertes Wasser | q.s. |

Herstellung

Die festen Ingredienzen werden zunächst durch ein Sieb mit 0,6 mm Maschenweite getrieben. Dann werden Wirkstoff, Lactose, Talkum, Magnesiumstearat und die Hälfte der Stärke innig vermischt.

Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von 10 mm Durchmesser mit Bruchkerbe auf der Oberseite verpresst.

In analoger Weise können auch Tabletten, enthaltend 100 mg einer Verbindung der Formel I gemäss den Beispielen 1 bis 25 hergestellt werden, wobei Verbindungen auch in Form von Säureadditionssalzen, wie Hydrochloriden, und Verbindungen, worin $R_2$ 1-Carboxymethyl ist, auch in Form von Salzen mit Basen, wie Natrium-, Kalium- oder Zinksalzen vorliegen können.

**Patentansprüche** (für die Vertragsstaaten : BE, CH, DE, FR, IT, LI, LU, NL, SE)

1. N,N'-überbrückte Carbonsäureamidine der allgemeinen Formel

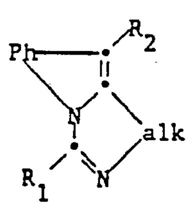

(I)

worin $R_1$ gegebenenfalls durch Niederalkyl, Niederalkoxy, gegebenenfalls Halogen enthaltendes Niederalkylthio, Niederalkansulfinyl bzw. Niederalkansulfonyl, gegebenenfalls durch Niederalkyl mono- oder disubstituiertes Sulfamoyl und/oder Halogen substituiertes Phenyl oder gegebenenfalls Niederalkyl, Niederalkoxy und/oder Halogen enthaltendes 5- oder 6-gliedriges und als Heteroatom Stickstoff, Sauerstoff oder Schwefel bzw. Stickstoff und zusätzlich Schwefel oder Sauerstoff aufweisendes, monocyclisches Heteroaryl darstellt, $R_2$ 1-Carboxyniederalkyl der Formel $—CH(R_3)—R_2'$ ist, in der $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia oder 3-Azaalkylen zweifach substituiertes Carbamoyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1-3 C-Atome trennendes Niederalkylen bzw. durch 2 C-Atome trennendes Niederalkenylen ist, und

ihre Salze, wobei unter mit « nieder » bezeichneten Resten solche zu verstehen sind, die bis und mit 7 C-Atome enthalten.

2. Verbindungen der Formel I nach Anspruch 1, worin $R_1$ gegebenenfalls durch Niederalkyl, Niederalkoxy, Niederalkylthio, Halogenniederalkylthio, Niederalkansulfinyl, Halogenniederalkansulfinyl, Niederalkansulfonyl, Halogenniederalkansulfonyl, Sulfamoyl, N-Mono- bzw. N,N-Diniederalkylsulfamoyl und/oder Halogen substituiertes Phenyl oder gegebenenfalls durch Niederalkyl, Niederalkoxy und/oder Halogen substituiertes Pyrrolyl, Furyl, Thienyl, Thiazolyl, Pyridyl bzw. Pyrimidyl bedeutet, $R_2$ eine Gruppe der Formel $-CH(R_3)-R_2'$ ist, in der $R_2'$ Carboxy, gegebenenfalls durch Niederalkyl, Niederalkoxy oder Halogen substituiertes Phenyl- bzw. Pyridylniederalkoxycarbonyl, Hydroxy- bzw. Niederalkoxyniederalkoxycarbonyl, Niederalkoxycarbonyl, Carbamoyl, N-Hydroxy- bzw. N-Aminocarbamoyl, N-Mono- bzw. N,N-Diniederalkylcarbamoyl bzw. -hydroxyniederalkylcarbamoyl oder durch 4- bis 7-gliedriges Niederalkylen substituiertes Carbamoyl bzw. 3-Oxa-, 3-Thia- oder 3-Azaalkylenaminocarbonyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1 bis 3 C-Atome trennendes Niederalkylen bzw. durch 2 C-Atome trennendes Niederalkenylen ist, sowie ihre Salze.

3. Verbindungen der Formel I nach Anspruch 1, worin $R_1$ gegebenenfalls durch Niederalkyl mit bis und mit 4 C-Atomen, Niederalkoxy mit bis und mit 4 C-Atomen, Halogenniederalkylthio mit bis und mit 4 C-Atomen, Niederalkylthio mit bis und mit 4 C-Atomen, Halogenniederalkansulfinyl mit bis und 4 C-Atomen, Niederalkansulfinyl mit bis und mit 4 C-Atomen, Halogenniederalkansulfonyl, Niederalkansulfonyl mit bis und mit 4 C-Atomen, Sulfamoyl, N-Mono- bzw. N,N-Diniederalkansulfamoyl mit jeweils bis und mit 4 C-Atomen im Alkyl und/oder durch Halogen mit Atomnummer bis und mit 35 substituiertes Phenyl oder gegebenenfalls durch Niederalkyl mit bis und mit 4 C-Atomen, durch Niederalkoxy mit bis und 4 C-Atomen, und/oder Halogen mit Atomnummer bis und mit 35 substituiertes Pyridyl bzw. Thienyl bedeutet, $R_2$ eine Gruppe der Formel $-CH(R_3)-R_2'$, in der $R_2'$ Carboxy, gegebenenfalls durch Niederalkyl mit bis und mit 4 C-Atomen, Niederalkoxy mit bis und mit 4 C-Atomen und/oder Halogen substituiertes Phenyl- bzw. Pyridylniederalkoxycarbonyl, Niederalkoxycarbonyl, Mono-bzw. Dihydroxy-niederalkoxycarbonyl, Niederalkoxyniederalkoxycarbonyl, N-Hydroxy- bzw. N-Aminocarbamoyl, N-Mono- bzw. N,N-Diniederalkoxycarbamoyl oder Carbamoyl ist, $R_3$ Wasserstoff oder Niederalkyl mit bis und mit 4 C-Atomen bedeutet, Ph gegebenenfalls durch Niederalkyl mit bis und mit 4 C-Atomen, Niederalkoxy mit bis und mit 4 C-Atomen und/oder Halogen bis und mit Atomnummer 35 substituiertes 1,2-Phenylen bedeutet und alk 1,2-Ethylen ist, sowie ihre Salze.

4. Verbindungen der Formel I nach Anspruch 1, worin $R_1$ gegebenenfalls durch Halogen, Niederalkylthio, Niederalkansulfinyl, Niederalkansulfonyl, Halogenniederalkylthio und/oder Sulfamoyl substituiertes Phenyl, Pyridyl oder Thienyl bedeutet, $R_2$ eine Gruppe der Formel $-CH_2-R_2'$ ist, in der $R_2'$ Carboxy, Niederalkoxycarbonyl oder Carbamoyl darstellt, Ph gegebenenfalls durch Niederalkoxy, Niederalkyl und/oder Halogen substituiertes 1,2-Phenylen bedeutet und alk 1,2-Ethylen oder Vinylen ist, sowie ihre Salze.

5. Verbindungen der Formel I nach Anspruch 1, worin $R_1$ gegebenenfalls durch Halogen mit Atomnummer bis und mit 35, Niederalkylthio mit bis und mit 4 C-Atomen, Niederalkansulfinyl mit bis und mit 4 C-Atomen, Halogenniederalkylthio mit bis und mit 4 C-Atomen und/oder Sulfamoyl substituiertes Phenyl, Pyridyl oder Thienyl bedeutet, $R_2$ eine Gruppe der Formel $-CH_2-R_2'$ ist, in der $R_2'$ Carboxy, Niederalkoxycarbonyl mit bis und mit 5 C-Atomen oder Carbamoyl darstellt, Ph gegebenenfalls durch Niederalkoxy mit bis und mit 4 C-Atomen, Niederalkyl mit bis und mit 4 C-Atomen und/oder Halogen mit Atomnummer bis und mit 35 substituiertes 1,2-Phenylen bedeutet und alk 1,2-Ethylen oder Vinylen ist, sowie ihre Salze.

6. Verbindungen der Formel I nach Anspruch 1, worin $R_1$ gegebenenfalls durch Halogenniederalkylthio mit bis und mit 4 C-Atomen, Niederalkylthio mit bis und mit 4 C-Atomen, Niederalkansulfinyl mit bis und mit 4 C-Atomen oder Halogen mit Atomnummer bis und mit 35 in p-Stellung oder durch Sulfamoyl in 3-Stellung und zusätzlich durch Halogen mit Atomnummer bis und mit 35 in 4-Stellung substituiertes Phenyl, Pyridyl oder Thienyl bedeutet, $R_2$ eine Gruppe der Formel $-CH_2-R_2'$, in der $R_2'$ Carboxy, Niederalkoxycarbonyl mit bis und mit 5 C-Atomen oder Carbamoyl darstellt, Ph gegebenenfalls in bezug auf das gebundene Stickstoffatom in 4-Stellung durch Niederalkoxy mit bis und mit 4 C-Atomen in 3- und 5-Stellung durch Niederalkyl mit bis und mit 4 C-Atomen oder in 4-Stellung durch Halogen mit Atomnummer bis und mit 35 substituiertes 1,2-Phenylen darstellt und alk 1,2-Ethylen oder Vinylen ist, sowie ihre Salze.

7. Verbindungen der Formel I nach Anspruch 1, worin $R_1$ gegebenenfalls in p-Stellung durch Niederalkylthio mit bis und mit 4 C-Atomen, durch Niederalkansulfinyl mit bis und mit 4 C-Atomen oder Halogen bis und mit Atomnummer 35 substituiertes Phenyl bedeutet, $R_2$ Niederalkoxycarbonylmethyl mit bis und mit 6 C-Atomen, wie Ethoxycarbonylmethyl ist, Ph gegebenenfalls in p-Stellung zum Stickstoffatom durch Niederalkoxy mit bis und mit 4 C-Atomen oder Halogen bis und mit Atomnummer 35 substituiertes 1,2-Phenylen darstellt und alk 1,2-Ethylen bedeutet, sowie ihre Salze.

8. Verbindungen der Formel I nach Anspruch 1, worin $R_1$ gegebenenfalls in p-Stellung durch Niederalkylthio mit bis und mit 4 C-Atomen, durch Niederalkansulfinyl mit bis und mit 4 C-Atomen oder Halogen bis und mit Atomnummer 35 substituiertes Phenyl bedeutet, $R_2$ Carboxymethyl ist, Ph gegebenenfalls in p-Stellung zum Stickstoffatom durch Niederalkoxy mit bis und mit 4 C-Atomen oder

Halogen bis und mit Atomnummer 35 substituiertes 1,2-Phenylen darstellt und alk 1,2-Ethylen bedeutet, sowie ihre Salze.

9. 7-Methoxy-1-(p-chlorphenyl)-3,4-dihydro-pyrimido [1,6-a] indol-essigsäureethylester oder ein Salz davon.

10. 1-Phenyl-3,4-dihydro-pyrimido [1,6-a] indol-5-essigsäureethylester oder ein Salz davon.

11. 7-Fluor-1-(p-methylthio-phenyl)-3,4-dihydro-pyrimido [1,6-a] indol-5-essigsäureethylester oder ein Salz davon.

12. 6,8-Dimethyl-1-phenyl-3,4-dihydro-pyrimido [1,6-a] indol-5-essigsäureethylester oder ein Salz davon.

13. 1-(3-Sulfamoyl-4-chlor-phenyl)-3,4-dihydro-pyrimido [1,6-a] indol-5-essigsäureethylester oder ein Salz davon.

14. 1-(2,6-Dichlorphenyl)-3,4-dihydro-pyrimido [1,6-a] indol-5-essigsäureethylester oder ein Salz davon.

15. 1-(2-Picolinyl)-3,4-dihydro-pyrimido [1,6-a] indol-5-essigsäure-ethylester oder ein Salz davon.

16. 1-(2-Thienyl)-3,4-dihydro-pyrimido [1,6-a] indol-5-essigsäureethylester oder ein Salz davon.

17. 7-Methoxy-1-(p-chlorphenyl)-3,4-dihydro-pyrimido [1,6-a] indol-5-essigsäure oder ein Salz davon.

18. 1-Phenyl-3,4-dihydro-pyrimido [1,6-a] indol-5-essigsäure oder ein Salz davon.

19. 7-Fluor-1-(p-methylthio-phenyl)-3,4-dihydro-pyrimido [1,6-a] indol-5-essigsäure oder ein Salz davon.

20. 7-Fluor-1-(p-methylsulfinyl-phenyl)-3,4-dihydro-pyrimido [1,6-a]-indol-5-essigsäure oder ein Salz davon.

21. 6,8-Dimethyl-1-phenyl-3,4-dihydro-pyrimido [1,6-a] indol-5-essigsäure oder ein Salz davon.

22. 1-(3-Sulfamoyl-4-chlor-phenyl)-3,4-dihydro-pyrimido [1,6-a] indol-5-essigsäure oder ein Salz davon.

23. 1-(2,6-Dichlorphenyl)-3,4-dihydro-pyrimido [1,6-a] indol-5-essigsaüre oder ein Salz davon.

24. 1-(2-Thienyl)-3,4-dihydro-pyrimido [1,6-a] indol-5-essigsäure oder ein Salz davon.

25. 1-Phenyl-3,4-dihydro-pyrimido [1,6-a] indol-5-essigsäureamid oder ein Salz davon.

26. 1-(3-Sulfamoyl-4-chlor-phenyl)-3,4-dihydro-pyrimido [1,6-a] indol-5-acetamid oder ein Salz davon.

27. 1-(p-Methylsulfoxy-phenyl)-3,4-dihydro-pyrimido [1,6-a] indol-5-essigsäureethylester oder ein Salz davon.

28. 7-Fluor-1-(p-chlormethylthio-phenyl)-3,4-dihydro-pyrimido [1,6-a]-indol-5-essigsäureethylester oder ein Salz davon.

29. 7-Fluor-1-(p-methylsulfoxyphenyl)-3,4-dihydro-pyrimido [1,6-a] indol-5-essigsäureethylester oder ein Salz davon.

30. 7-Fluor-1-(p-methylthio-phenyl)-pyrimido [1,6-a] indol-5-essigsäure-ethylester oder ein Salz davon.

31. 1-Phenyl-pyrimido [1,6-a] indol-5-essigsäureethylester oder ein Salz davon.

32. 7-Methoxy-1-(p-chlorphenyl)-pyrimido [1,6-a] indol-5-essigsäure-ethylester oder ein Salz davon.

33. 7-Fluor-1-(p-methylthiophenyl)-pyrimido [1,6-a] indol-5-essigsäure oder ein Salz davon.

34. Verbindungen der Formel I gemäss einem der Ansprüche 1 bis 33 als Antiinflammatorikum und/oder Analgetikum.

35. Pharmazeutische Präparate enthaltend eine Verbindung der Formel I gemäss einem der Ansprüche 1 bis 3 und 8 bis 27 in freier Form oder in Form eines pharmazeutisch verwendbaren Salzes zusammen mit den üblichen pharmazeutischen Hilfs- und Trägerstoffen.

36. Pharmazeutische Präparate enthaltend eine Verbindung der Formel I gemäss einem der Ansprüche 4 bis 7 und 28 bis 34 in freier Form oder in Form eines pharmazeutischen verwendbaren Salzes zusammen mit den üblichen pharmazeutischen Hilfs- und Trägerstoffen.

37. Verbindungen der Formel I gemäß einem der Ansprüche 1 bis 34 zur Anwendung in einem Verfahren zur therapeutischen Behandlung des menschlichen oder tierischen Körpers.

38. Verbindung der Formel I gemäss einem der Ansprüche 1 bis 34 zur Herstellung von pharmazeutischen Präparaten.

39. Verbindung der Formel I gemäss einem der Ansprüche 1 bis 34 zur Verwendung gemäss Anspruch 37 zur Behandlung entzündlicher Erkrankungen und/oder von Schmerzzuständen.

40. Verfahren zur Herstellung von Verbindungen der Formel

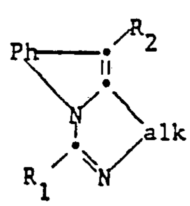

(I)

worin $R_1$ gegebenenfalls durch Niederalkyl, Niederalkoxy, gegebenenfalls Halogen enthaltendes Niederalkylthio, Niederalkansulfinyl bzw. Niederalkansulfonyl, gegebenenfalls durch Niederalkyl mono- oder

disubstituiertes Sulfamoyl und/oder Halogen substituiertes Phenyl oder gegebenenfalls Niederalkyl, Niederalkoxy und/oder Halogen enthaltendes 5- oder 6-gliedriges und als Heteroatom Stickstoff, Sauerstoff oder Schwefel bzw. Stickstoff und zusätzlich Schwefel oder Sauerstoff aufweisendes, monocyclisches Heteroaryl darstellt, $R_2$ 1-Carboxyniederalkyl der Formel —CH($R_3$)—$R_2'$ ist, in der $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1-3 C-Atome trennendes Niederalkylen bzw. durch 2 C-Atome trennendes Niederalkenylen ist, und ihre Salze, wobei unter mit « nieder » bezeichneten Resten solche zu verstehen sind, die bis und mit 7 C-Atome enthalten, dadurch gekennzeichnet, dass man aus Verbindungen der allgemeinen Formeln

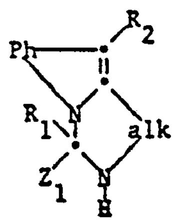 (II) oder 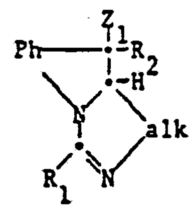 (III)

worin $Z_1$ gegebenenfalls abgewandeltes Hydroxy oder Mercapto bedeutet, oder Salzen davon unter Einführung einer zusätzlichen Bindung H—$Z_1$ abspaltet oder Verbindungen der Formel

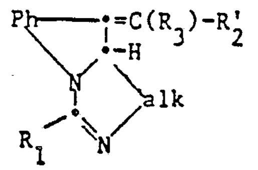 (V)

worin $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, oder Salze davon isomerisiert oder eine Verbindung der allgemeinen Formel

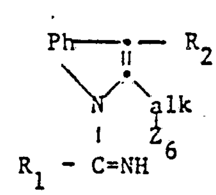 (VIII)

worin $Z_6$ gegebenenfalls funktionell abgewandeltes Hydroxy bzw. Mercapto oder Amino bedeutet, bzw. ein Salz davon cyclisiert oder in einer Verbindung der Formel

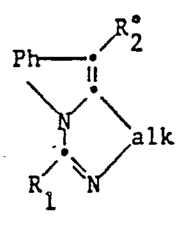 (X)

worin $R_2°$ eine Gruppe der Formel —CH($R_3$)—$R_2''$ bedeutet und wobei $R_3$ Wasserstoff oder Niederalkyl und $R_2''$ von $R_2'$ verschiedenes funktionell abgewandeltes Carboxy, eine Gruppe der Formel —C(=O)—$N_2^{\oplus}B^{\ominus}$, wobei $B^{\ominus}$ das Anion einer Mineralsäure ist, oder gegebenenfalls bis zur Stufe der Formyl oxidiertes Methyl bedeutet, oder Salzen davon $R_2°$ solvolytisch oder oxidativ in die Gruppe $R_2$ überführt, bzw. zur Herstellung von Verbindungen der Formel I, worin $R_2$ amidiertes 1-Carboxyniederalkyl der Formel —CH($R_3$)—$R_2'$ ist, in der $R_2'$ gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, und Ph, $R_1$ und alk obige Bedeutungen haben, gekennzeichnet durch Addition von Wasser und Abspaltung eines Protons an Verbindungen der Formel

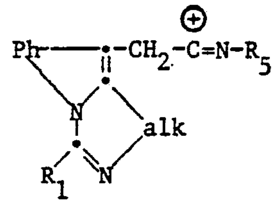

(XIa)

worin $R_5$ gegebenenfalls substituiertes Niederalkyl bzw. Phenyl bedeutet, bzw. zur Herstellung von Verbindungen der Formel I, worin $R_2$ eine Gruppe der Formel —CH($R_3$)—$R_2'$ ist, in der $R_2'$ gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, alk Vinylen bedeutet und $R_1$ und Ph obige Bedeutungen haben, dadurch gekennzeichnet, dass man Verbindungen der Formel

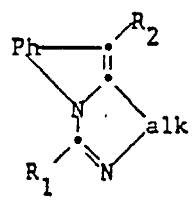

(I)

worin alk Ethylen bedeutet, unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

41. Verfahren nach Anspruch 40 zur Herstellung von Verbindungen de Formel I gemäss Anspruch 1, dadurch gekennzeichnet, dass man aus Verbindungen der allgemeinen Formel

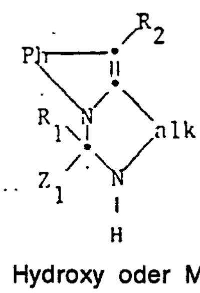

(II)

worin $Z_1$ gegebenenfalls abgewandeltes Hydroxy oder Mercapto bedeutet, oder Salzen davon unter Einführung einer zusätzlichen Bindung H—$Z_1$ abspaltet oder in einer Verbindung der Formel

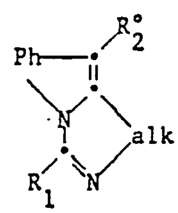

(X)

worin $R_2°$ eine Gruppe der Formel —CH($R_3$)—$R_2''$ bedeutet und wobei $R_3$ Wasserstoff oder Niederalkyl und $R_2''$ von $R_2'$ verschiedenes funktionell abgewandeltes Carboxy darstellen, oder Salzen davon, $R_2°$ solvolytisch in die Gruppe $R_2$ überführt, bzw. zur Herstellung von Verbindungen der Formel I, worin $R_2$ eine Gruppe der Formel —CH($R_3$)—$R_2'$ ist, in der $R_2'$ gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl darstellt, alk Vinylen bedeutet und $R_1$ und Ph obige Bedeutungen haben, dadurch gekennzeichnet, dass man Verbindungen der Formel

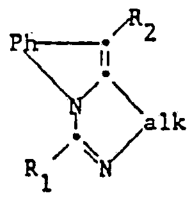

(I)

worin alk Ethylen bedeutet, unter Abspaltung von Wasserstoff bei gleichzeitiger Bildung einer zusätzlichen Bindung dehydriert und gewünschtenfalls eine erfindungsgemäß erhältliche Verbindung in eine andere Verbindung der Formel I bzw. eine erfindungsgemäß erhältliche freie Verbindung der Formel I in ein Salz oder ein verfahrensgemäss erhältliches Salz in die freie Verbindung der Formel I bzw. in ein anderes Salz überführt.

42. Die nach dem Verfahren gemäss einem der Ansprüche 40-41 erhältlichen Verbindungen.

**Patentansprüche** (für den Vertragsstaat AT)

1. Verfahren zur Herstellung N,N'-überbrückter Carbonsäureamidine der allgemeinen Formel

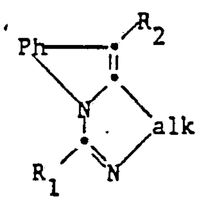

(I)

worin $R_1$ gegebenenfalls durch Niederalkyl, Niederalkoxy, gegebenenfalls Halogen enthaltendes Niederalkylthio, Niederalkansulfinyl bzw. Niederalkansulfonyl, gegebenenfalls durch Niederalkyl mono- oder disubstituiertes Sulfamoyl und/oder Halogen substituiertes Phenyl oder gegebenenfalls Niederalkyl, Niederalkoxy und/oder Halogen enthaltendes 5- oder 6-gliedriges und als Heteroatom Stickstoff, Sauerstoff oder Schwefel bzw. Stickstoff und zusätzlich Schwefel oder Sauerstoff aufweisendes, monocyclisches Heteroaryl darstellt, $R_2$ 1-Carboxyniederalkyl der Formel —$CH(R_3)$—$R_2'$ ist, in der $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, Ph gegebenenfalls durch Niederalkyl, Niederalkoxy, Halogen und/oder Trifluormethyl substituiertes 1,2-Phenylen bedeutet und alk die Methin- von der Iminogruppe durch 1-3 C-Atome trennendes Niederalkylen bzw. durch 2 C-Atome trennendes Niederalkenylen ist, und ihre Salze, wobei unter mit « nieder » bezeichneten Resten solche zu verstehen sind, die bis und mit 7 C-Atome enthalten, dadurch gekennzeichnet, dass man aus Verbindungen der allgemeinen Formel

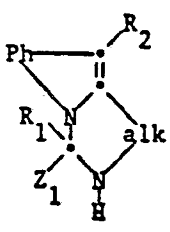

(II) oder

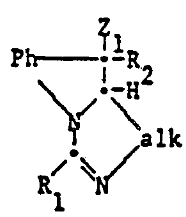

(III)

worin $Z_1$ gegebenenfalls abgewandeltes Hydroxy oder Mercapto bedeutet, oder Salzen davon unter Einführung einer zusätzlichen Bindung H—$Z_1$ abspaltet oder verbindungen der Formel

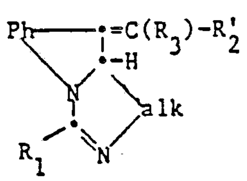

(V)

worin $R_2'$ Carboxy, gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch

Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, oder Salze davon isomerisiert oder eine Verbindung der allgemeinen Formel

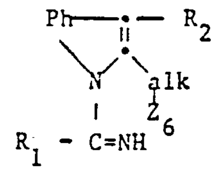

(VIII)

worin $Z_6$ gegebenenfalls funktionell abgewandeltes Hydroxy bzw. Mercapto oder Amino bedeutet, bzw. ein Salz davon cyclisiert oder in einer Verbindung der Formel

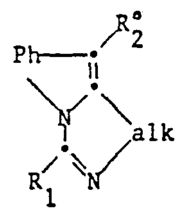

(X)

worin $R_2°$ eine Gruppe der Formel —$CH(R_3)$—$R_2''$ bedeutet und wobei $R_3$ Wasserstoff oder Niederalkyl und $R_2''$ von $R_2'$ verschiedenes funktionell abgewandeltes Carboxy, eine Gruppe der Formel —C(=O)—$N_2^{\oplus}B^{\ominus}$, wobei $B^{\ominus}$ das Anion einer Mineralsäure ist, oder gegebenenfalls bis zur Stufe des Formyl oxidiertes Methyl bedeutet, oder Salzen davon $R_2°$ solvolytisch oder oxidativ in die Gruppe $R_2$ überführt, bzw. zur Herstellung von Verbindungen der Formel I, worin $R_2$ amidiertes 1-Carboxyniederalkyl der Formel —$CH(R_3)$—$R_2'$ ist, in der $R_2'$ gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, und Ph, $R_1$ und alk obige Bedeutungen haben, gekennzeichnet durch Addition von Wasser und Abspaltung eines Protons an Verbindungen der Formel

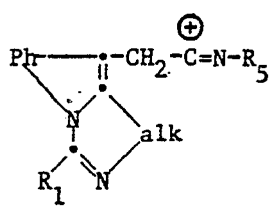

(XIa),

worin $R_5$ gegebenenfalls substituiertes Niederalkyl bzw. Phenyl bedeuten, bzw. zur Herstellung von Verbindungen der Formel I, worin $R_2$ eine Gruppe der Formel —$CH(R_3)$—$R_2'$ ist, in der $R_2'$ gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeutet, alk Vinylen bedeutet und $R_1$ und Ph obige Bedeutungen haben, dadurch gekennzeichnet, dass man Verbindungen der Formel

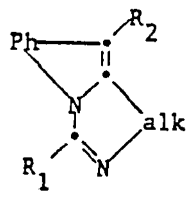

(I)

worin alk Ethylen bedeutet, unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

2. Verfahren nach Anspruch 1, dadurch gekennzeichnet, dass man aus Verbindungen der allgemeinen Formel

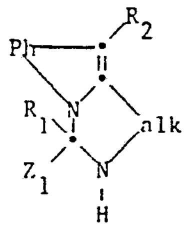

(II)

worin $Z_1$ gegebenenfalls abgewandeltes Hydroxy oder Mercapto bedeutet, oder Salzen davon unter Einführung einer zusätzlichen Bindung $H—Z_1$ abspaltet oder in einer Verbindung der Formel

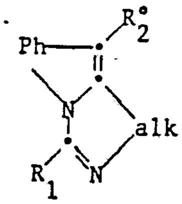

(X)

worin $R_2°$ eine Gruppe der Formel $—CH(R_3)—R_2''$ bedeutet und wobei $R_3$ Wasserstoff oder Niederalkyl und $R_2''$ von $R_2'$ verschiedenes funktionell abgewandeltes Carboxy darstellen, oder Salzen davon $R_2°$ solvolytisch in die Gruppe $R_2$ überführt, bzw. zur Herstellung von Verbindungen der Formel I, worin $R_2$ eine Gruppe der Formel $—CH(R_3)—R_2'$ ist, in der $R_2'$ gegebenenfalls durch Niederalkyl, Niederalkoxy oder Halogen enthaltendes Phenyl bzw. Pyridyl, Hydroxy oder Niederalkoxy substituiertes Niederalkoxycarbonyl oder gegebenenfalls durch Hydroxy bzw. Amino einfach, durch Niederalkyl bzw. Hydroxyniederalkyl ein- bzw. zweifach, durch 4- bis 7-gliedriges Niederalkylen bzw. 3-Oxa-, 3-Thia- oder 3-Azaalkylen zweifach substituiertes Carbamoyl ist, $R_3$ Wasserstoff oder Niederalkyl bedeuten darstellt, alk Vinylen bedeutet und $R_1$ und Ph obige Bedeutungen haben, dadurch gekennzeichnet, dass man Verbindungen der Formel

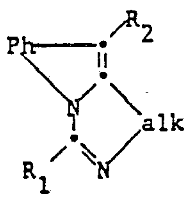

(I)

worin alk Ethylen bedeutet, unter Abspaltung von Wasserstoff bei gleichzeitiger Bildung einer zusätzlichen Bindung dehydriert und gewünschtenfalls eine erfindungsgemäss erhältliche Verbindung in eine andere Verbindung der Formel I bzw. eine erfindungsgemäss erhältliche freie Verbindung der Formel I in ein Salz oder ein verfahrensgemäss erhältliches Salz in die freie Verbindung der Formel I bzw. in ein anderes Salz überführt.

3. Verfahren nach Anspruch 1 oder 2, dadurch gekennzeichnet, dass man das Ausgangsmaterial der Formel (II) unter den Reaktionsbedingungen ohne Isolierung *in situ* durch Cyclisierung von Verbindungen der Formel

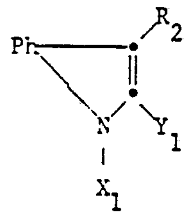

(IVa)

oder deren Salzen bildet, worin $X_1$ Wasserstoff ist und $Y_1$ eine Gruppe der Formel $—alk—NH—C(=Z_1)(R_1)$ und $Z_1$ Oxo bedeuten und Ph, $R_1$ und $R_2$ die in Anspruch 1 angegebenen Bedeutungen haben.

4. Verfahren nach einem der Ansprüche 1 bis 3 zur Herstellung von Verbindungen der Formel I, worin $R_1$ gegebenenfalls durch Niederalkyl, Niederalkoxy, Niederalkylthio, Halogenniederalkylthio, Niederalkansulfinyl, Halogenniederalkansulfinyl, Niederalkansulfonyl, Halogenniederalkansulfonyl, Sulfamoyl, N-Mono- bzw. N,N-Diniederalkylsulfamoyl und/oder Halogen substituiertes Phenyl oder gegebenenfalls durch Niederalkyl, Niederalkoxy und/oder Halogen substituiertes Pyrrolyl, Furyl, Thienyl, Thiazolyl, Pyridyl bzw. Pyrimidyl bedeutet, $R_2$ eine Gruppe der Formel $—CH(R_3)—R_2'$ ist, in der $R_2'$ Carboxy, gegebenenfalls durch Niederalkyl, Niederalkoxy oder Halogen substituiertes Phenyl- bzw. Pyridylniederalkoxycarbonyl, Hydroxy- bzw. Niederalkoxyniederalkoxycarbonyl, Niederalkoxycarbonyl, Carbamoyl, N-Hydroxy- bzw. N-Aminocarbamoyl, N-Mono- bzw. N,N-Diniederalkylcarbamoyl bzw. -hydroxyniederalkylcarbamoyl oder durch 4- bis 7-gliedriges Niederalkylen substituiertes Carbamoyl bzw. 3-Oxa-, 3-Thia- oder 3-Azaalkylenaminocarbonyl ist, $R_3$ Wasserstoff oder Niederalkyl bedeutet, Ph gegebenenfalls

durch Niederalkyl, Niederalkoxy, Halogen und/oder Trifluormethyl substituiertes 1,2-Phenylen bedeutet und alk die Methin- von der Iminogruppe durch 1 bis 3 C-Atome trennendes Niederalkylen bzw. durch 2 C-Atome trennendes Niederalkenylen ist, sowie ihrer Salze, dadurch gekennzeichnet, dass man von Verbindungen der Formeln II bzw. III, V, VIII, X, XIa oder I ausgeht, worin die Variablen die in Anspruch 1 angegebenen Bedeutungen haben.

5. Verfahren nach einem der Ansprüche 1 bis 3 zur Herstellung von Verbindungen der Formel I, worin $R_1$ gegebenenfalls durch Niederalkyl mit bis und mit 4 C-Atomen, Niederalkoxy mit bis und mit 4 C-Atomen, Halogenniederalkylthio mit bis und mit 4 C-Atomen, Niederalkylthio mit bis und mit 4 C-Atomen, Halogenniederalkansulfinyl mit bis und mit 4 C-Atomen, Niederalkansulfinyl mit bis und mit 4 C-Atomen, Halogenniederalkansulfonyl, Niederalkansulfonyl mit bis und mit 4 C-Atomen, Sulfamoyl, N-Mono bzw. N, N-Diniederalkansulfamoyl mit jeweils bis und mit 4 C-Atomen im Alkyl und/oder durch Halogen mit Atomnummer bis und mit 35 substituiertes Phenyl oder gegebenenfalls durch Niederalkyl mit bis und mit 4 C-Atomen, durch Niederalkoxy mit bis und mit 4 C-Atomen, und/oder Halogen mit Atomnummer bis und mit 35 substituiertes Pyridyl bzw. Thienyl bedeutet, $R_2$ eine Gruppe der Formel $—CH(R_3)—R_2'$, in der $R_2'$ Carboxy, gegebenenfalls durch Niederalkyl mit bis und mit 4 C-Atomen, Niederalkoxy mit bis und mit 4 C-Atomen, und/oder Halogen substituiertes Phenyl- bzw. Pyridylniederalkoxycarbonyl, Niederalkoxycarbonyl, Mono- bzw. Dihydroxy-niederalkoxycarbonyl, Niederalkoxyniederalkoxycarbonyl, N-Hydroxy- bzw. N-Aminocarbamoyl, N-Mono- bzw. N,N-Diniederalkoxycarbamoyl oder Carbamoyl ist, $R_3$ Wasserstoff oder Niederalkyl mit bis und mit 4 C-Atomen, bedeutet, Ph gegebenenfalls durch Niederalkyl mit bis und mit 4 C-Atomen, Niederalkoxy mit bis und mit 4 C-Atomen und/oder Halogen bis und mit Atomnummer 35 substituiertes 1,2-Phenylen bedeutet und alk 1,2-Ethylen ist, sowie ihrer Salze, dadurch gekennzeichnet, dass man von Verbindungen der Formeln II bzw. III, V, VIII, X oder XIa ausgeht, worin die Variablen die in Anspruch 1 angegebenen Bedeutungen haben.

6. Verfahren nach einem der Ansprüche 1 bis 3 zur Herstellung von Verbindungen der Formel I, worin $R_1$ gegebenenfalls durch Halogen, Niederalkylthio, Niederalkansulfinyl, Niederalkansulfonyl, Halogenniederalkylthio und/oder Sulfamoyl substituiertes Phenyl, Pyridyl oder Thienyl bedeutet, $R_2$ eine Gruppe der Formel $—CH_2—R_2'$ ist, in der $R_2'$ Carboxy, Niederalkoxycarbonyl oder Carbamoyl darstellt, Ph gegebenenfalls durch Niederalkoxy, Niederalkyl und/oder Halogen substituiertes 1,2-Phenylen bedeutet und alk 1,2-Ethylen oder Vinylen ist, sowie ihrer Salze, dadurch gekennzeichnet, dass man von Verbindungen der Formeln II bzw. III, V, VIII, X, XIa oder I ausgeht, worin die Variablen die in Anspruch 1 angegebenen Bedeutungen haben.

7. Verfahren nach einem der Ansprüche 1 bis 3 zur Herstellung von Verbindungen der Formel I, worin $R_1$ gegebenenfalls durch Halogen mit Atomnummer bis und mit 35, Niederalkylthio mit bis und mit 4 C-Atomen, Niederalkansulfinyl mit bis und mit 4 C-Atomen, Halogenniederalkylthio mit bis und mit 4 C-Atomen und/oder Sulfamoyl substituiertes Phenyl, Pyridyl oder Thienyl bedeutet, $R_2$ eine Gruppe der Formel $—CH_2—R_2'$ ist, in der $R_2'$ Carboxy, Niederalkoxycarbonyl mit bis und mit 5 C-Atomen oder Carbamoyl darstellt, Ph gegebenenfalls durch Niederalkoxy mit bis und mit 4 C-Atomen, Niederalkyl mit bis und mit 4 C-Atomen oder Halogen mit Atomnummer bis und mit 35 substituiertes 1,2-Phenylen bedeutet und alk 1,2-Ethylen oder Vinylen ist, sowie ihrer Salze, dadurch gekennzeichnet, dass man von Verbindungen der Formeln II bzw. III, V, VIII, X, XIa oder I ausgeht, worin die Variablen die in Anspruch 1 angegebenen Bedeutungen haben.

8. Verfahren nach einem der Ansprüche 1 bis 3 zur Herstellung von Verbindungen der Formel I, worin $R_1$ gegebenenfalls durch Halogenniederalkylthio mit bis und mit 4 C-Atomen, Niederalkylthio mit bis und mit 4 C-Atomen, Niederalkansulfinyl mit bis und mit 4 C-Atomen, oder Halogen mit Atomnummer bis und mit 35, in p-Stellung oder durch Sulfamoyl in 3-Stellung und zusätzlich durch Halogen mit Atomnummer bis und mit 35 in 4-Stellung substituiertes Phenyl, Pyridyl oder Thienyl bedeutet, $R_2$ eine Gruppe der Formel $—CH_2—R_2'$, in der $R_2'$ Carboxy, Niederalkoxycarbonyl mit bis und mit 5 C-Atomen oder Carbamoyl darstellt, Ph gegebenenfalls in bezug auf das gebundene Stickstoffatom in 4-Stellung durch Niederalkoxy mit bis und mit 4 C-Atomen in 3- und 5-Stellung durch Niederalkyl mit bis und mit 4 C-Atomen oder in 4-Stellung durch Halogen mit Atomnummer bis und mit 35 substituiertes 1,2-Phenylen darstellt und alk 1,2-Ethylen oder Vinylen ist, sowie ihrer Salze, dadurch gekennzeichnet, dass man von Verbindungen der Formeln II bzw. III, V, VIII, X, XIa oder I ausgeht, worin die Variablen die in Anspruch 1 angegebenen Bedeutungen haben.

9. Verfahren nach einem der Ansprüche 1 zur Herstellung von Verbindungen der Formel I, worin $R_1$ gegebenenfalls in p-Stellung durch Niederalkylthio mit bis und mit 4 C-Atomen durch Niederalkansulfinyl mit bis und mit 4 C-Atomen oder Halogen bis und mit Atomnummer 35 substituiertes Phenyl bedeutet, $R_2$ Niederalkoxycarbonylmethyl mit bis und mit 6 C-Atomen ist, Ph gegebenenfalls in p-Stellung zum Stickstoffatom durch Niederalkoxy mit bis und mit 4 C-Atomen oder Halogen bis und mit Atomnummer 35 substituiertes 1,2-Phenylen darstellt und alk 1,2-Ethylen bedeutet, sowie ihrer Salze, dadurch gekennzeichnet, dass man von Verbindungen der Formeln II bzw. III, V, VIII, X oder XIa ausgeht, worin die Variablen die in Anspruch 1 angegebenen Bedeutungen haben.

10. Verfahren nach einem der Ansprüche 1 bis 3 zur Herstellung von Verbindungen der Formel I, worin $R_1$ gegebenenfalls in p-Stellung durch Niederalkylthio mit bis und mit 4 C-Atomen durch Niederalkansulfinyl mit bis und mit 4 C-Atomen oder Halogen bis und mit Atomnummer 35 substituiertes

Phenyl bedeutet, $R_2$ Carboxymethyl ist, Ph gegebenenfalls in p-Stellung zum Stickstoffatom durch Niederalkoxy mit bis und mit 4 C-Atomen oder Halogen bis und mit Atomnummer 35 substituiertes 1,2-Phenylen darstellt und alk 1,2-Ethylen bedeutet, sowie ihrer Salze, dadurch gekennzeichnet, dass man von Verbindungen der Formeln II bzw. III, V, VIII, X oder XIa ausgeht, worin die Variablen die in Anspruch 1 angegebenen Bedeutungen haben.

11. Verfahren nach einem der Ansprüche 1 bis 3 zur Herstellung von 7-Methoxy-1-(p-chlorphenyl)-3,4-dihydro-pyrimido [1,6-a] indol-essigsäureethylester oder einem Salz davon, dadurch gekennzeichnet, dass man von Verbindungen der Formeln II bzw. III, V, VIII, X oder XIa ausgeht.

12. Verfahren nach einem der Ansprüche 1 bis 3 zur Herstellung von 1-Phenyl-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

13. Verfahren nach einem der Ansprüche 1 bis 3 zur Herstellung von 7-Fluor-1-(p-methylthio-phenyl-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

14. Verfahren nach einem der Ansprüche 1 bis 3 zur Herstellung von 6,8-Dimethyl-1-phenyl-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

15. Verfahren nach einem der Ansprüche 1 bis 3 zur Herstellung von 1-(3-Sulfamoyl-4-chlor-phenyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

16. Verfahren nach einem der Ansprüche 1 bis 3 zur Herstellung von 1-(2,6-Dichlorphenyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

17. Verfahren nach einem der Ansprüche 1 bis 3 zur Herstellung von 1-(2-Picolinyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

18. Verfahren nach einem der Ansprüche 1 bis 3 zur Herstellung von 1-(2-Thienyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

19. Verfahren nach einem der Ansprüche 1 bis 3 zur Herstellung von 7-Methoxy-1-(p-chlorphenyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

20. Verfahren nach einem der Ansprüche 1 bis 3 zur Herstellung von 1-Phenyl-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

21. Verfahren nach einem der Ansprüche 1 bis 3 zur Herstellung von 7-Fluor-1-(p-methylthio-phenyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

22. Verfahren nach einem der Ansprüche 1 bis 3 zur Herstellung von 7-Fluor-1-p-methylsulfinyl-phenyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

23. Verfahren nach einem der Ansprüche 1 bis 3 zur Herstellung von 6,8-Dimethyl-1-phenyl-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

24. Verfahren nach einem der Ansprüche 1 bis 3 zur Herstellung von 1-(3-Sulfamoyl-4-chlor-phenyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

25. Verfahren nach einem der Ansprüche 1 bis 3 zur Herstellung von 1-(2,6-Dichlorphenyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

26. Verfahren nach einem der Ansprüche 1 bis 3 zur Herstellung von 1-(2-Thienyl)-3,4-dihydro-pyrimido [1,6-a] indol-5-essigsäure oder einem Salz davon, dadurch gekennzeichnet, dass man von Verbindungen der Formeln II bzw. III, V, VIII, X oder XIa ausgeht.

27. Verfahren nach einem der Ansprüche 1 bis 3 zur Herstellung von 1-Phenyl-3,4-dihydro-pyrimido [1,6a] indol-5-essigsäureamid oder einem Salz davon, dadurch gekennzeichnet, dass man von Verbindungen der Formeln II bzw. III, V, VIII, X oder XIa ausgeht.

28. Verfahren nach einem der Ansprüche 1 bis 3 zur Herstellung von 1-(3-Sulfamoyl-4-chlor-phenyl)-3,4-dihydro-pyrimido [1,6-a] indol-5-acetamid oder einem Salz davon, dadurch gekennzeichnet, dass man von Verbindungen der Formeln II bzw. III, V, VIII, X oder XIa ausgeht.

29. Verfahren nach einem der Ansprüche 1 bis 3 zur Herstellung von 1-(p-Methyl-sulfoxy-phenyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

30. Verfahren nach einem der Ansprüche 1 bis 3 zur Herstellung von 7-Fluor-1-(p-chlormethylthio-phenyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

31. Verfahren nach einem der Ansprüche 1 bis 3 zur Herstellung von 7-Fluor-1-(p-methylsulfoxyphenyl)-3,4-dihydro-pyrimido [1,6-a] indol-5-essigsäureethylester oder einem Salz davon, dadurch gekennzeichnet, dass man von Verbindungen der Formeln II bzw. III, V, VIII, X oder XIa ausgeht.

32. Verfahren nach einem der Ansprüche 1 bis 3 zur Herstellung von 7-Fluor-1-(p-methylthio-phenyl)-pyrimido [1,6-a] indol-5-essigsäureethylester oder einem Salz davon, dadurch gekennzeichnet, dass man von der entsprechenden Verbindung der Formel I ausgeht.

33. Verfahren nach einem der Ansprüche 1 bis 3 zur Herstellung von 1-Phenyl-pyrimido [1,6-a] indol-5-essigsäureethylester oder einem Salz davon, dadurch gekennzeichnet, dass man von der entsprechenden Verbindung der Formel I ausgeht.

34. Verfahren nach einem der Ansprüche 1 bis 3 zur Herstellung von 7-Methoxy-1-(p-chlorphenyl)-pyrimido [1,6-a] indol-5-essigsäureethylester oder einem Salz davon, dadurch gekennzeichnet, dass man von der entsprechenden Verbindung der Formel I ausgeht.

35. Verfahren nach einem der Ansprüche 1 bis 3 zur Herstellung von 7-Fluor-1-(p-methylthiophenyl)-pyrimido [1,6-a] indol-5-essigsäure oder einem Salz davon, dadurch gekennzeichnet, dass man von der entsprechenden Verbindung der Formel I ausgeht.

36. Verfahren zur Herstellung pharmazeutischer Präparate, dadurch gekennzeichnet, dass man eine Verbindung gemäss einem der Ansprüche 1 bis 35 in freier Form oder in Form eines pharmazeutischen verwendbaren Salzes mit üblichen pharmazeutischen Hilfs- und/oder Trägerstoffen vermischt.

**Claims** (for the Contracting States : BE, CH, DE, FR, IT, LI, LU, NL, SE)

1. An N,N'-bridged carboxylic acid amidine of the general formula

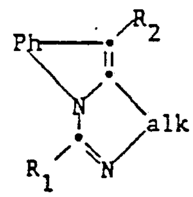

(I)

in which $R_1$ is phenyl which is unsubstituted or substituted by lower alkyl, by lower alkoxy, by unsubstituted or halogen-substituted lower alkylthio, lower alkanesulphinyl or lower alkane sulphonyl, by sulphamoyl which is unsubstituted or mono- or disubstituted by lower alkyl, and/or said phenyl is substituted by halogen, or $R_1$ is 5- to 6-membered monocyclic heteroaryl which is unsubstituted or substituted by lower alkyl, lower alkoxy and/or halogen and which contains as hetero atom nitrogen, oxygen or sulphur, or nitrogen and, in additon, sulphur or oxygen, $R_2$ is 1-carboxy-lower alkyl of the formula $—CH(R_3)—R_2'$, in which $R_2'$ is carboxy, or is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower lakoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, Ph is 1,2-phenylene which is unsubstituted or substituted by lower alkyl, lower alkoxy, halogen and/or trifluoromethyl, and alk is lower alkylene separating the methine group from the imino group by 1 to 3 carbon atoms or lower alkylene separating the methine group from the imino group by 2 carbon atoms ; or a salt thereof, with the radicals qualified by the term « lower » containing up to and including 7 carbon atoms.

2. A compound of the formula I according to claim 1, in which $R_1$ is phenyl which is unsubstituted or substituted by lower alkyl, lower alkoxy, lower alkylthio, halo-lower alkylthio, lower alkanesulphinyl, halo-lower alkanesulphinyl, lower alkanesulphonyl, halo-lower alkanesulphonyl, sulphamoyl, N-mono- or N,N-di-lower alkylsulphamoyl and/or halogen, or is pyrrolyl, furyl, thienyl, thiazolyl, pyridyl or pyrimidyl, each unsubstituted or substituted by lower alkyl, lower alkoxy and/or halogen, $R_2$ is a group of the formula $—CH(R_3)—R_2'$, in which $R_2'$ is carboxy, or is phenyl-lower or pyridyl-lower alkoxycarbonyl, each unsubstituted or substituted by lower alkyl, lower alkoxy or halogen, or is hydroxy-lower or lower alkoxy-lower alkoxycarbonyl, lower alkoxycarbonyl, carbamoyl, N-hydroxycarbamoyl, N-aminocarbamoyl, N-mono- or N,N-di-lower alkylcarbamoyl or N-monohydroxy- or N,N-dihydroxy-lower alkylcarbamoyl, or is carbamoyl substituted by 4- to 7-membered lower alkylene, or is 3-oxa, 3-thia- or 3-aza-alkyleneaminocarbonyl, $R_3$ is hydrogen or lower alkyl, Ph is 1,2-phenylene which is unsubstituted or substituted by lower alkyl, lower alkoxy, halogen and/or trifluoromethyl, and alk is lower alkylene separating the methine group from the imino group by 1 to 3 carbon atoms or is lower alkenylene separating the methine group from the imino group by 2 carbon atoms ; or a salt thereof.

3. A compound of the formula I according to claim 1, in which $R_1$ is phenyl which is unsubstituted or substituted by lower alkyl having up to and including 4 carbon atoms, by lower alkoxy having up to and including 4 carbon atoms, by halo-lower alkylthio having up to and including 4 carbon atoms, by lower

alkylthio having up to and including 4 carbon atoms, by halo-lower alkanesulphinyl having up to and including 4 carbon atoms, by lower alkanesulphinyl having up to and including 4 carbon atoms, by halo-lower alkanesulphonyl, by lower alkanesulphonyl having up to and including 4 carbon atoms, by sulphamoyl by N-mono- or N,N-di-lower alkanesulphamoyl each having up to and including 4 carbon atoms in the alkyl radical, and/or by halogen having an atomic number of up to and including 35, or $R_1$ is pyridyl or thienyl, each unsubstituted or substituted by lower alkyl having up to and including 4 carbon atoms, by lower alkoxy having up to and including 4 carbon atoms, and/or by halogen having an atomic number of up to and including 35, $R_2$ is a group of the formula $-CH(R_3)-R_2'$, in which $R_2'$ is carboxy, or is phenyl-lower or pyridyl-lower alkoxycarbonyl, each unsubstituted or substituted by lower alkyl having up to and including 4 carbon atoms, by lower alkoxy having up to and including 4 carbon atoms, and/or by halogen, or is lower alkoxycarbonyl, mono- or dihydroxy-lower alkoxycarbonyl, lower alkoxy-lower alkoxycarbonyl, N-hydroxy- or N-amino-carbamoyl, N-mono- or N,N-di-lower alkoxycarbamoyl, or carbamoyl, $R_3$ is hydrogen or lower alkyl having up to and including 4 carbon atoms, Ph is 1,2-phenylene which is unsubstituted or substituted by lower alkyl having up to and including 4 carbon atoms, by lower alkoxy having up to and including 4 carbon atoms, and/or by halogen having an atomic number of up to and including 35, and alk is 1,2-ethylene ; or a salt thereof.

4. A compound of the formula I according to claim 1, in which $R_1$ is phenyl which is unsubstituted or substituted by halogen, lower alkylthio, lower alkanesulphinyl, lower alkanesulphonyl, halo-lower alkylthio and/or sulphamoyl, or is pyridyl or thienyl, $R_2$ is a group of the formula $-CH_2-R_2'$, in which $R_2'$ is carboxy, lower alkoxycarbonyl or carbamoyl, Ph is 1,2-phenylene which is unsubstituted or substituted by lower alkoxy, lower alkyl and/or halogen, and alk is 1,2-ethylene or vinylene ; or a salt thereof.

5. A compound of the formula I according to claim 1, in which $R_1$ is phenyl which is unsubstituted or substituted by halogen having an atomic number of up to and including 35, by lower alkylthio having up to and including 4 carbon atoms, by lower alkanesulphinyl having up to and including 4 carbon atoms, by halo-lower alkylthio having up to and including 4 carbon atoms, and/or by sulphamoyl, or is pyridyl or thienyl, $R_2$ is a group of the formula $-CH_2-R_2'$, in which $R_2'$ is carboxy, or is lower alkoxycarbonyl having up to and including 5 carbon atoms, or carbamoyl, Ph is 1,2-phenylene which is unsubstituted or substituted by lower alkoxy having up to and including 4 carbon atoms, by lower alkyl having up to and including 4 carbon atoms, and/or by halogen having an atomic number of up to and including 35, and alk is 1,2-ethylene or vinylene ; or a salt thereof.

6. A compound of the formula I according to claim 1, in which $R_1$ is phenyl which is unsubstituted or substituted in the p-position by halo-lower alkylthio having up to and including 4 carbon atoms, by lower alkylthio having up to and including 4 carbon atoms, by lower alkanesulphinyl having up to and including 4 carbon atoms, or by halogen having an atomic number of up to and including 35, or in the 3-position by sulphamoyl and, in addition, in the 4-position by halogen having an atomic number of up to and including 35, or is pyridyl or thienyl, $R_2$ is a group of the formula $-CH_2-R_2'$, in which $R_2'$ is carboxy, or is lower alkoxycarbonyl having up to and including 5 carbon atoms, or carbamoyl, Ph is 1,2-phenylene which is unsubstituted or substituted in the 4-position relative to the bonded nitrogen atom by lower alkoxy having up to and including 4 carbon atoms, in the 3- and 5-positions by lower alkyl having up to and including 4 carbon atoms, or in the 4-position by halogen having an atomic number of up to and including 35, and alk is 1,2-ethylene or vinylene ; or a salt thereof.

7. A compound of the formula I according to claim 1, in which $R_1$ is phenyl which is unsubstituted or substituted in the p-position by lower alkylthio having up to and including 4 carbon atoms, by lower alkanesulphinyl having up to and including 4 carbon atoms, or by halogen having an atomic number of up to and including 35, $R_2$ is lower alkoxycarbonylmethyl having up to and including 6 carbon atoms, e. g. ethoxycarbonylmethyl, Ph is 1,2-phenylene which is unsubstituted or substituted in the p-position relative to the nitrogen atom by lower alkoxy having up to and including 4 carbon atoms, or by halogen having an atomic number of up to and including 35, and alk is 1,2-ethylene ; or a salt thereof.

8. A compound of the formula I according to claim 1, in which $R_1$ is phenyl which is unsubstituted or substituted in the p-position by lower alkylthio having up to and including 4 carbon atoms, by lower alkanesulphinyl having up to and including 4 carbon atoms, or by halogen having an atomic number of up to and including 35, $R_2$ is carboxymethyl, Ph is 1,2-phenylene which is unsubstituted or substituted in the p-position relative to the nitrogen atom by lower alkoxy having up to and including 4 carbon atoms or by halogen having an atomic number of up to and including 35, and alk is 1,2-ethylene ; or a salt thereof.

9. Ethyl 7-methoxy-1-(p-chlorophenyl)-3,4-dihydropyrimido [1,6-a] indoleacetate, or a salt thereof.

10. Ethyl 1-phenyl-3,4-dihydropyrimido [1,6-a] indole-5-acetate, or a salt thereof.

11. Ethyl 7-fluoro-1-(p-methylthiophenyl)-3,4-dihydropyrimido [1,6-a] indole-5-acetate, or a salt thereof.

12. Ethyl 6,8-dimethyl-1-phenyl-3,4-dihydropyrimido [1,6-a] indole-5-acetate, or a salt thereof.

13. Ethyl 1-(3-sulphamoyl-4-chlorophenyl)-3,4-dihydropyrimido [1,6-a] indole-5-acetate, or a salt thereof.

14. Ethyl 1-(2,6-dichlorophenyl)-3,4-dihydropyrimido [1,6-a] indole-5-acetate, or a salt thereof.

15. Ethyl 1-(2-picolinyl)-3,4-dihydropyrimido [1,6-a] indole-5-acetate, or a salt thereof.

16. Ethyl 1-(2-thienyl)-3,4-dihydropyrimido [1,6-a] indole-5-acetate, or a salt thereof.

17. 7-Methoxy-1-(p-chlorophenyl)-3,4-dihydropyrimido [1,6-a] indole-5-acetic acid, or a salt thereof.

18. 1-Phenyl-3,4-dihydropyrimido [1,6-a] indole-5-acetic acid, or a salt thereof.

19. 7-Fluoro-1-(p-methylthiophenyl)-3,4-dihydropyrimido [1,6-a] indole-5-acetic acid, or a salt thereof.

20. 7-Fluoro-1-(p-methylsulphinylphenyl)-3,4-dihydropyrimido [1,6-a] indole-5-acetic acid, or a salt thereof.

21. 6,8-Dimethyl-1-phenyl-3,4-dihydropyrimido [1,6-a] indole-5-acetic acid, or a salt thereof.

22. 1-(3-Sulphamoyl-4-chlorophenyl)-3,4-dihydropyrimido [1,6-a] indole-5-acetic acid, or a salt thereof.

23. 1-(2,6-Dichlorophenyl)-3,4-dihydropyrimido [1,6-a] indole-5-acetic acid, or a salt thereof.

24. 1-(2-Thienyl)-3,4-dihydropyrimido [1,6-a] indole-5-acetic acid, or a salt thereof.

25. 1-Phenyl-3,4-dihydropyrimido [1,6-a] indole-5-acetamide, or a salt thereof.

26. 1-(3-Sulphamoyl-4-chlorophenyl)-3,4-dihydropyrimido [1,6-a] indole-5-acetamide, or a salt thereof.

27. Ethyl 1-(p-methylsulphoxyphenyl)-3,4-dihydropyrimido [1,6-a] indole-5-acetate, or a salt thereof.

28. Ethyl 7-fluoro-1-(p-chloromethylthiophenyl)-3,4-dihydropyrimido [1,6-a] indole-5-acetate, or a salt thereof.

29. Ethyl 7-fluoro-1-(p-methylsulphoxyphenyl-3,4-dihydropyrimido [1,6-a] indole-5-acetate, or a salt thereof.

30. Ethyl 7-fluoro-1-(p-methylthiophenyl)pyrimido [1,6-a] indole-5-acetate, or a salt thereof.

31. Ethyl 1-phenylpyrimido [1,6-a] indole-5-acetate, or a salt thereof.

32. Ethyl 7-methoxy-1-(p-chlorophenyl)pyrimido [1,6-a] indole-5-acetate, or a salt thereof.

33. 7-Fluoro-1-(p-methylthiophenyl)pyrimido [1,6-a] .indole-5-acetic acid, or a salt thereof.

34. Use of a compound of the formula I according to any one of claims 1 to 33 as anti-inflammatory agent and/or analgesic.

35. A pharmaceutical composition containing a compound of the formula I according to any one of claims 1 to 3 and 8 to 27 in the free form or in the form of a pharmaceutically acceptable salt, together with the customary pharmaceutical auxiliaries and carriers.

36. A pharmaceutical composition containing a compound of the formula I according to any one of claims 4 to 7 and 28 to 34 in the free form or in the form of a pharmaceutically acceptable salt, together with the customary pharmaceutical auxiliaries and carriers.

37. Use of a compound of the formula I according to any one of claims 1 to 34 in a therapeutic method of treating the human or animal body.

38. A compound of the formula I according to any one of claims 1 to 34 for the preparation of a pharmaceutical composition.

39. Use according to claim 37 of a compound of the formula I according to any one of claims 1 to 34 in a method of treating inflammatory and/or painful conditions.

40. A process for the preparation of a compound of the formula

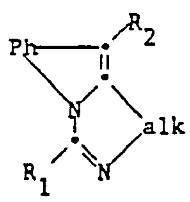

(I)

in which $R_1$ is phenyl which is unsubstituted or substituted by lower alkyl, lower alkoxy, by unsubstituted or halogen-substituted lower alkylthio, lower alkanesulphinyl or lower alkane sulphonyl, by sulphamoyl which is unsubstituted or mono- or disubstituted by lower alkyl, and/or said phenyl is substituted by halogen, or $R_1$ is 5- to 6-membered monocyclic heteroaryl which is unsubstituted or substituted by lower alkyl, lower alkoxy and/or halogen and which contains as hetero atom nitrogen, oxygen or sulphur, or nitrogen and, in addition, sulphur or oxygen, $R_2$ is 1-carboxy-lower alkyl of the formula $-CH(R_3)-R_2'$, in which $R_2'$ is carboxy, or is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or, disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, Ph is 1,2-phenylene which is unsubstituted or substituted by lower alkyl, lower alkoxy, halogen and/or trifluoromethyl, and alk is lower alkylene separating the methine group from the imino group by 1 to 3 carbon atoms or lower alkylene separating the methine group from the imino group by 2 carbon atoms ; or of a salt thereof, with the radicals qualified by the term « lower » containing up to and including 7 carbon atoms, which process comprises removing $H-Z_1$ from a compound of the general formula

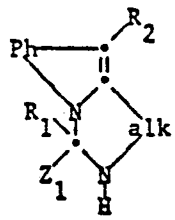 (II) or 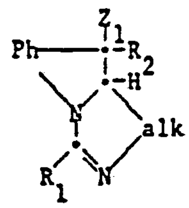 (III)

in which formulae $Z_1$ is free or modified hydroxy or mercapto, or from a salt thereof, to introduce an additional bond, or isomerising a compound of the formula

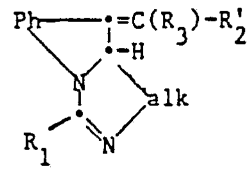

(V)

in which $R_2'$ is carboxy, or is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, or a salt thereof, or cyclising a compound of the general formula

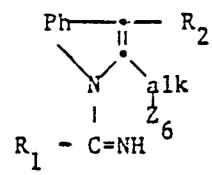

(VIII)

in which $Z_6$ is free or functionally modified hydroxy or mercapto, or amino, or a salt thereof, or in a compound of the formula

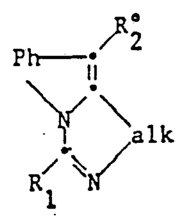

(X)

in which $R_2°$ is a group of the formula $—CH(R_3)—R_2''$, in which $R_3$ is hydrogen or lower alkyl, and $R_2''$ is functionally modified carboxy which differs from $R_2'$, or is a group of the formula $—C(=O)—N^{\oplus}B^{\ominus}$, in which $B^{\ominus}$ is the anion of a mineral acid, or is methyl or methyl oxidised to the formyl stage, or in a salt thereof, converting $R_2°$ into the group $R_2$ by solvolysis or oxidation, or

to prepare a compound of the formula I, in which $R_2$ is amidated 1-carboxy-lower alkyl of the formula $—CH(R_3)—R_2'$, in which $R_2'$ is carbamoyl which is unsubstituted or monosubstituted by hydroxy or amino, or mono- or disubstituted by lower alkyl or hydroxy-lower alkyl, or disubstituted by 4 to 7-membered lower alkylene or 3-oxa-, 3-thia- or 3-azaalkylene, $R_3$ is hydrogen or lower alkyl, and Ph, $R_1$ and alk are as defined above, adding water and removing a proton from a compound of the formula

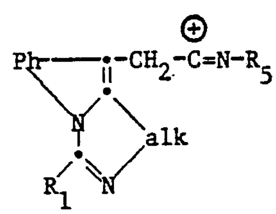

(XIa)

in which $R_5$ is lower alkyl or phenyl, each unsubstituted or substituted, or to prepare a compound of the formula I, in which $R_2$ is a group of the formula $—CH(R_3)—R_2'$, in which $R_2'$ is lower alkoxycarbonyl

which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa, 3-thia- or 3-azaalkylene, $R_3$ is hydrogen or lower alkyl, alk is vinylene, and $R_1$ and Ph are as defined above, dehydrogenating a compound of the formula

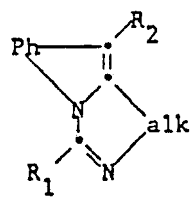

(I)

in which alk is ethylene, to remove hydrogen with simultaneous formation of an additional bond, and, if desired, converting a compound obtainable by a process of this invention into another compound of the formula I, or converting a free compound of the formula I obtainable by a process of this invention into a salt, or converting a salt obtainable by a process of this invention into the free compound of the formula I or into another salt.

41. A process according to claim 40 for the preparation of a compound of the formula I according to claim 1, which process comprises removing H—$Z_1$ from a compound of the general formula

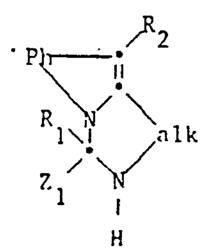

(II)

wherein $Z_1$ is free or modified hydroxy or mercapto, or from a salt thereof, to introduce an additional bond, or in a compound of the formula

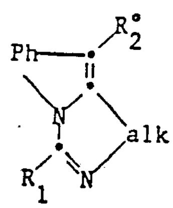

(X)

in which $R_2°$ is a group of the formula —CH($R_3$)—$R_2''$, in which $R_3$ is hydrogen or lower alkyl, and $R_2''$ is functionally modifed carboxy which differs from $R_2'$, or in a salt thereof, converting $R_2°$ into the group $R_2$ by solvolysis, or to prepare a compound of the formula I, in which $R_2$ is a group of the formula —CH($R_3$)—$R_2'$, in which $R_2'$ is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, alk is vinylene, and $R_1$ and Ph are as defined above, dehydrogenating a compound of the formula

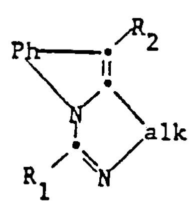

(I)

in which alk is ethylene, to remove hydrogen with simultaneous formation of an additional bond, and, if desired, converting a compound obtainable by a process of this invention into another compound of the

formula I, or converting a free compound of the formula I obtainable by a process of this invention into a salt, or converting a salt obtainable by a process of this invention into the free compound of the formula I or into another salt.

42. A compound obtainable by a process according to either claim 40 or claim 41.

**Claims** (for the Contracting State AT)

1. A process for the preparation of an N,N'-bridged carboxylic acid amidine of the general formula

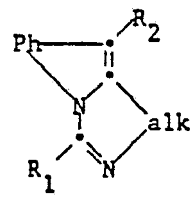

(I)

in which $R_1$ is phenyl which is unsubstituted or substituted by lower alkyl, lower alkoxy, by unsubstituted or halogen-substituted lower alkylthio, lower alkanesulphinyl or lower alkane sulphonyl, by sulphamoyl which is unsubstituted or mono- or disubstituted by lower alkyl, and/or said phenyl is substituted by halogen, or $R_1$ is 5- to 6-membered monocyclic heteroaryl which is unsubstituted or substituted by lower alkyl, lower alkoxy and/or halogen and which contains as hetero atom nitrogen, oxygen or sulphur, or nitrogen and, in addition, sulphur or oxygen, $R_2$ is 1-carboxy-lower alkyl of the formula $—CH(R_3)—R_2'$ in which $R_2'$ is carboxy, or is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, Ph is 1,2-phenylene which is unsubstituted or substituted by lower alkyl, lower alkoxy, halogen and/or trifluoromethyl, and alk is lower alkylene separating the methine group from the imino group by 1 to 3 carbon atoms or lower alkylene separating the methine group from the imino group by 2 carbon atoms ; or of a salt thereof, with the radicals qualified by the term « lower » containing up to and including 7 carbon atoms, which process comprises removing $H—Z_1$ from a compound of the general formula

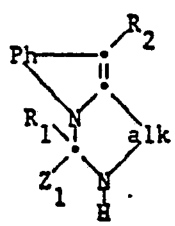

(II) or

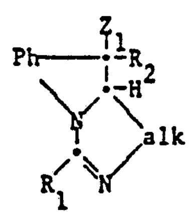

(III)

in which formulae $Z_1$ is free or modified hydroxy or mercapto, or from a salt thereof, to introduce an additional bond, or isomerising a compound of the formula

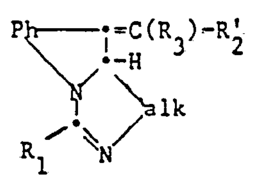

(V)

in which $R_2'$ is carboxy, or is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, or a salt thereof, or cyclising a compound of the general formula

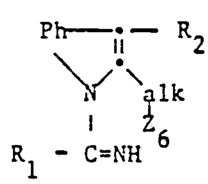

(VIII)

in which $Z_6$ is free or functionally modified hydroxy or mercapto, or amino, or a salt thereof, or in a compound of the formula

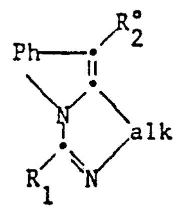

(X)

in which $R_2°$ is a group of the formula $-CH(R_3)-R_2''$, in which $R_3$ is hydrogen or lower alkyl, and $R_2''$ is functionally modified carboxy which differs from $R_2'$, or is a group of the formula $-C(=O)-N^{\oplus}B^{\ominus}$, in which $B^{\ominus}$ is the anion of a mineral acid, or is methyl or methyl oxidised to the formyl stage, or in a salt thereof, converting $R_2°$ into the group $R_2$ by solvolysis or oxidation, or to prepare a compound of the formula I, in which $R_2$ is amidated 1-carboxy-lower alkyl of the formula $-CH(R_3)-R_2'$ in which $R_2'$ is carbamoyl which is unsubstituted or monosubstituted by hydroxy or amino, or mono- or disubstituted by lower alkyl or hydroxy-lower alkyl, or disubstituted by 4 to 7-membered lower alkylene or 3-oxa-, 3-thia- or 3-azaalkylene, $R_3$ is hydrogen or lower alkyl, and Ph, $R_1$ and alk are as defined above, adding water and removing a proton from a compound of the formula

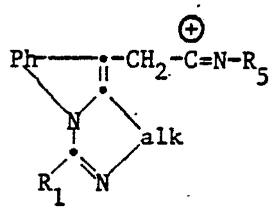

(XIa)

in which $R_5$ is lower alkyl or phenyl, each unsubstituted or substituted, or to prepare a compound of the formula I, in which $R_2$ is a group of the formula $-CH(R_3)-R_2'$, in which $R_2'$ is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, alk is vinylene, and $R_1$ and Ph are as defined above, dehydrogenating a compound of the formula

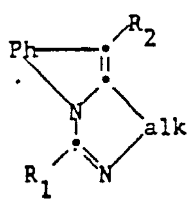

(I)

in which alk is ethylene, to remove hydrogen with simultaneous formation of an additional bond, and, if desired, converting a compound obtainable by a process of this invention into another compound of the formula I, or converting a free compound of the formula I obtainable by a process of this invention into a salt, or converting a salt obtainable by a process of this invention into the free compound of the formula I or into another salt.

2. A process according to claim 1, which comprises removing $H-Z_1$ from a compound of the general formula

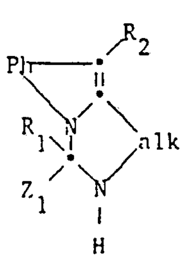

(II)

wherein $Z_1$ is free or modified hydroxy or mercapto, or from a salt thereof, to introduce an additional bond, or in a compound of the formula

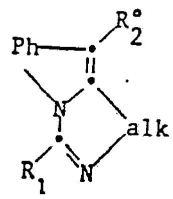

(X)

in which $R_2^o$ is a group of the formula —CH($R_3$)—$R_2''$, in which $R_3$ is hydrogen or lower alkyl, and $R_2''$ is functionally modified carboxy which differs from $R_2'$, or in a salt thereof, converting $R_2^o$ into the group $R_2$ by solvolysis, or to prepare a compound of the formula I, in which $R_2$ is a group of the formula —CH($R_3$)—$R_2'$, in which $R_2'$ is lower alkoxycarbonyl which is unsubstituted or substituted by phenyl or pyridyl, each in turn substituted by lower alkyl, lower alkoxy or halogen, or said lower alkoxycarbonyl is substituted by hydroxy or lower alkoxy, or $R_2'$ is carbamoyl which is unsubstituted or monosubstituted by hydroxy or amino, or mono- or disubstituted by lower alkyl or hydroxy-lower alkyl, or disubstituted by 4- to 7-membered lower alkylene or 3-oxa-, 3-thia- or 3-azaalkylene, $R_3$ is hydrogen or lower alkyl, alk is vinylene, and $R_1$ and Ph are as defined above, dehydrogenating a compound of the formula

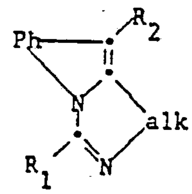

(I)

in which alk is ethylene, to remove hydrogen with simultaneous formation of an additional bond, and, if desired, converting a compound obtainable by a process of this invention into another compound of the formula I, or converting a free compound of the formula I obtainable by a process of this invention into a salt, or converting a salt obtainable by a process of this invention into the free compound of the formula I or into another salt.

3. A process according to either claim 1 or claim 2, which process comprises forming a starting material of the formula II, or a salt thereof, *in situ* under the reaction conditions, without isolation, by cyclising a compound of the formula

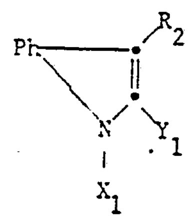

(IVa)

in which $X_1$ is hydrogen, $Y_1$ is a group of the formula —alk—NH—C(=$Z_1$)($R_1$), and $Z_1$ is oxo, and Ph, $R_1$ and $R_2$ are as defined in claim 1.

4. A process according to any one of claims 1 to 3 for the preparation of a compound of the formula I, in which $R_1$ is phenyl which is unsubstituted or substituted by lower alkyl, lower alkoxy, lower alkylthio, halo-lower alkylthio, lower alkanesulphinyl, halo-lower alkanesulphinyl, lower alkanesulphonyl, halo-lower alkanesulphonyl, sulphamoyl, N-mono- or N,N-di-lower alkylsulphamoyl and/or halogen, or is pyrrolyl, furyl, thienyl, thiazolyl, pyridyl or pyrimidyl, each unsubstituted or substituted by lower alkyl, lower alkoxy and/or halogen, $R_2$ is a group of the formula —CH($R_3$)—$R_2'$, in which $R_2'$ is carboxy, or is phenyl-lower or pyridyl-lower alkoxycarbonyl, each unsubstituted or substituted by lower alkyl, lower alkoxy or halogen, or is hydroxy-lower or lower alkoxy-lower alkoxycarbonyl, lower alkoxycarbonyl, carbamoyl, N-hydroxycarbamoyl, N-aminocarbamoyl, N-mono- or N,N-di-lower alkylcarbamoyl or N-monohydroxy- or N,N-dihydroxy-lower alkylcarbamoyl, or is carbamoyl substituted by 4- to 7-membered lower alkylene, or is 3-oxa-, 3-thia- or 3-aza-alkyleneaminocarbonyl, $R_3$ is hydrogen or lower alkyl, Ph is 1,2-phenylene which is unsubstituted or substituted by lower alkyl, lower alkoxy, halogen and/or trifluoromethyl, and alk is lower alkylene separating the methine group from the imino group by 1 to 3 carbon atoms or is lower alkenylene separating the methine group from the imino group by 2 carbon atoms ; or of a salt thereof, which process comprises starting from a compound of the formula II or III, V, VIII, X, XIa or I, in which the variables are as defined in claim 1.

5. A process according to any one of claims 1 to 3 for the preparation of a compound of the formula I, in which $R_1$ is phenyl which is unsubstituted or substituted by lower alkyl having up to and including 4 carbon atoms, by lower alkoxy having up to and including 4 carbon atoms, by halo-lower alkylthio having

up to and including 4 carbon atoms, by lower alkylthio having up to and including 4 carbon atoms, by halo-lower alkanesulphinyl having up to and including 4 carbon atoms, by lower alkanesulphinyl having up to and including 4 carbon atoms, by halo-lower alkanesulphonyl, by lower alkanesulphonyl having up to and including 4 carbon atoms, by sulphamoyl, by N-mono- or N,N-di-lower alkanesulphamoyl each having up to and including 4 carbon atoms in the alkyl radical, and/or by halogen having an atomic number of up to and including 35, or $R_1$ is pyridyl or thienyl, each unsubstituted or substituted by lower alkyl having up to and including 4 carbon atoms, by lower alkoxy having up to and including 4 carbon atoms, and/or by halogen having an atomic number of up to and including 35, $R_2$ is a group of the formula —CH($R_3$)—$R_2'$, in which $R_2'$ is carboxy, or is phenyl-lower or pyridyl-lower alkoxycarbonyl, each unsubstituted or substituted by lower alkyl having up to and including 4 carbon atoms, by lower alkoxy having up to and including 4 carbon atoms, and/or by halogen, or is lower alkoxycarbonyl, mono- or dihydroxy-lower alkoxycarbonyl, lower alkoxy-lower alkoxycarbonyl, N-hydroxy- or N-amino-carbamoyl, N-mono- or N,N-di-lower alkoxycarbamoyl, or carbamoyl, $R_3$ is hydrogen or lower alkyl having up to and including 4 carbon atoms, Ph is 1,2-phenylene which is unsubstituted or substituted by lower alkyl having up to and including 4 carbon atoms, by lower alkoxy having up to and including 4 carbon atoms, and/or by halogen having an atomic number of up to and including 35, and alk is 1,2-ethylene ; or of a salt thereof, which process comprises starting from a compound of the formula II or III, V, VIII, X or IXa, in which the variables are as defined in claim 1.

6. A process according to any one of claims 1 to 3 for the preparation of a compound of the formula I, in which $R_1$ is phenyl which is unsubstituted or substituted by halogen, lower alkylthio, lower alkanesulphinyl, lower alkanesulphonyl, halo-lower alkylthio and/or sulphamoyl, or is pyridyl or thienyl, $R_2$ is a group of the formula —$CH_2$—$R_2'$, in which $R_2'$ is carboxy, lower alkoxycarbonyl or carbamoyl, Ph is 1,2-phenylene which is unsubstituted or substituted by lower alkoxy, lower alkyl and/or halogen, and alk is 1,2-ethylene or vinylene ; or of a salt thereof, which process comprises starting from a compound of the formula II or III, V, VIII, X, XIa or I, in which the variables are as defined in claim 1.

7. A process according to any one of claims 1 to 3 for the preparation of a compound of the formula I, in which $R_1$ is phenyl which is unsubstituted or substituted by halogen having an atomic number of up to and including 35, by lower alkylthio having up to and including 4 carbon atoms, by lower alkanesulphinyl having up to and including 4 carbon atoms, by halo-lower alkylthio having up to and including 4 carbon atoms, and/or by sulphamoyl, or is pyridyl or thienyl, $R_2$ is a group of the formula —$CH_2$—$R_2'$, in which $R_2'$ is carboxy, or is lower alkoxycarbonyl having up to and including 5 carbon atoms, or carbamoyl, Ph is 1,2-phenylene which is unsubstituted or substituted by lower alkoxy having up to and including 4 carbon atoms, by lower alkyl having up to and including 4 carbon atoms, and/or by halogen having an atomic number of up to and including 35, and alk is 1,2-ethylene or vinylene ; or of a salt thereof, which process comprises starting from a compound of the formula II or III, V, VIII, X, XIa or I, in which the variables are as defined in claim 1.

8. A process according to any one of claims 1 to 3 for the preparation of a compound of the formula I, in which $R_1$ is phenyl which is unsubstituted or substituted in the p-position by halo-lower alkylthio having up to and including 4 carbon atoms, by lower alkylthio having up to and including 4 carbon atoms, by lower alkanesulphinyl having up to and including 4 carbon atoms, or by halogen having an atomic number of up to and including 35, or in 4-position by halogen having an atomic number of up to and including 35, or is pyridyl or thienyl, $R_2$ is a group of the formula —$CH_2$—$R_2'$, in which $R_2'$ is carboxy, or is lower alkoxycarbonyl having up to and including 5 carbon atoms, or carbamoyl, Ph is 1,2-phenylene which is unsubstituted or substituted in the 4-position relative to the bonded nitrogen atom by lower alkoxy having up to and including 4 carbon atoms, in the 3- and 5-positions by lower alkyl having up to and including 4 carbon atoms, or in the 4-position by halogen having an atomic number of up to and including 35, and alk is 1,2-ethylene or vinylene ; or of a salt thereof, which process comprises starting from a compound of the formula II or III, V, VIII, X, XIa or I, in which the variables are as defined in claim 1.

9. A process according to any one of claims 1 to 3 for the preparation of a compound of the formula I, in which $R_1$ is phenyl which is unsubstituted or substituted in the p-position by lower alkylthio having up to and including 4 carbon atoms, by lower alkanesulphinyl having up to and including 4 carbon atoms, or by halogen having an atomic number of up to and including 35, $R_2$ is lower alkoxycarbonylmethyl having up to and including 6 carbon atoms, e.g. ethoxycarbonylmethyl, Ph is 1,2-phenylene which is unsubstituted or substituted in the p-position relative to the nitrogen atom by lower alkoxy having up to and including 4 carbon atoms, or by halogen having an atomic number of up to and including 35, and alk is 1,2-ethylene ; or of a salt thereof, which process comprises starting from a compound of the formula II or III, V, VIII, X or XIa, in which the variables are as defined in claim 1.

10. A process according to any one of claims 1 to 3 for the preparation of a compound of the formula I, in which $R_1$ is phenyl which is unsubstituted or substituted in the p-position by lower alkylthio having up to and including 4 carbon atoms, by lower alkanesulphinyl having up to and including 4 carbon atoms, or by halogen having an atomic number of up to and including 35, $R_2$ is carboxymethyl, Ph is 1,2-phenylene which is unsubstituted or substituted in the p-position relative to the nitrogen atom by lower alkoxy having up to and including 4 carbon atoms or by halogen having an atomic number of up to and including 35, and alk is 1,2-ethylene ; or a salt thereof, which process comprises starting from a compound of the formula II or III, V, VIII, X or XIa, in which the variables are as defined in claim 1.

11. A process according to any one of claims 1 to 3 for the preparation of ethyl 7-methoxy-1-(p-chlorophenyl)-3,4-dihydropyrimido [1,6-a] indoleacetate, or of a salt thereof, which process comprises starting from a compound of the formula II or III, V, VIII, X or XIa.

12. A process according to any one of claims 1 to 3 for the preparation of ethyl 1-phenyl-3,4-dihydropyrimido [1,6-a] indole-5-acetate, or of a salt thereof, which process comprises starting from a compound of the formula II or III, V, VIII, X or XIa.

13. A process according to any one of claims 1 to 3 for the preparation of ethyl 7-fluoro-1-(p-methylthiophenyl)-3,4-dihydropyrimido [1,6-a] indole-5-acetate, or of a salt thereof, which process comprises starting from a compound of the formula II or III, V, VIII, X or XIa.

14. A process according to any one of claims 1 to 3 for the preparation of ethyl 6,8-dimethyl-1-phenyl-3,4-dihydropyrimido [1,6-a] indole-5-acetate, or of a salt thereof, which process comprises starting from a compound of the formula II or III, V, VIII, X or XIa.

15. A process according to any one of claims 1 to 3 for the preparation of ethyl 1-(3-sulphamoyl-4-chlorophenyl)-3,4-dihydropyrimido [1,6-a] indole-5-acetate, or of a salt thereof, which process comprises starting from a compound of the formula II or III, V, VIII, X or XIa.

16. A process according to any one of claims 1 to 3 for the preparation of ethyl 1-(2,6-dichlorophenyl)-3,4-dihydropyrimido [1,6-a] indole-5-acetate, or of a salt thereof, which process comprises starting from a compound of the formula II or III, V, VIII, X or XIa.

17. A process according to any one of claims 1 to 3 for the preparation of ethyl 1-(2-picolinyl)-3,4-dihydropyrimido [1,6-a] indole-5-acetate, or of a salt thereof, which process comprises starting from a compound of the formula II or III, V, VIII, X or XIa.

18. A process according to any one of claims 1 to 3 for the preparation of ethyl 1-(2-thienyl)-3,4-dihydropyrimido [1,6-a] indole-5-acetate, or of a salt thereof, which process comprises starting from a compound of the formula II or III, V, VIII, X or XIa.

19. A process according to any one of claims 1 to 3 for the preparation of 7-methoxy-1-(p-chlorophenyl)-3,4-dihydropyrimido [1,6-a] indole-5-acetic acid, or of a salt thereof, which process comprises starting from a compound of the formula II or III, V, VIII, X or XIa.

20. A process according to any one of claims 1 to 3 for the preparation of 1-phenyl-3,4-dihydropyrimido [1,6-a] indole-5-acetic acid, or of a salt thereof, which process comprises starting from a compound of the formula II or III, V, VIII, X or XIa.

21. A process according to any one of claims 1 to 3 for the preparation of 7-fluoro-1-(p-methylthiophenyl)-3,4-dihydropyrimido [1,6-a] indole-5-acetic acid, or of a salt thereof, which process comprises starting from a compound of the formula II or III, V, VIII, X or XIa.

22. A process according to any one of claims 1 to 3 for the preparation of 7-fluoro-1-(p-methylsulphinylphenyl)-3,4-dihydropyrimido [1,6-a] indole-5-acetic acid, or of a salt thereof, which process comprises starting from a compound of the formula II or III, V, VIII, X or XIa.

23. A process according to any one of claims 1 to 3 for the preparation of 6,8-dimethyl-1-phenyl-3,4-dihydropyrimido [1,6-a] indole-5-acetic acid, or of a salt thereof, which process comprises starting from a compound of the formula II or III, V, VIII, X or XIa.

24. A process according to any one of claims 1 to 3 for the preparation of 1-(3-sulphamoyl-4-chlorophenyl)-3,4-dihydropyrimido [1,6-a] indole-5-acetic acid, or of a salt thereof, which process comprises starting from a compound of the formula II or III, V, VIII, X or XIa.

25. A process according to any one of claims 1 to 3 for the preparation of 1-(2,6-dichlorophenyl)-3,4-dihydropyrimido [1,6-a] indole-5-acetic acid, or of a salt thereof, which process comprises starting from a compound of the formula II or III, V, VIII, X or XIa.

26. A process according to any one of claims 1 to 3 for the preparation of 1-(2-thienyl)-3,4-dihydropyrimido [1,6-a] indole-5-acetic acid, or of a salt thereof, which process comprises starting from a compound of the formula II or III, V, VIII, X or XIa.

27. A process according to any one of claims 1 to 3 for the preparation of 1-phenyl-3,4-dihydropyrimido [1,6-a] indole-5-acetamide, or of a salt thereof, which process comprises starting from a compound of the formula II or III, V, VIII, X or XIa.

28. A process according to any one of claims 1 to 3 for the preparation of 1-(3-sulphamoyl-4-chlorophenyl)-3,4-dihydropyrimido [1,6-a] indole-5-acetamide, or of a salt thereof, which process comprises starting from a compound of the formula II or III, V, VIII, X or XIa.

29. A process according to any one of claims 1 to 3 for the preparation of ethyl 1-(p-methylsulphoxyphenyl)-3,4-dihydropyrimido [1,6-a] indole-5-acetate, or of a salt thereof, which process comprises starting from a compound of the formula II or III, V, VIII, X or XIa.

30. A process according to any one of claims 1 to 3 for the preparation of ethyl 7-fluoro-1-(p-chloromethylthiophenyl)-3,4-dihydropyrimido [1,6-a] indole-5-acetate, or of a salt thereof, which process comprises starting from a compound of the formula II or III, V, VIII, X or XIa.

31. A process according to any one of claims 1 to 3 for the preparation of ethyl 7-fluoro-1-(p-methylsulphoxyphenyl-3,4-dihydropyrimido [1,6-a] indole-5-acetate, or of a salt thereof, which process comprises starting from a compound of the formula II or III, V, VIII, X or XIa.

32. A process according to any one of claims 1 to 3 for the preparation of ethyl 7-fluoro-1-(p-methylthiophenyl)-pyrimido [1,6-a] indole-5-acetate, or of a salt thereof, which process comprises starting

from the corresponding compound of the formula I.

33. A process according to any one of claims 1 to 3 for the preparation of ethyl 1-phenylpyrimido [1,6-a] indole-5-acetate, or of a salt thereof, which process comprises starting from the corresponding compound of the formula I.

34. A process according to any one of claims 1 to 3 for the preparation of ethyl 7-methoxy-1-(p-chlorophenyl) pyrimido [1,6-a] indole-5-acetate, or of a salt thereof, which process comprises starting from the corresponding compound of the formula I.

35. A process according to any one of claims 1 to 3 for the preparation of 7-fluoro-1-(p-methylthiophenyl) pyrimido [1,6-a] indole-5-acetic acid, or of a salt thereof, which process comprises starting from the corresponding compound of the formula I.

36. A process for the preparation of a pharmaceutical composition, which process comprises mixing a compound according to any one of claims 1 to 35 in the free form or in the form of a pharmaceutically acceptable salt with customary pharmaceutical auxiliaries and/or carriers.

**Revendications** (pour les Etats contractants : BE, CH, DE, FR, IT, LI, LU, NL, SE)

1. Amidines d'acides carboxyliques pontées en N,N' de formule générale

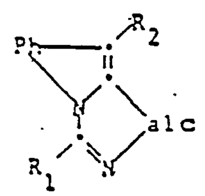

(I)

où $R_1$ représente un phényle éventuellement substitué par un alcoyle inférieur, un alcoxy inférieur, un alcoyle inférieur-thio, un alcane inférieur-sulfinyle ou un alcane inférieur sulfonyle contenant éventuellement un halogène, un sulfamoyle éventuellement mono- ou disubstitué par un alcoyle inférieur et/ou un halogène ou un hétérocycle monocyclique à 5 ou 6 chaînons contenant éventuellement un alcoyle inférieur, un alcoxy inférieur et/ou un halogène et présentant comme hétéroatome un azote, un oxygène ou un soufre ou selon les cas un azote et en outre un soufre ou un oxygène, $R_2$ est un 1-carboxyalcoyle inférieur de formule $-CH(R_3)-R_2'$, où $R_2'$ est un carboxy, un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia- ou 3-aza-alcoylène, $R_3$ représente un hydrogène ou un alcoyle inférieur, Ph représente un 1,2-phénylène éventuellement substitué par un alcoyle inférieur, un alcoxy inférieur, un halogène et/ou un trifluorométhyle et alc est un alcoylène inférieur séparant le groupe méthine du groupe imino par de 1 à 3 atomes de carbone ou un alcényle le séparant par 2 atomes de carbone, et de leurs sels, où il faut comprendre sous les radicaux décrits comme « inférieurs » ceux qui contiennent jusqu'à 7 atomes compris.

2. Composés de formule I selon la revendication 1, où $R_1$ représente un phényle éventuellement substitué par un alcoyle inférieur, un alcoxy inférieur, un alcoyle inférieur-thio, un halogène-alcoyle inférieur-thio, un alcane inférieur-sulfinyle, un halogène-alcane inférieur-sulfinyle, un alcane inférieur-sulfonyle, un halogène-alcane inférieur-sulfonyle, un sulfamoyle, un N-mono- ou N,N-dialcoyle inférieur-sulfamoyle et/ou un halogène ou un pyrrolyle, furyle, thiényle, thiazolyle, pyridyle ou pyrimidyle éventuellement substitué par un alcoyle inférieur, un alcoxy inférieur et/ou un halogène, $R_2$ est un groupe de formule $-CH(R_3)-R_2'$, où $R_2'$ est un carboxy, un phényl- ou pyridylalcoxy-carbonyle éventuellement substitué par un alcoyle inférieur, un alcoxy inférieur ou un halogène, un hydroxy- ou alcoxy inférieur-alcoxy inférieur-carbonyle, un alcoxy inférieur-carbonyle, un carbamoyle, un N-hydroxy- ou un N-amino-carbamoyle, un N-mono- ou N,N-dialcoyle inférieur-carbamoyle ou -hydroxyalcoyle inférieur-carbamoyle ou un carbamoyle substitué par un alcoylène inférieur à 4 à 7 chaînons, ou un 3-oxa-, 3-thia- ou 3-azaalcoylènaminocarbonyle, $R_3$ représente un hydrogène ou un alcoyle inférieur, Ph représente un 1,2-phénylène éventuellement substitué par un alcoyle inférieur, un alcoxy inférieur, un halogène et/ou un trifluorométhyle et alc est un alcoylène inférieur séparant le groupe méthine du groupe imino par 1 à 3 atomes de carbone ou un alcénylène inférieur le séparant par 2 atomes de carbone, ainsi que leurs sels.

3. Composés de formule I selon la revendication 1, où $R_1$ représente un phényle éventuellement substitué par un alcoyle inférieur ayant jusqu'à 4 atomes de carbone compris, un alcoxy inférieur ayant jusqu'à 4 atomes de carbone compris, un halogènealcoyle inférieur-thio ayant jusqu'à 4 atomes de carbone compris, un alcoyle inférieur-thio ayant jusqu'à 4 atomes de carbone compris, un halogène-alcane inférieur-sulfinyle ayant jusqu'à 4 atomes de carbone compris, un alcane inférieur-sulfinyle ayant jusqu'à 4 atomes de carbone compris, un halogène-alcane inférieur-sulfonyle, un alcane inférieur-sulfonyle ayant jusqu'à 4 atomes de carbone compris, un sulfamoyle, un N-mono- ou N,N-dialcane

inférieur-sulfamoyle ayant à chaque fois jusqu'à 4 atomes de carbone compris dans la fraction alcoyle et/ou par un halogène de numéro atomique allant jusqu'à 35 compris, ou un pyridyle ou thiényle éventuellement substitué par un alcoyle inférieur ayant jusqu'à 4 atomes de carbone compris, par un alcoxy inférieur ayant jusqu'à 4 atomes de carbone compris, et/ou un halogène de numéro atomique allant jusqu'à 35 compris, $R_2$ représente un groupe de formule —CH($R_3$)—$R_2'$, où $R_2'$ représente un carboxy, $R_2'$ est un phényl- ou pyridyl-alcoxy inférieur-carbonyle éventuellement substitué par un alcoyle inférieur ayant jusqu'à 4 atomes de carbone compris, un alcoxy inférieur ayant jusqu'à 4 atomes de carbone compris, et/ou un halogène, un alcoxy inférieur-carbonyle, un mono- ou dihydroxy-alcoxy inférieur-carbonyle, un alcoxy inférieur-alcoxy inférieur-carbonyle, un N-hydroxy- ou N-amino-carbamoyle, un N-mono- ou N,N-dialcoxy inférieur-carbamoyle ou un carbamoyle, $R_3$ représente un hydrogène ou un alcoyle inférieur ayant jusqu'à 4 atomes de carbone compris, Ph représente un 1,2-phénylène éventuellement substitué par un alcoyle inférieur ayant jusqu'à 4 atomes de carbone compris, un alcoxy inférieur ayant jusqu'à 4 atomes de carbone compris et/ou un halogène de numéro atomique allant jusqu'à 35 compris, et alc est un 1,2-éthylène, ainsi que leurs sels.

4. Composés de formule I selon la revendication 1, où $R_1$ représente un phényle éventuellement substitué par un halogène, un alcoyle inférieur-thio, un alcane inférieur-sulfinyle, un alcane inférieur-sulfonyle, un halogène-alcoyle inférieur-thio et/ou un sulfamoyle, ou un pyridyle ou un thiényle ayant les mêmes substituants, $R_2$ est un groupe de formule —$CH_2$—$R_2'$ où $R_2'$ représente un carboxy, un alcoxy inférieur-carbonyle ou un carbamoyle, Ph représente un 1,2-phénylène éventuellement substitué par un alcoxy inférieur, un alcoyle inférieur et/ou un halogène et alc est un 1,2-éthylène ou un vinylène, ainsi que leurs sels.

5. Composés de formule I selon la revendication 1, où $R_1$ représente un phényle, pyridyle ou thiényle éventuellement substitué par un halogène de numéro atomique allant jusqu'à 35 compris, un alcoyle inférieur-thio ayant jusqu'à 4 atomes de carbone compris, un alcane inférieur-sulfinyle ayant jusqu'à 4 atomes de carbone compris, un halogène-alcoyle inférieur-thio ayant jusqu'à 4 atomes de carbone compris et/ou un sulfamoyle, $R_2$ est un groupe de formule —$CH_2$—$R_2'$, où $R_2'$ représente un carboxy, un alcoxy inférieur-carbonyle ayant jusqu'à 5 atomes de carbone compris ou un carbamoyle, Ph représente un 1,2-phénylène éventuellement substitué par un alcoxy inférieur ayant jusqu'à 4 atomes de carbone compris, un alcoyle inférieur ayant jusqu'à 4 atomes de carbone compris ou un halogène de numéro atomique allant jusqu'à 35 compris et alc est un 1,2-éthylène ou un vinylène, ainsi que leurs sels.

6. Composés de formule I selon la revendication 1, où $R_1$ représente un phényle, pyridyle ou thiényle éventuellement substitué par un halogène-alcoyle inférieur-thio ayant jusqu'à 4 atomes de carbone compris, un alcoyle inférieur-thio ayant jusqu'à 4 atomes de carbone compris, un alcane inférieur-sulfinyle ayant jusqu'à 4 atomes de carbone, ou un halogène de numéro atomique allant jusqu'à 35 compris, en position p ou par un sulfamoyle en position 3 et en outre par un halogène de numéro atomique allant jusqu'à 35 compris en position 4, $R_2$ représente un groupe de formule —$CH_2$—$R_2'$ où $R_2'$ est un carboxy, un alcoxy inférieur-carbonyle ayant jusqu'à 5 atomes de carbone compris ou un carbamoyle, Ph représente un 1,2-phénylène éventuellement substitué par rapport à l'atome d'azote lié en position 4 par un alcoxy inférieur ayant jusqu'à 4 atomes de carbone compris, en positions 3 et 5 par un alcoyle inférieur ayant jusqu'à 4 atomes de carbone compris ou en position 4 par un halogène de numéro atomique allant jusqu'à 35 compris et alc est un 1,2-éthylène ou un vinylène, ainsi que leurs sels.

7. Composés de formule I selon la revendication 1, où $R_1$ représente un phényle éventuellement substitué en position p par un alcoyle inférieur-thio ayant jusqu'à 4 atomes de carbone compris, par un alcane inférieur-sulfinyle ayant jusqu'à 4 atomes de carbone compris ou un halogène de numéro atomique allant jusqu'à 35 compris, $R_2$ représente un alcoxy inférieur-carbonylméthyle ayant jusqu'à 6 atomes de carbone compris, Ph représente un 1,2-phénylène éventuellement substitué en position p par rapport à l'atome d'azote par un alcoxy inférieur ayant jusqu'à 4 atomes de carbone compris ou un halogène de numéro atomique allant jusqu'à 35 compris et alc représente un 1,2-éthylène, ainsi que leurs sels.

8. Composés de formule I selon la revendication 1, où $R_1$ représente un phényle éventuellement substitué en position p par un alcoyle inférieur-thio ayant jusqu'à 4 atomes de carbone compris, par un alcane inférieur-sulfinyle ayant jusqu'à 4 atomes de carbone compris ou un halogène de numéro atomique allant jusqu'à 35 compris, $R_2$ est un carboxyméthyle, Ph représente un 1,2-phénylène éventuellement substitué en position p par rapport à l'atome d'azote par un alcoxy inférieur ayant jusqu'à 4 atomes de carbone compris ou un halogène de numéro atomique allant jusqu'à 35 compris et alc représente un 1,2-éthylène, ainsi que leurs sels.

9. Ester éthylique de l'acide 7-méthoxy-1-(p-chloro-phényl)-3,4-dihydro-pyrimido [1,6-a] indol-acétique ou un de ses sels.

10. Ester éthylique de l'acide 1-phényl-3,4-dihydro-pyrimido [1,6-a] indol-acétique ou un de ses sels.

11. Ester éthylique de l'acide 7-fluoro-1-(p-méthyl-thio-phényl)-3,4-dihydro-pyrimido [1,6-a] indol-5-acétique ou un de ses sels.

12. Ester éthylique de l'acide 6,8-diméthyl-1-phényl-3,4-dihydro-pyrimido [1,6-a] indol-5-acétique ou un de ses sels.

13. Ester éthylique de l'acide 1-(3-sulfamoyl-4-chlorophényl)-3,4-dihydro-pyrimido [1,6-a] indol-5-acétique ou un de ses sels.

14. Ester éthylique de l'acide 1-(2,6-dichlorophényl)-3,4-dihydro-pyrimido [1,6-a] indol-5-acétique ou un de ses sels.

15. Ester éthylique de l'acide 1-(2-picolinyl)-3,4-dihydro-pyrimido [1,6-a] indol-5-acétique ou un de ses sels.

16. Ester éthylique de l'acide 1-(2-thiényl)-3,4-dihydro-pyrimido [1,6-a] indol-5-acétique ou un de ses sels.

17. Acide 7-méthoxy-1-(p-chlorophényl)-3,4-dihydro-pyrimido [1,6-a] indol-5-acétique ou un de ses sels.

18. Acide 1-phényl-3,4-dihydro-pyrimido [1,6-a] indol-5-acétique ou un de ses sels.

19. Acide 7-fluoro-1-(p-méthylthio-phényl)-3,4-dihydro-pyrimido [1,6-a] indol-5-acétique ou un de ses sels.

20. Acide 7-fluoro-1-(p-méthylsulfinyl-phényl)-3,4-dihydro-pyrimido [1,6-a] indol-5-acétique ou un de ses sels.

21. Acide 6,8-diméthyl-1-phényl-3,4-dihydro-pyrimido [1,6-a] indol-5-acétique ou un de ses sels.

22. Acide 1-(3-sulfamoyl-4-chloro-phényl)-3,4-dihydro-pyrimido [1,6-a] indol-5-acétique ou un de ses sels.

23. Acide 1-(2,6-dichlorophényl)-3,4-dihydro-pyrimido [1,6-a] indol-5-acétique ou un de ses sels.

24. Acide 1-(2-thiényl)-3,4-dihydro-pyrimido [1,6-a] indol-5-acétique ou un de ses sels.

25. Amide de l'acide 1-phényl-3,4-dihydro-pyrimido [1,6-a] indol-5-acétique ou un de ses sels.

26. 1-(3-sulfamoyl-4-chloro-phényl)-3,4-dihydro-pyrimido [1,6-a] indol-5-acétamide ou un de ses sels.

27. Ester éthylique de l'acide 1-(p-méthyl-sulfoxy-phényl)-3,4-dihydro-pyrimido [1,6-a] indol-5-acétique ou un de ses sels.

28. Ester éthylique de l'acide 7-fluoro-1-(p-chloro-méthylthio-phényl)-3,4-dihydro-pyrimido [1,6-a] indol-5-acétique ou un de ses sels.

29. Ester éthylique de l'acide 7-fluoro-1-(p-méthyl-sulfoxyphényl)-3,4-dihydro-pyrimido [1,6-a] indol-5-acétique ou un de ses sels.

30. Ester éthylique de l'acide 7-fluoro-1-(p-méthyl-thio-phényl)-pyrimido [1,6-a] indol-5-acétique ou un de ses sels.

31. Ester éthylique de l'acide 1-phényl-pyrimido [1,6-a] indol-5-acétique ou un de ses sels.

32. Ester éthylique de l'acide 7-méthoxy-1-(p-chloro-phényl)-pyrimido [1,6-a] indol-5-acétique ou d'un de ses sels.

33. Acide 7-fluoro-1-(p-méthylthiophényl)-pyrimido [1,6-a] indol-5-acétique ou un de ses sels.

34. Composés de formule I selon l'une des revendications 1 à 33 comme agent anti-inflammatoire et/ou analgésique.

35. Préparations pharmaceutiques contenant un composé de formule I selon l'une des revendications 1 à 3 et 8 à 27 sous forme libre ou sous la forme d'un sel pharmaceutiquement acceptable avec les additifs et supports pharmaceutiques habituels.

36. Préparations pharmaceutiques contenant un composé de formule I selon l'une des revendications 4 à 7 et 28 à 34 sous forme libre ou sous la forme d'un sel pharmaceutiquement acceptable avec les supports et additifs pharmaceutiques habituels.

37. Composés de formule I selon l'une des revendications 1 à 34 aux fins d'application dans un procédé de traitement thérapeutique du corps humain ou animal.

38. Composé de formule I selon l'une des revendications 1 à 34 aux fins de préparation de préparations pharmaceutiques.

39. Composé de formule I selon l'une des revendications 1 à 34 aux fins d'application selon la revendication 37 pour le traitement des maladies inflammatoires et/ou des états douloureux.

40. Procédé de préparation de composés de formule

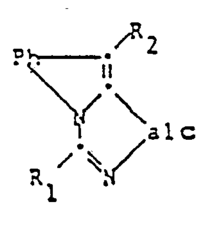

(I)

où $R_1$ représente un phényle éventuellement substitué par un alcoyle inférieur, un alcoxy inférieur, un alcoyle inférieur-thio, un alcane inférieur-sulfinyle ou un alcane inférieur-sulfonyle contenant éventuellement un halogène, un sulfamoyle éventuellement mono- ou disubstitué par un alcoyle inférieur et/ou un halogène ou un hétéroaryle monocyclique à 5 ou 6 chaînons contenant éventuellement un alcoyle inférieur, un alcoxy inférieur et/ou un halogène et présentant comme hétéroatome un azote, un oxygène ou un soufre ou selon les cas un azote et en outre un soufre ou un oxygène, $R_2$ est un 1-carboxyalcoyle inférieur de formule —$CH(R_3)$—$R_2'$, où $R_2'$ est un carboxy, un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par

un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia- ou 3-aza-alcoylène, $R_3$ représente un hydrogène ou un alcoyle inférieur, Ph représente un 1,2-phénylène éventuellement substitué par un alcoyle inférieur, un alcoxy inférieur, un halogène et/ou un trifluorométhyle et alc est un alcoylène inférieur séparant le groupe méthine du groupe imino par de 1 à 3 atomes de carbone ou un alcényle le séparant par 2 atomes de carbone, et de leurs sels, où il faut comprendre sous les radicaux décrits comme « inférieurs » ceux qui contiennent jusqu'à 7 atomes de carbone compris, caractérisé en ce qu'on sépare à partir des composés de formule générale

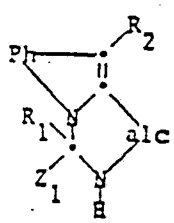 (II) ou 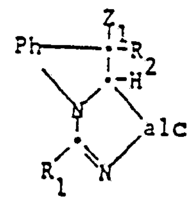 (III)

où $Z_1$ représente un hydroxy ou mercapto éventuellement modifié, ou de leurs sels, avec introduction d'une liaison supplémentaire, $H—Z_1$, ou en ce qu'on isomérise les composés de formule

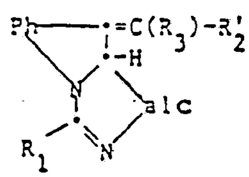 (V)

où $R_2'$ est un carboxy, un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, disubstitué par un alcoylène inférieur à 4 à 7 chaînons ou un 3-oxa-, 3-thia- ou 3-aza-alcoylène, $R_3$ représente un hydrogène ou un alcoyle inférieur, ou leurs sels, ou en ce qu'on cyclise un composé de formule générale

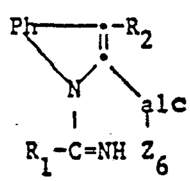 (VIII)

où $Z_6$ représente un hydroxy ou selon les cas un mercapto ou amino éventuellement fonctionnellement modifié, ou un de ses sels, ou en ce qu'on transforme par solvolyse ou oxydation $R_2°$ en le groupe $R_2$ dans un composé de formule

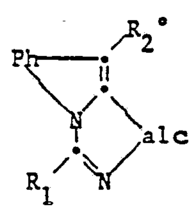 (X)

où $R_2°$ représente un groupe de formule $—CH(R_3)—R_2''$ et où $R_3$ est un hydrogène ou un alcoyle inférieur et $R_2''$ est un carboxy fonctionnellement modifié différent de $R_2'$, un groupe $—C(=O)—N^{\oplus}{}_2B^{\ominus}$, où $B^{\ominus}$ est l'anion d'un acide minéral, ou un méthyle éventuellement oxydé jusqu'à l'étape du formyle, ou ses sels, ou selon les cas pour préparer des composés de formule I où $R_2$ est un 1-carboxyalcoyle inférieur amidé de formule $—CH(R_3)—R_2'$, où $R_2'$ est un carbamoyle éventuellement mono-substitué par un hydroxy ou un amino, mono- ou di-substitué par un alcoyle inférieur ou un hydroxy-alcoyle inférieur, disubstitué par un alcoylène inférieur à 4 à 7 chaînons ou un 3-oxa-, 3-thia- ou 3-aza-alcoylène, $R_3$ représente un hydrogène ou un alcoyle inférieur, et Ph, $R_1$ et alc ont les significations données ci-dessus, caractérisé par l'addition d'eau et la séparation d'un proton sur des composés de formule

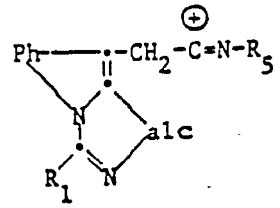

(XIa)

où $R_5$ représente un alcoyle inférieur éventuellement substitué ou un phényle, ou pour préparer des composés de formule I, où $R_2$ est un groupe de formule —$CH(R_3)$—$R_2'$, où $R_2'$ est un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxyalcoyle inférieur, disubstitué par un alcoylène inférieur à 4 à 7 chaînons ou un 3-oxa-, 3-thia- ou 3-aza-alcoylène, $R_3$ représente un hydrogène ou un alcoyle inférieur, alc représenté un vinylène et $R_1$ et Ph ont les significations données ci-dessus, caractérisé en ce qu'on déshydrogène des composés de formule

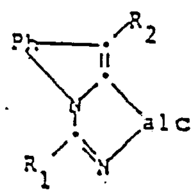

(I)

où alc représente un éthylène, avec séparation d'hydrogène et formation simultanée d'une liaison supplémentaire et si on le désire en ce qu'on transforme un composé obtenu selon l'invention en un autre composé de formule I ou un composé libre de formule I obtenu selon l'invention en un sel ou un sel obtenu selon le procédé en le composé libre de formule I ou en un autre sel.

41. Procédé selon la revendication 40 de préparation de composés de formule I selon la revendication 1, caractérisé en ce qu'on sépare H—$Z_1$ dans des composés de formule générale

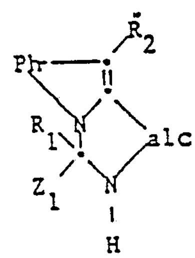

(II)

où $Z_1$ représente un hydroxy ou mercapto éventuellement modifié, ou leurs sels avec introduction d'une liaison supplémentaire ou en ce qu'on transforme par solvolyse $R_2^o$ en le groupe $R_2$ dans un composé de formule

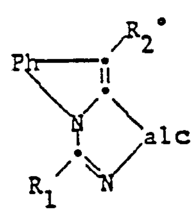

(X)

où $R_2^o$ représente un groupe de formule —$CH(R_3)$—$R_2''$ et où $R_3$ représente un hydrogène ou un alcoyle inférieur et $R_2''$ représente un carboxy fonctionnellement modifié différent de $R_2'$, ou ses sels, ou selon les cas en ce que pour préparer des composés de formule I où $R_2$ est un groupe de formule —$CH(R_3)$—$R_2'$, où $R_2'$ est un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, disubstitué par un alcoylène inférieur à 4 à 7 chaînons ou un 3-oxa-, 3-thia- ou 3-aza-alcoylène, $R_3$ représente un hydrogène ou un alcoyle inférieur, alc représente un vinylène et $R_1$ et Ph ont les significations données ci-dessus, caractérisé en ce qu'on déshydrogène des composés de formule

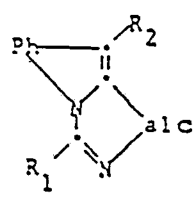

(I)

où alc représente un éthylène, avec séparation d'hydrogène et formation simultanée d'une liaison supplémentaire et si on le désire en ce qu'on transforme un composé obtenu selon l'invention en un autre composé de formule I ou un composé libre de formule I obtenu selon l'invention en un sel ou un sel obtenu selon le procédé en le composé libre de formule I ou en un autre sel.

42. Les composés obtenus selon le procédé d'une des revendications 40-41.

**Revendications** (pour l'Etat contractant AT)

1. Procédé de préparation d'amidines d'acides carboxyliques pontées en N,N' de formule générale

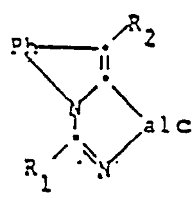

(I).

où $R_1$ représente un phényle éventuellement substitué par un alcoyle inférieur, un alcoxy inférieur, un alcoyle inférieur-thio, un alcane inférieur-sulfinyle ou un alcane inférieur-sulfonyle contenant éventuellement un halogène, un sulfamoyle éventuellement mono- ou disubstitué par un alcoyle inférieur et/ou un halogène ou un hétéroaryle monocyclique à 5 ou 6 chaînons contenant éventuellement un alcoyle inférieur, un alcoxy inférieur et/ou un halogène et présentant comme hétéroatome un azote, un oxygène ou un soufre ou selon les cas un azote et en outre un soufre ou un oxygène, $R_2$ est un 1-carboxyalcoyle inférieur de formule $-CH(R_3)-R_2'$, où $R_2'$ est un carboxy, un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia ou 3-aza-alcoylène, $R_3$ représente un hydrogène ou un alcoyle inférieur, Ph représente un 1,2-phénylène éventuellement substitué par un alcoyle inférieur, un alcoxy inférieur, un halogène et/ou un trifluorométhyle et alc est un alcoylène inférieur séparant le groupe méthine du groupe imino par de 1 à 3 atomes de carbone ou un alcényle le séparant par 2 atomes de carbone, et de leurs sels, où il faut comprendre sous les radicaux décrits comme « inférieurs » ceux qui contiennent jusqu'à 7 atomes de carbone compris, caractérisé en ce qu'on sépare à partir des composés de formule générale

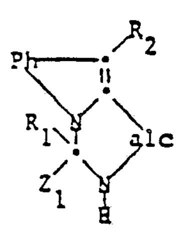

(II) ou

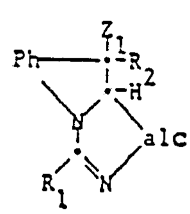

(III)

où $Z_1$ représente un hydroxy ou mercapto éventuellement modifié, ou de leurs sels, avec introduction d'une liaison supplémentaire, $H-Z_1$, ou en ce qu'on isomérise les composés de formule

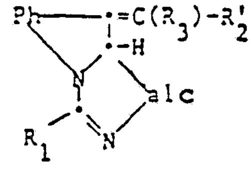

(V)

où $R_2'$ est un carboxy, un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa, 3-thia- ou 3-aza-alcoylène, $R_3$ représente un hydrogène ou un alcoyle inférieur, ou leurs sels, ou en ce qu'on cyclise un composé de formule générale

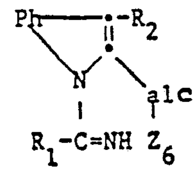

(VIII),

où $Z_6$ représente un hydroxy ou selon les cas un mercapto ou amino éventuellement fonctionnellement modifié, ou un de ses sels, ou en ce qu'on transforme par solvolyse ou oxydation $R_2^o$ en le groupe $R_2$ dans un composé de formule

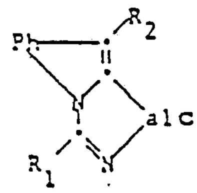

(X),

où $R_2^o$ représente un groupe de formule —$CH(R_3)$—$R_2''$ et où $R_3$ est un hydrogène ou un alcoyle inférieur et $R_2''$ est un carboxy fonctionnellement modifié différent de $R_2'$, un groupe de formule —$C(=O)$—$N_2^{\oplus}B^{\ominus}$, où $B^{\ominus}$ est l'anion d'un acide minéral, ou un méthyle éventuellement oxydé jusqu'à l'étape de formyle, ou ses sels, ou selon les cas pour préparer des composés de formule I où $R_2$ est un 1-carboxyalcoyle inférieur amidé de formule —$CH(R_3)$—$R_2'$, où $R_2'$ est un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia- ou 3-aza-alcoylène, $R_3$ représente un hydrogène ou un alcoyle inférieur, et Ph, $R_1$ et alc ont les significations données ci-dessus, caractérisé par l'addition d'eau et la séparation d'un proton sur des composés de formule

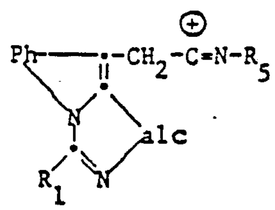

(XIa)

où $R_5$ représente un alcoyle inférieur éventuellement substitué ou un phényle, ou pour préparer des composés de formule I, où $R_2$ est un groupe de formule —$CH(R_3)$—$R_2'$, où $R_2'$ est un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia- ou 3-aza-alcoylène, $R_3$ représente un hydrogène ou un alcoyle inférieur, alc représente un vinylène et $R_1$ et Ph ont les significations données ci-dessus, caractérisé en ce qu'on déshydrogène des composés de formule

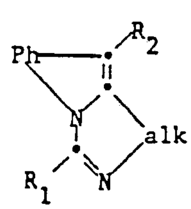

(I)

où alc représente un éthylène, avec séparation d'hydrogène et formation simultanée d'une liaison

supplémentaire et si on le désire en ce qu'on transforme un composé obtenu selon l'invention en un autre composé de formule I ou un composé libre de formule I obtenu selon l'invention en un sel ou un sel obtenu selon le procédé en le composé libre de formule I ou en un autre sel.

2. Procédé selon la revendication 1, caractérisé en ce qu'on sépare $H—Z_1$ dans des composés de formule générale

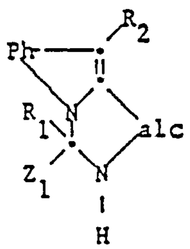

(II)

où $Z_1$ représente un hydroxy ou mercapto éventuellement modifié, ou leurs sels avec introduction d'une liaison supplémentaire ou en ce qu'on transforme par solvolyse $R_2°$ en le groupe $R_2$ dans un composé de formule

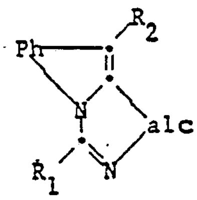

(X)

où $R_2°$ représente un groupe de formule $—CH(R_3)—R_2''$ et où $R_3$ représente un hydrogène ou un alcoyle inférieur et $R_2''$ représente un carboxy fonctionnellement modifié différent de $R_2'$, ou ses sels, ou selon les cas en ce que pour préparer des composés de formule I où $R_2$ est un groupe de formule $—CH(R_3)—R_2'$, où $R_2'$ est un alcoxy inférieur-carbonyle éventuellement substitué par un phényle ou selon les cas un pyridyle contenant un alcoyle inférieur, un alcoxy inférieur ou un halogène, un hydroxy ou un alcoxy inférieur ou un carbamoyle éventuellement mono-substitué par un hydroxy ou un amino, mono- ou di-substitué par un alcoyle inférieur ou un hydroxy-alcoyle inférieur, di-substitué par un alcoylène inférieur à 4 à 7 chaînons ou un 3-oxa-, 3-thia- ou 3-aza-alcoylène, $R_3$ représente un hydrogène ou un alcoyle inférieur, alc représente un vinylène et $R_1$ et Ph ont les significations données ci-dessus, caractérisé en ce qu'on déshydrogène des composés de formule

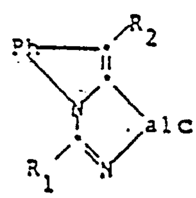

(I)

où alc représente un éthylène, avec séparation d'hydrogène et formation simultanée d'une liaison supplémentaire et si on le désire en ce qu'on transforme un composé obtenu selon l'invention en un autre composé de formule I ou un composé libre de formule I obtenu selon l'invention en un sel ou un sel obtenu selon le procédé en le composé libre de formule I ou en un autre sel.

3. Procédé selon les revendications 1 et 2, caractérisé en ce qu'on forme le produit de départ de formule (II) dans les conditions de la réaction sans isolement *in situ* par cyclisation de composés de formule

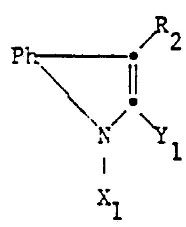

(IVa)

ou de leurs sels, où $X_1$ est un hydrogène et $Y_1$ est un groupe de formule $—alc—NH—C(=Z_1)(R_1)$ et $Z_1$ représente un oxo et Ph, $R_1$ et $R_2$ ont les significations données dans la revendication 1.

4. Procédé selon l'une des revendications 1 à 3 de préparation de composés de formule I, où $R_1$ représente un phényle éventuellement substitué par un alcoyle inférieur, un alcoxy inférieur, un alcoyle inférieur-thio, un alcane inférieur-sulfinyle, un halogène-alcane inférieur-sulfinyle, un alcane inférieur-sulfonyle, un halogène-alcane inférieur-sulfonyle, un sulfamoyle, un N-mono- ou N,N-dialcoyle inférieur-sulfamoyle et/ou un halogène ou un pyrrolyle, furyle, thiényle, thiazolyle, pyridyle ou pyrimidyle éventuellement substitué par un alcoyle inférieur, un alcoxy inférieur et/ou un halogène, $R_2$ est un groupe de formule —CH($R_3$)—$R_2'$, où $R_2'$ est un carboxy, un phényl- ou pyridylalcoxy-carbonyle éventuellement substitué par un alcoyle inférieur, un alcoxy inférieur ou un halogène, un hydroxy- ou alcoxy inférieur-alcoxy inférieur-carbonyle, un alcoxy inférieur-carbonyle, un carbamoyle, un N-hydroxy- ou un N-amino-carbamoyle, un N-mono- ou N,N-dialcoyle inférieur-carbamoyle ou -hydroxyalcoyle inférieur-carbamoyle ou un carbamoyle substitué par un alcoylène inférieur à 4 à 7 chaînons, ou un 3-oxa-, 3-thia- ou 3-azaalcoylèneaminocarbonyle, $R_3$ représente un hydrogène ou un alcoyle inférieur, Ph représente un 1,2-phénylène éventuellement substitué par un alcoyle inférieur, un alcoxy inférieur, un halogène et/ou un trifluorométhyle et alc est un alcoylène inférieur séparant le groupe méthine du groupe imino par 1 à 3 atomes de carbone ou un alcénylène inférieur le séparant par 2 atomes de carbone, ainsi que de leurs sels, caractérisé en ce qu'on part de composés de formules II ou III, V, VIII, X, XIa ou I où les variables ont les significations données dans la revendication 1.

5. Procédé selon l'une des revendications 1 à 3 de préparation de composés de formule I où $R_1$ représente un phényle éventuellement substitué par un alcoyle inférieur ayant jusqu'à 4 atomes de carbone compris, un alcoxy inférieur ayant jusqu'à 4 atomes de carbone compris, un halogènealcoyle inférieur-thio ayant jusqu'à 4 atomes de carbone compris, un alcoyle inférieur-thio ayant jusqu'à 4 atomes de carbone compris, un halogène-alcane inférieur-sulfinyle ayant jusqu'à 4 atomes de carbone compris, un alcane inférieur-sulfinyle ayant jusqu'à 4 atomes de carbone compris, un halogène-alcane inférieur-sulfonyle, un alcane inférieur-sulfonyle ayant jusqu'à 4 atomes de carbone compris, un sulfamoyle, un N-mono- ou N,N-dialcane inférieur-sulfamoyle ayant à chaque fois jusqu'à 4 atomes de carbone compris dans la fraction alcoyle et/ou par un halogène de numéro atomique allant jusqu'à 35 compris, ou un pyridyle ou thiényle éventuellement substitué par un alcoyle inférieur ayant jusqu'à 4 atomes de carbone compris, par un alcoxy inférieur ayant jusqu'à 4 atomes de carbone compris, et/ou un halogène de numéro atomique allant jusqu'à 35 compris, $R_2$ représente un groupe de formule —CH($R_3$)—$R_2'$, où $R_2'$ représente un carboxy, $R_2'$ est un phényl- ou pyridyl-alcoxy inférieur-carbonyle éventuellement substitué par un alcoyle inférieur ayant jusqu'à 4 atomes de carbone compris, un alcoxy inférieur ayant jusqu'à 4 atomes de carbone compris, et/ou un halogène, un alcoxy inférieur-carbonyle, un mono- ou dihydroxy-alcoxy inférieur-carbonyle, un alcoxy inférieur-alcoxy inférieur-carbonyle, un N-hydroxy- ou N-amino-carbamoyle, un N-mono- ou N,N-dialcoxy inférieur-carbamoyle ou un carbamoyle, $R_3$ représente un hydrogène ou un alcoyle inférieur ayant jusqu'à 4 atomes de carbone compris, Ph représente un 1,2-phénylène éventuellement substitué par un alcoyle inférieur ayant jusqu'à 4 atomes de carbone compris, un alcoxy inférieur ayant jusqu'à 4 atomes de carbone compris et/ou un halogène de numéro atomique allant jusqu'à 35 compris, et alc est un 1,2-éthylène, ainsi que de leurs sels, caractérisé en ce qu'on part de composés de formules II ou III, V, VIII, X ou XIa où les variables ont les significations données dans la revendication 1.

6. Procédé selon l'une des revendications 1 à 3 de préparation de composés de formule I où $R_1$ représente un phényle éventuellement substitué par un halogène, un alcoyle inférieur-thio, un alcane inférieur-sulfinyle, un alcane inférieur-sulfonyle, un halogène-alcoyle inférieur-thio et/ou un sulfamoyle, ou un pyridyle ou un thiényle ayant les mêmes substituants, $R_2$ est un groupe de formule —$CH_2$—$R_2'$ où $R_2'$ représente un carboxy, un alcoxy inférieur-carbonyle ou un carbamoyle, Ph représente un 1,2-phénylène éventuellement substitué par un alcoxy inférieur, un alcoyle inférieur et/ou un halogène et alc est un 1,2-éthylène ou un vinylène, ainsi que de leurs sels, caractérisé en ce qu'on part de composés de formules II ou III, V, VIII, X, XIa ou I où les variables ont les significations données dans la revendication 1.

7. Procédé selon l'une des revendications 1 à 3 de préparation de composés de formule I où $R_1$ représente un phényle, pyridyle ou thiényle éventuellement substitué par un halogène de numéro atomique allant jusqu'à 35 compris, un alcoyle inférieur-thio ayant jusqu'à 4 atomes de carbone compris, un alcane inférieur-sulfinyle ayant jusqu'à 4 atomes de carbone compris, un halogène-alcoyle inférieur-thio ayant jusqu'à 4 atomes de carbone compris et/ou un sulfamoyle, $R_2$ est un groupe de formule —$CH_2$—$R_2'$, où $R_2'$ représente un carboxy, un alcoxy inférieur-carbonyle ayant jusqu'à 5 atomes de carbone compris ou un carbamoyle, Ph représente un 1,2-phénylène éventuellement substitué par un alcoxy inférieur ayant jusqu'à 4 atomes de carbone compris, un alcoyle inférieur ayant jusqu'à 4 atomes de carbone compris ou un halogène de numéro atomique allant jusqu'à 35 compris et alc est un 1,2-éthylène ou un vinylène, ainsi que de leurs sels, caractérisé en ce qu'on part de composés de formules II ou III, V, VIII, X, XIa ou I où les variables ont les significations données dans la revendication 1.

8. Procédé selon l'une des revendications 1 à 3 de préparation de composés de formule I où $R_1$ représente un phényle, pyridyle ou thiényle éventuellement substitué par un halogène-alcoyle inférieur-thio ayant jusqu'à 4 atomes de carbone compris, un alcoyle inférieur-thio ayant jusqu'à 4 atomes de carbone compris, un alcane inférieur-sulfinyle ayant jusqu'à 4 atomes de carbone, ou un halogène de numéro atomique allant jusqu'à 35 compris, en position p ou par un sulfamoyle en position 3 et en outre par un halogène de numéro atomique allant jusqu'à 35 compris en position 4, $R_2$ représente un groupe de

formule $—CH_2—R_2'$ où $R_2'$ est un carboxy, un alcoxy inférieur-carbonyle ayant jusqu'à 5 atomes de carbone compris ou un carbamoyle, Ph représente un 1,2-phénylène éventuellement substitué par rapport à l'atome d'azote lié en position 4 par un alcoxy inférieur ayant jusqu'à 4 atomes de carbone compris, en positions 3 et 5 par un alcoyle inférieur ayant jusqu'à 4 atomes de carbone compris ou en position 4 par un halogène de numéro atomique allant jusqu'à 35 compris et alc est un 1,2-éthylène ou un vinylène, ainsi que de leurs sels, caractérisé en ce qu'on part de composés de formules II ou III, V, VIII, X, XIa ou I où les variables ont les significations données dans la revendication 1.

9. Procédé selon l'une des revendications 1 à 3 de préparation de composés de formule I où $R_1$ représente un phényle éventuellement substitué en position p par un alcoyle inférieur-thio ayant jusqu'à 4 atomes de carbone compris, par un alcane inférieur-sulfinyle ayant jusqu'à 4 atomes de carbone compris ou un halogène de numéro atomique allant jusqu'à 35 compris, $R_2$ représente un alcoxy inférieur-carbonylméthyle ayant jusqu'à 6 atomes de carbone compris, Ph représente un 1,2-phénylène éventuellement substitué en position p par rapport à l'atome d'azote par un alcoxy inférieur ayant jusqu'à 4 atomes de carbone compris ou un halogène de numéro atomique allant jusqu'à 35 compris et alc représente un 1,2-éthylène, ainsi que de leurs sels, caractérisé en ce qu'on part de composés de formules II ou III, V, VIII, X ou XIa où les variables ont les significations données dans la revendication 1.

10. Procédé selon l'une des revendications 1 à 3 de préparation de composés de formule I où $R_1$ représente un phényle éventuellement substitué en position p par un alcoyle inférieur-thio ayant jusqu'à 4 atomes de carbone compris, par un alcane inférieur-sulfinyle ayant jusqu'à 4 atomes de carbone compris ou un halogène de numéro atomique allant jusqu'à 35 compris, $R_2$ est un carboxyméthyle, Ph représente un 1,2-phénylène éventuellement substitué en position p par rapport à l'atome d'azote par un alcoxy inférieur ayant jusqu'à 4 atomes de carbone compris ou un halogène de numéro atomique allant jusqu'à 35 compris et alc représente un 1,2-éthylène, ainsi que de leurs sels, caractérisé en ce qu'on part de composés de formules II ou III, V, VIII, X ou XIa où les variables ont les significations données dans la revendication 1.

11. Procédé selon l'une des revendications 1 à 3 de préparation d'ester éthylique de l'acide 7-méthoxy-1-(p-chlorophényl)-3,4-dihydro-pyrimido [1,6-a] indol-acétique ou d'un de ses sels, caractérisé en ce qu'on part de composés de formules II ou III, V, VIII, X ou XIa.

12. Procédé selon l'une des revendications 1 à 3 de préparation de l'ester éthylique de l'acide 1-phényl-3,4-dihydro-pyrimido [1,6-a] indol-acétique ou d'un de ses sels, caractérisé en ce qu'on part de composés de formules II ou III, V, VIII, X ou XIa.

13. Procédé selon l'une des revendications 1 à 3 de préparation d'ester éthylique de l'acide 7-fluoro-1-(p-méthylthio-phényl)-3,4-dihydro-pyrimido [1,6-a] indol-5-acétique ou d'un de ses sels, caractérisé en ce qu'on part de composés de formules II ou III, V, VIII, X ou XIa.

14. Procédé selon l'une des revendications 1 à 3 de préparation d'ester éthylique de l'acide 6,8-diméthyl-1-phényl-3,4-dihydro-pyrimido [1,6-a] indol-5-acétique ou d'un de ses sels, caractérisé en ce qu'on part de composés de formules II ou III, V, VIII, X ou XIa.

15. Procédé selon l'une des revendications 1 à 3 de préparation d'ester éthylique de l'acide 1-(3-sulfamoyl-4-chloro-phényl)-3,4-dihydro-pyrimido [1,6-a] indol-5-acétique ou d'un de ses sels, caractérisé en ce qu'on part de composés de formules II ou III, V, VIII, X ou XIa.

16. Procédé selon l'une des revendications 1 à 3 de préparation d'ester éthylique de l'acide 1-(2,6-dichlorophényl)-3,4-dihydro-pyrimido [1,6-a] indol-5-acétique ou d'un de ses sels, caractérisé en ce qu'on part de composés de formules II ou III, V, VIII, X ou XIa.

17. Procédé selon l'une des revendications 1 à 3 de préparation d'ester éthylique de l'acide 1-(2-picolinyl)-3,4-dihydro-pyrimido [1,6-a] indol-5-acétique ou d'un de ses sels, caractérisé en ce qu'on part de composés de formules II ou III, V, VIII, X ou XIa.

18. Procédé selon l'une des revendications 1 à 3 de préparation d'ester éthylique de l'acide 1-(2-thiényl)-3,4-dihydro-pyrimido [1,6-a] indol-5-acétique ou d'un de ses sels, caractérisé en ce qu'on part de composés de formules II ou III, V, VIII, X ou XIa.

19. Procédé selon l'une des revendications 1 à 3 de préparation de l'acide 7-méthoxy-1-(p-chlorophényl)-3,4-dihydro-pyrimido [1,6-a] indol-5-acétique ou d'un de ses sels, caractérisé en ce qu'on part de composés de formules II ou III, V, VIII, X ou XIa.

20. Procédé selon l'une des revendications 1 à 3 de préparation d'acide 1-phényl-3,4-dihydro-pyrimido [1,6-a] indol-5-acétique ou d'un de ses sels, caractérisé en ce qu'on part de composés de formules II ou III, V, VIII, X ou XIa.

21. Procédé selon l'une des revendications 1 à 3 de préparation de l'acide 7-fluoro-1-(p-méthylthio-phényl)-3,4-dihydro-pyrimido [1,6-a] indol-5-acétique ou d'un de ses sels, caractérisé en ce qu'on part de composés de formules II ou III, V, VIII, X ou XIa.

22. Procédé selon l'une des revendications 1 à 3 de préparation de l'acide 7-fluoro-1-(p-méthylsulfinyl-phényl)-3,4-dihydro-pyrimido [1,6-a] indol-5-acétique ou d'un de ses sels, caractérisé en ce qu'on part de composés de formules II ou III, V, VIII, X ou XIa.

23. Procédé selon l'une des revendications 1 à 3 de préparation de l'acide 6,8-diméthyl-1-phényl-3,4-dihydro-pyrimido [1,6-a] indol-5-acétique ou d'un de ses sels, caractérisé en ce qu'on part de composés de formules II ou III, V, VIII, X ou XIa.

24. Procédé selon l'une des revendications 1 à 3 de préparation de l'acide 1-(3-sulfamoyl-4-chloro-

phényl)-3,4-dihydro-pyrimido [1,6-a] indol-5-acétique ou d'un de ses sels, caractérisé en ce qu'on part de composés de formules II ou III, V, VIII, X ou XIa.

25. Procédé selon l'une des revendications 1 à 3 de préparation de l'acide 1-(2,6-dichlorophényl)-3,4-dihydro-pyrimido [1,6-a] indol-5-acétique ou d'un de ses sels, caractérisé en ce qu'on part de composés de formules II ou III, V, VIII, X ou XIa.

26. Procédé selon l'une des revendications 1 à 3 de préparation de l'acide 1-(2-thiényl)-3,4-dihydro-pyrimido [1,6-a] indol-5-acétique ou d'un de ses sels, caractérisé en ce qu'on part de composés de formules II ou III, V, VIII, X ou XIa.

27. Procédé selon l'une des revendications 1 à 3 de préparation de l'amide de l'acide 1-phényl-3,4-dihydro-pyrimido [1,6-a] indol-5-acétique ou d'un de ses sels, caractérisé en ce qu'on part de composés de formules II ou III, V, VIII, X ou XIa.

28. Procédé selon l'une des revendications 1 à 3 de préparation du 1-(3-sulfamoyl-4-chloro-phényl)-3,4-dihydro-pyrimido [1,6-a] indol-5-acétamide ou d'un de ses sels, caractérisé en ce qu'on part de composés de formules II ou III, V, VIII, X ou XIa.

29. Procédé selon l'une des revendications 1 à 3 de préparation de l'ester éthylique de l'acide 1-(p-méthyl-sulfoxy-phényl)-3,4-dihydro-pyrimido [1,6-a] indol-5-acétique ou d'un de ses sels, caractérisé en ce qu'on part de composés de formules II ou III, V, VIII, X ou XIa.

30. Procédé selon l'une des revendications 1 à 3 de préparation de l'ester éthylique de l'acide 7-fluoro-1-(p-chlorométhylthio-phényl)-3,4-dihydro-pyrimido [1,6-a] indol-5-acétique ou d'un de ses sels, caractérisé en ce qu'on part de composés de formules II ou III, V, VIII, X ou XIa.

31. Procédé selon l'une des revendications 1 à 3 de préparation de l'ester éthylique de l'acide 7-fluoro-1-(p-méthylsulfoxyphényl)-3,4-dihydro-pyrimido [1,6-a] indol-5-acétique ou d'un de ses sels, caractérisé en ce qu'on part de composés de formules II ou III, V, VIII, X ou XIa.

32. Procédé selon l'une des revendications 1 à 3 de préparation de l'ester éthylique de l'acide 7-fluoro-1-(p-méthylthio-phényl)-pyrimido [1,6-a] indol-5-acétique ou d'un de ses sels, caractérisé en ce qu'on part du composé de formule I correspondant.

33. Procédé selon l'une des revendications 1 à 3 de préparation de l'ester éthylique de l'acide 1-phényl-pyrimido [1,6-a] indol-5-acétique ou d'un de ses sels, caractérisé en ce qu'on part du composé de formule I correspondant.

34. Procédé selon l'une des revendications 1 à 3 de préparation de l'ester éthylique de l'acide 7-méthoxy-1-(p-chlorophényl)-pyrimido [1,6-a] indol-5-acétique ou d'un de ses sels, caractérisé en ce qu'on part du composé de formule I correspondant.

35. Procédé selon l'une des revendications 1 à 3 de préparation de l'acide 7-fluoro-1-(p-méthylthio-phényl)-pyrimido [1,6-a] indol-5-acétique ou d'un de ses sels, caractérisé en ce qu'on part du composé de formule I correspondant.

36. Procédé de préparation de préparations pharmaceutiques, caractérisé en ce qu'on mélange un composé selon l'une des revendications 1 à 35 sous forme libre ou sous la forme d'un sel pharmaceutiquement acceptable avec des adjuvants et/ou supports pharmaceutiques habituels.